# EUROPEAN PATENT APPLICATION

(11) **EP 2 407 783 A1**
(43) Date of publication of application: **18.01.2012**
(21) Application number: 11182489.2
(22) Date of filing: 29.10.2007
(51) Int. Cl.: G01N 33/50, G01N 33/68, C12Q 1/48, G01N 33/574

(54) **A method for pharmacologically profiling compounds**

(30) Priority: 27.10.2006 EP 06123106
(62) Divisional of application: 07821970.6
(71) Applicant: Janssen Pharmaceutica, N.V., 2340 Beerse (BE)
(72) Inventor: Versele, Matthias Luc A, 2340 Beerse (BE); Perera, Timothy Pietro Suren, 2340 Beerse (BE); Talloen, Willem Jan-Paul Edmond, 2340 Beerse (BE); Houtman, René, 4105VG Culemborg (NL); Ruijtenbeek, Robby, 3524BK Utrecht (NL)
(74) Representative: Vandersteen, Pieter

(57) **Abstract**

The present invention is concerned with method for pharmacologically profiling compounds using an array of substrates, in particular kinase substrates, immobilized on a porous matrix. This method was found particular useful in the prediction of drug response, i.e. to enable the distinction between responders and non-responders in the treatment of cells, tissues, organs or warm-blooded animals with the compound to be tested, and in compound differentiation.

SEQUENCE LISTING

<110>  Janssen Pharmaceutica NV
       Versele, Matthias
       Perera, Timothy
       Talloen, Willem

<120>  A method for pharmacologically profiling compounds

<130>  PRD2759-PCT

<160>  337

<170>  PatentIn version 3.4

<210>  1
<211>  13
<212>  PRT
<213>  Homo sapiens

<400>  1

Arg Leu Asp Gly Glu Asn Ile Tyr Ile Arg His Ser Asn
1               5                   10


<210>  2
<211>  13
<212>  PRT
<213>  Homo sapiens

<400>  2

Trp Gly Arg Gly Thr Asp Glu Tyr Phe Ile Arg Lys Pro
1               5                   10


<210>  3
<211>  13
<212>  PRT
<213>  Homo sapiens

<400>  3

Tyr Ile Ser Lys Ala Glu Glu Tyr Phe Leu Leu Lys Ser
1               5                   10


<210>  4
<211>  13
<212>  PRT
<213>  Homo sapiens

<400>  4

Glu Arg Glu Gly Ser Lys Arg Tyr Cys Ile Gln Thr Lys
1               5                   10


<210>  5
<211>  13
<212>  PRT
<213>  Homo sapiens

<400>  5

Ile Glu Asn Glu Glu Gln Glu Tyr Val Gln Thr Val Lys
1               5                   10

<210>  6
<211>  13
<212>  PRT
<213>  Homo sapiens

<400>  6

His Ser Thr Pro Pro Ser Ala Tyr Gly Ser Val Lys Ala
1               5                   10


<210>  7
<211>  13
<212>  PRT
<213>  Homo sapiens

<400>  7

Thr Glu Ala Thr Ala Thr Asp Tyr His Thr Thr Ser His
1               5                   10


<210>  8
<211>  13
<212>  PRT
<213>  Homo sapiens

<400>  8

Thr Glu Ala Ser Gly Tyr Ile Ser Ser Leu Glu Tyr Pro
1               5                   10


<210>  9
<211>  13
<212>  PRT
<213>  Homo sapiens

<400>  9

Phe Asp Lys Asp Gly Asn Gly Tyr Ile Ser Ala Ala Glu
1               5                   10


<210>  10
<211>  13
<212>  PRT
<213>  Homo sapiens

<400>  10

Lys Asp Gly Asn Gly Tyr Ile Ser Ala Ala Glu Leu Arg
1               5                   10


<210>  11
<211>  13
<212>  PRT
<213>  Homo sapiens

<400>  11

Glu Gly Glu Glu Asp Thr Glu Tyr Met Thr Pro Ser Ser
1               5                   10


<210>  12
<211>  13

<212>  PRT
<213>  Homo sapiens

<400>  12

Ser Thr Ala Thr Lys Asp Thr Tyr Asp Ala Leu His Met
1               5                   10


<210>  13
<211>  13
<212>  PRT
<213>  Homo sapiens

<400>  13

Glu Tyr Glu Asp Glu Asn Leu Tyr Glu Gly Leu Asn Leu
1               5                   10


<210>  14
<211>  13
<212>  PRT
<213>  Homo sapiens

<400>  14

Glu Lys Ile Gly Glu Gly Thr Tyr Gly Val Val Tyr Lys
1               5                   10


<210>  15
<211>  13
<212>  PRT
<213>  Homo sapiens

<400>  15

Gly Leu Ala Lys Ser Phe Gly Ser Pro Asn Arg Ala Tyr
1               5                   10


<210>  16
<211>  13
<212>  PRT
<213>  Homo sapiens

<400>  16

Gln Lys Arg Arg Glu Ile Leu Ser Arg Arg Pro Ser Tyr
1               5                   10


<210>  17
<211>  13
<212>  PRT
<213>  Homo sapiens

<400>  17

Gly Pro Pro Glu Pro Gly Pro Tyr Ala Gln Pro Ser Val
1               5                   10


<210>  18
<211>  13
<212>  PRT
<213>  Homo sapiens

<400>  18

Val Ala Asp Ile Asp Gly Gln Tyr Ala Met Thr Arg Ala
1               5                   10


<210>  19
<211>  13
<212>  PRT
<213>  Homo sapiens

<400>  19

Gly Ile Val Tyr Ala Val Ser Ser Asp Arg Phe Arg Ser
1               5                   10


<210>  20
<211>  13
<212>  PRT
<213>  Homo sapiens

<400>  20

Leu Leu Leu Ser Asn Pro Ala Tyr Arg Leu Leu Leu Ala
1               5                   10


<210>  21
<211>  13
<212>  PRT
<213>  Homo sapiens

<400>  21

Phe Gly Met Ser Arg Asn Leu Tyr Ala Gly Asp Tyr Tyr
1               5                   10


<210>  22
<211>  13
<212>  PRT
<213>  Homo sapiens

<400>  22

Arg Asn Leu Tyr Ser Gly Asp Tyr Tyr Arg Ile Gln Gly
1               5                   10


<210>  23
<211>  13
<212>  PRT
<213>  Homo sapiens

<400>  23

Lys His Asp Thr Glu Met Lys Tyr Tyr Ile Val His Leu
1               5                   10


<210>  24
<211>  13
<212>  PRT
<213>  Homo sapiens

<400>  24

Cys Gln Leu Gly Gln Arg Ile Tyr Gln Tyr Ile Gln Ser

1                   5                        10


<210>  25
<211>  13
<212>  PRT
<213>  Homo sapiens

<400>  25

Gly Gly Thr Asp Glu Gly Ile Tyr Asp Val Pro Leu Leu
1               5                   10


<210>  26
<211>  13
<212>  PRT
<213>  Homo sapiens

<400>  26

Gly Gly Thr Asp Glu Gly Ile Phe Asp Val Pro Leu Leu
1               5                   10


<210>  27
<211>  13
<212>  PRT
<213>  Homo sapiens

<400>  27

Glu Asp Ser Phe Leu Gln Arg Tyr Ser Ser Asp Pro Thr
1               5                   10


<210>  28
<211>  13
<212>  PRT
<213>  Homo sapiens

<400>  28

Gly Ser Val Gln Asn Pro Val Tyr His Asn Gln Pro Leu
1               5                   10


<210>  29
<211>  13
<212>  PRT
<213>  Homo sapiens

<400>  29

Ala Pro Ser Arg Asp Pro His Tyr Gln Asp Pro His Ser
1               5                   10


<210>  30
<211>  13
<212>  PRT
<213>  Homo sapiens

<400>  30

Ile Ser Leu Asp Asn Pro Asp Tyr Gln Gln Asp Phe Phe
1               5                   10

<210>  31
<211>  13
<212>  PRT
<213>  Homo sapiens

<400>  31

Ser Thr Ala Glu Asn Ala Glu Tyr Leu Arg Val Ala Pro
1               5                   10


<210>  32
<211>  13
<212>  PRT
<213>  Homo sapiens

<400>  32

Leu Gly Ala Glu Glu Lys Glu Tyr His Ala Glu Gly Gly
1               5                   10


<210>  33
<211>  13
<212>  PRT
<213>  Homo sapiens

<400>  33

Met Thr Phe Gly Ser Lys Pro Tyr Asp Gly Ile Pro Ala
1               5                   10


<210>  34
<211>  13
<212>  PRT
<213>  Homo sapiens

<400>  34

Ser Gly Ala Ser Thr Gly Ile Tyr Glu Ala Leu Glu Leu
1               5                   10


<210>  35
<211>  13
<212>  PRT
<213>  Homo sapiens

<400>  35

Leu Asp Asp Phe Asp Gly Thr Tyr Glu Thr Gln Gly Gly
1               5                   10


<210>  36
<211>  13
<212>  PRT
<213>  Homo sapiens

<400>  36

Gln Leu Lys Pro Leu Lys Thr Tyr Val Asp Pro His Thr
1               5                   10


<210>  37
<211>  13
<212>  PRT

<213>  Homo sapiens

<400>  37

Glu Asp Asp Pro Glu Ala Thr Tyr Thr Thr Ser Gly Gly
1               5                   10


<210>  38
<211>  13
<212>  PRT
<213>  Homo sapiens

<400>  38

Leu Asn Gln Gly Val Arg Thr Tyr Val Asp Pro Phe Thr
1               5                   10


<210>  39
<211>  13
<212>  PRT
<213>  Homo sapiens

<400>  39

Gln Ala Ile Lys Met Asp Arg Tyr Lys Asp Asn Phe Thr
1               5                   10


<210>  40
<211>  13
<212>  PRT
<213>  Homo sapiens

<400>  40

Thr Tyr Ile Asp Pro Glu Thr Tyr Glu Asp Pro Asn Arg
1               5                   10


<210>  41
<211>  13
<212>  PRT
<213>  Homo sapiens

<400>  41

Asp Asp Thr Ser Asp Pro Thr Tyr Thr Ser Ser Leu Gly
1               5                   10


<210>  42
<211>  13
<212>  PRT
<213>  Homo sapiens

<400>  42

Ser Ala Ile Lys Met Val Gln Tyr Arg Asp Ser Phe Leu
1               5                   10


<210>  43
<211>  13
<212>  PRT
<213>  Homo sapiens

<400>  43

Ile Gly His Gly Thr Lys Val Tyr Ile Asp Pro Phe Thr
1               5                   10

<210>  44
<211>  13
<212>  PRT
<213>  Homo sapiens

<400>  44

Ser Glu His Ala Gln Asp Thr Tyr Leu Val Leu Asp Lys
1               5                   10

<210>  45
<211>  13
<212>  PRT
<213>  Homo sapiens

<400>  45

Ala Ser Ala Ala Ser Phe Glu Tyr Thr Ile Leu Asp Pro
1               5                   10

<210>  46
<211>  13
<212>  PRT
<213>  Homo sapiens

<400>  46

Pro Thr Ala Glu Asn Pro Glu Tyr Leu Gly Leu Asp Val
1               5                   10

<210>  47
<211>  13
<212>  PRT
<213>  Homo sapiens

<400>  47

Leu Asp Ile Asp Glu Thr Glu Tyr His Ala Asp Gly Gly
1               5                   10

<210>  48
<211>  13
<212>  PRT
<213>  Homo sapiens

<400>  48

Pro Ile Ser Thr Ile Asp Val Tyr Met Ile Met Val Lys
1               5                   10

<210>  49
<211>  13
<212>  PRT
<213>  Homo sapiens

<400>  49

Gln Ala Leu Asp Asn Pro Glu Tyr His Asn Ala Ser Asn
1               5                   10

<210>  50
<211>  13
<212>  PRT
<213>  Homo sapiens

<400>  50

Ile Val Ala Glu Asn Pro Glu Tyr Leu Ser Glu Phe Ser
1               5                   10


<210>  51
<211>  13
<212>  PRT
<213>  Homo sapiens

<400>  51

Arg Leu Gln Arg Arg Arg Gly Ser Ser Ile Pro Gln Phe
1               5                   10


<210>  52
<211>  13
<212>  PRT
<213>  Homo sapiens

<400>  52

Asp Ser Lys Asn Phe Asp Asp Tyr Met Lys Ser Leu Gly
1               5                   10


<210>  53
<211>  13
<212>  PRT
<213>  Homo sapiens

<400>  53

Arg Tyr Met Glu Asp Ser Thr Tyr Tyr Lys Ala Ser Lys
1               5                   10


<210>  54
<211>  13
<212>  PRT
<213>  Homo sapiens

<400>  54

Arg Tyr Ile Glu Asp Glu Asp Tyr Tyr Lys Ala Ser Val
1               5                   10


<210>  55
<211>  13
<212>  PRT
<213>  Homo sapiens

<400>  55

Arg Gln Glu Asp Gly Gly Val Tyr Ser Ser Ser Gly Leu
1               5                   10


<210>  56

<211>  13
<212>  PRT
<213>  Homo sapiens

<400>  56

Arg Glu Glu Ala Asp Gly Val Tyr Ala Ala Ser Gly Gly
1               5                   10


<210>  57
<211>  13
<212>  PRT
<213>  Homo sapiens

<400>  57

Ala Leu Thr Ser Asn Gln Glu Tyr Leu Asp Leu Ser Met
1               5                   10


<210>  58
<211>  13
<212>  PRT
<213>  Homo sapiens

<400>  58

Thr Leu Thr Thr Asn Glu Glu Tyr Leu Asp Leu Ser Gln
1               5                   10


<210>  59
<211>  13
<212>  PRT
<213>  Homo sapiens

<400>  59

Asp Val His Asn Leu Asp Tyr Tyr Lys Lys Thr Thr Asn
1               5                   10


<210>  60
<211>  13
<212>  PRT
<213>  Homo sapiens

<400>  60

Thr Val Thr Ser Thr Asp Glu Tyr Leu Asp Leu Ser Ala
1               5                   10


<210>  61
<211>  13
<212>  PRT
<213>  Homo sapiens

<400>  61

Lys Val Asp Asn Glu Asp Ile Tyr Glu Ser Arg His Glu
1               5                   10


<210>  62
<211>  13
<212>  PRT
<213>  Homo sapiens

<400>  62

Arg Gly Glu Pro Asn Val Ser Tyr Ile Cys Ser Arg Tyr
1               5                   10


<210>  63
<211>  13
<212>  PRT
<213>  Homo sapiens

<400>  63

Asp Gly Lys Lys Arg Lys Arg Ser Arg Lys Glu Ser Tyr
1               5                   10


<210>  64
<211>  13
<212>  PRT
<213>  Homo sapiens

<400>  64

Ser Leu Gly Phe Lys Arg Ser Tyr Glu Glu His Ile Pro
1               5                   10


<210>  65
<211>  13
<212>  PRT
<213>  Homo sapiens

<400>  65

Tyr Ala Ser Ser Asn Pro Glu Tyr Leu Ser Ala Ser Asp
1               5                   10


<210>  66
<211>  13
<212>  PRT
<213>  Homo sapiens

<400>  66

Ser Ser Glu Asp Leu Ser Ala Tyr Ala Ser Ile Ser Phe
1               5                   10


<210>  67
<211>  13
<212>  PRT
<213>  Homo sapiens

<400>  67

Gly Gly Gly Gly Gly Glu Phe Tyr Gly Tyr Met Thr Met
1               5                   10


<210>  68
<211>  13
<212>  PRT
<213>  Homo sapiens

<400>  68

Ala Ile Glu Thr Asp Lys Glu Tyr Tyr Thr Val Lys Asp
1               5                   10

<210>  69
<211>  13
<212>  PRT
<213>  Homo sapiens

<400>  69

Gly Ala Gly Phe Gly Ser Arg Ser Leu Tyr Gly Leu Gly
1               5                   10

<210>  70
<211>  13
<212>  PRT
<213>  Homo sapiens

<400>  70

Ser Ala Tyr Gly Gly Leu Thr Ser Pro Gly Leu Ser Tyr
1               5                   10

<210>  71
<211>  13
<212>  PRT
<213>  Homo sapiens

<400>  71

Ala Leu Arg Ala Asp Glu Asn Tyr Tyr Lys Ala Gln Thr
1               5                   10

<210>  72
<211>  13
<212>  PRT
<213>  Homo sapiens

<400>  72

Met Glu Ser Ile Asp Asp Tyr Val Asn Val Pro Glu Ser
1               5                   10

<210>  73
<211>  13
<212>  PRT
<213>  Homo sapiens

<400>  73

Glu Glu Gly Ala Pro Asp Tyr Glu Asn Leu Gln Glu Leu
1               5                   10

<210>  74
<211>  13
<212>  PRT
<213>  Homo sapiens

<400>  74

Arg Leu Ile Glu Asp Asn Glu Tyr Thr Ala Arg Glu Gly
1               5                   10

<210>  75
<211>  13
<212>  PRT
<213>  Homo sapiens

<400>  75

Arg Asp Ile Tyr Arg Ala Ser Tyr Tyr Arg Arg Gly Asp
1               5                   10


<210>  76
<211>  13
<212>  PRT
<213>  Homo sapiens

<400>  76

Ala Arg Thr Ala His Tyr Gly Ser Leu Pro Gln Lys Ser
1               5                   10


<210>  77
<211>  13
<212>  PRT
<213>  Homo sapiens

<400>  77

Phe Gly Tyr Gly Gly Arg Ala Ser Asp Tyr Lys Ser Ala
1               5                   10


<210>  78
<211>  13
<212>  PRT
<213>  Homo sapiens

<400>  78

Gly Arg Ala Ser Asp Tyr Lys Ser Ala His Lys Gly Phe
1               5                   10


<210>  79
<211>  13
<212>  PRT
<213>  Homo sapiens

<400>  79

Arg Asp Met Tyr Asp Lys Glu Tyr Tyr Ser Val His Asn
1               5                   10


<210>  80
<211>  13
<212>  PRT
<213>  Homo sapiens

<400>  80

His Thr Gly Phe Leu Thr Glu Tyr Val Ala Thr Arg Trp
1               5                   10


<210>  81
<211>  13

<212>  PRT
<213>  Homo sapiens

<400>  81

Ile Met Leu Asn Ser Lys Gly Tyr Thr Lys Ser Ile Asp
1               5                   10


<210>  82
<211>  13
<212>  PRT
<213>  Homo sapiens

<400>  82

Ala Glu His Gln Tyr Phe Met Thr Glu Tyr Val Ala Thr
1               5                   10


<210>  83
<211>  13
<212>  PRT
<213>  Homo sapiens

<400>  83

Thr Ser Phe Met Met Thr Pro Tyr Val Val Thr Arg Tyr
1               5                   10


<210>  84
<211>  13
<212>  PRT
<213>  Homo sapiens

<400>  84

Ala Asp Ser Glu Met Thr Gly Tyr Val Val Thr Arg Trp
1               5                   10


<210>  85
<211>  13
<212>  PRT
<213>  Homo sapiens

<400>  85

Arg His Thr Asp Asp Glu Met Thr Gly Tyr Val Ala Thr
1               5                   10


<210>  86
<211>  13
<212>  PRT
<213>  Homo sapiens

<400>  86

Gln Arg Ser Arg Lys Arg Leu Ser Gln Asp Ala Tyr Arg
1               5                   10


<210>  87
<211>  13
<212>  PRT
<213>  Homo sapiens

<400>  87

Ala Lys Ala Leu Gly Lys Arg Thr Ala Lys Tyr Arg Trp
1               5                   10


<210>  88
<211>  13
<212>  PRT
<213>  Homo sapiens

<400>  88

His Ile Ile Glu Asn Pro Gln Tyr Phe Ser Asp Ala Cys
1               5                   10


<210>  89
<211>  13
<212>  PRT
<213>  Homo sapiens

<400>  89

Pro Val Ile Glu Asn Pro Gln Tyr Phe Gly Ile Thr Asn
1               5                   10


<210>  90
<211>  13
<212>  PRT
<213>  Homo sapiens

<400>  90

Phe Gly Met Ser Arg Asp Val Tyr Ser Thr Asp Tyr Tyr
1               5                   10


<210>  91
<211>  13
<212>  PRT
<213>  Homo sapiens

<400>  91

Arg Asp Val Tyr Ser Thr Asp Tyr Tyr Arg Val Gly Gly
1               5                   10


<210>  92
<211>  13
<212>  PRT
<213>  Homo sapiens

<400>  92

Asp Asp Ser Ser Ala Tyr Arg Ser Val Asp Glu Val Asn
1               5                   10


<210>  93
<211>  13
<212>  PRT
<213>  Homo sapiens

<400>  93

Ser Met Ser Asp Pro Gly Val Ser Tyr Arg Thr Arg Glu

1                   5                        10


<210>  94
<211>  13
<212>  PRT
<213>  Homo sapiens

<400>  94

Glu Pro His Val Thr Arg Arg Thr Pro Asp Tyr Phe Leu
1                   5                        10


<210>  95
<211>  13
<212>  PRT
<213>  Homo sapiens

<400>  95

Asn Glu Asn Thr Glu Asp Gln Tyr Ser Leu Val Glu Asp
1                   5                        10


<210>  96
<211>  13
<212>  PRT
<213>  Homo sapiens

<400>  96

Val Gly Glu Glu Glu His Val Tyr Ser Phe Pro Asn Lys
1                   5                        10


<210>  97
<211>  13
<212>  PRT
<213>  Homo sapiens

<400>  97

Phe Leu Ser Glu Glu Thr Pro Tyr Ser Tyr Pro Thr Gly
1                   5                        10


<210>  98
<211>  13
<212>  PRT
<213>  Homo sapiens

<400>  98

Ala Arg Thr Thr Ser Gln Leu Tyr Asp Ala Val Pro Ile
1                   5                        10


<210>  99
<211>  13
<212>  PRT
<213>  Homo sapiens

<400>  99

Asp Glu Asp Cys Tyr Gly Asn Tyr Asp Asn Leu Leu Ser
1                   5                        10

<210>  100
<211>  13
<212>  PRT
<213>  Homo sapiens

<400>  100

Lys Lys Asp Thr Glu Thr Val Tyr Ser Glu Val Arg Lys
1               5                   10


<210>  101
<211>  13
<212>  PRT
<213>  Homo sapiens

<400>  101

Gln Arg Ser Glu Leu Asp Lys Ser Ser Ala His Ser Tyr
1               5                   10


<210>  102
<211>  13
<212>  PRT
<213>  Homo sapiens

<400>  102

Leu Asp Thr Ser Ser Val Leu Tyr Thr Ala Val Gln Pro
1               5                   10


<210>  103
<211>  13
<212>  PRT
<213>  Homo sapiens

<400>  103

Val Ser Ser Asp Gly His Glu Tyr Ile Tyr Val Asp Pro
1               5                   10


<210>  104
<211>  13
<212>  PRT
<213>  Homo sapiens

<400>  104

Arg Pro Pro Ser Ala Glu Leu Tyr Ser Asn Ala Leu Pro
1               5                   10


<210>  105
<211>  13
<212>  PRT
<213>  Homo sapiens

<400>  105

Ser Ser Asn Tyr Met Ala Pro Tyr Asp Asn Tyr Val Pro
1               5                   10


<210>  106
<211>  13
<212>  PRT

<213>  Homo sapiens

<400>  106

Tyr Met Ala Pro Tyr Asp Asn Tyr Val Pro Ser Ala Pro
1               5                   10


<210>  107
<211>  13
<212>  PRT
<213>  Homo sapiens

<400>  107

Glu Gly Ser Phe Glu Ser Arg Tyr Gln Gln Pro Phe Glu
1               5                   10


<210>  108
<211>  13
<212>  PRT
<213>  Homo sapiens

<400>  108

Ile Gly Thr Ala Glu Pro Asp Tyr Gly Ala Leu Tyr Glu
1               5                   10


<210>  109
<211>  13
<212>  PRT
<213>  Homo sapiens

<400>  109

Glu Gly Arg Asn Pro Gly Phe Tyr Val Glu Ala Asn Pro
1               5                   10


<210>  110
<211>  13
<212>  PRT
<213>  Homo sapiens

<400>  110

Trp Thr Ala Ser Ser Pro Tyr Ser Thr Val Pro Pro Tyr
1               5                   10


<210>  111
<211>  13
<212>  PRT
<213>  Homo sapiens

<400>  111

Ser Lys Arg Lys Gly His Glu Tyr Thr Asn Ile Lys Tyr
1               5                   10


<210>  112
<211>  13
<212>  PRT
<213>  Homo sapiens

<400>  112

Arg Pro Arg Gly Gln Arg Asp Ser Ser Tyr Tyr Trp Glu
1               5                   10

<210>  113
<211>  13
<212>  PRT
<213>  Homo sapiens

<400>  113

Thr Val Asp Gly Lys Glu Ile Tyr Asn Thr Ile Arg Arg
1               5                   10

<210>  114
<211>  13
<212>  PRT
<213>  Homo sapiens

<400>  114

Ile Tyr Ile Ser Pro Leu Lys Ser Pro Tyr Lys Ile Ser
1               5                   10

<210>  115
<211>  13
<212>  PRT
<213>  Homo sapiens

<400>  115

Val Pro Thr Val Ser Lys Gly Thr Val Glu Gly Asn Tyr
1               5                   10

<210>  116
<211>  13
<212>  PRT
<213>  Homo sapiens

<400>  116

Thr Pro Ser Asp Ser Leu Ile Tyr Asp Asp Gly Leu Ser
1               5                   10

<210>  117
<211>  13
<212>  PRT
<213>  Homo sapiens

<400>  117

Ala Gln Ala Phe Pro Val Ser Tyr Ser Ser Ser Gly Ala
1               5                   10

<210>  118
<211>  13
<212>  PRT
<213>  Homo sapiens

<400>  118

Ser Ala Leu Leu Gly Asp His Tyr Val Gln Leu Pro Ala
1               5                   10

<210>  119
<211>  13
<212>  PRT
<213>  Homo sapiens

<400>  119

Tyr Val Gln Leu Pro Ala Thr Tyr Met Asn Leu Gly Pro
1               5                   10


<210>  120
<211>  13
<212>  PRT
<213>  Homo sapiens

<400>  120

Val Ser Gln Arg Glu Ala Glu Tyr Glu Pro Glu Thr Val
1               5                   10


<210>  121
<211>  13
<212>  PRT
<213>  Homo sapiens

<400>  121

Glu Tyr Glu Pro Glu Thr Val Tyr Glu Val Ala Gly Ala
1               5                   10


<210>  122
<211>  13
<212>  PRT
<213>  Homo sapiens

<400>  122

Tyr Gln Ala Glu Glu Asn Thr Tyr Asp Glu Tyr Glu Asn
1               5                   10


<210>  123
<211>  13
<212>  PRT
<213>  Homo sapiens

<400>  123

Leu Ala Lys Ala Val Asp Gly Tyr Val Lys Pro Gln Ile
1               5                   10


<210>  124
<211>  13
<212>  PRT
<213>  Homo sapiens

<400>  124

Asp Gly Pro Lys Gly Thr Gly Tyr Ile Lys Thr Glu Leu
1               5                   10


<210>  125

<211>  13
<212>  PRT
<213>  Homo sapiens

<400>  125

Asn Leu Gln Glu Arg Arg Lys Tyr Leu Lys His Arg Leu
1               5                   10


<210>  126
<211>  13
<212>  PRT
<213>  Homo sapiens

<400>  126

Asp Pro Gly Ser Ala Ala Pro Tyr Leu Lys Thr Lys Phe
1               5                   10


<210>  127
<211>  13
<212>  PRT
<213>  Homo sapiens

<400>  127

Thr Glu Arg Gly Asp Lys Gly Tyr Val Pro Ser Val Phe
1               5                   10


<210>  128
<211>  13
<212>  PRT
<213>  Homo sapiens

<400>  128

Pro Ser Asp Leu Leu Pro Met Ser Pro Ser Val Tyr Ala
1               5                   10


<210>  129
<211>  13
<212>  PRT
<213>  Homo sapiens

<400>  129

Asn Tyr Leu Arg Arg Arg Leu Ser Asp Ser Asn Phe Met
1               5                   10


<210>  130
<211>  13
<212>  PRT
<213>  Homo sapiens

<400>  130

Ser Gly Tyr Ser Ser Pro Gly Ser Pro Gly Thr Pro Gly
1               5                   10


<210>  131
<211>  13
<212>  PRT
<213>  Homo sapiens

<400>  131

Arg Tyr Phe Leu Asp Asp Gln Tyr Thr Ser Ser Ser Gly
1               5                   10


<210>  132
<211>  13
<212>  PRT
<213>  Homo sapiens

<400>  132

Ser Asp Thr Glu Glu Gln Glu Tyr Glu Glu Glu Gln Pro
1               5                   10


<210>  133
<211>  13
<212>  PRT
<213>  Homo sapiens

<400>  133

Lys Ile Tyr Ser Gly Asp Tyr Tyr Arg Gln Gly Cys Ala
1               5                   10


<210>  134
<211>  13
<212>  PRT
<213>  Homo sapiens

<400>  134

Lys Asn Pro Asp Tyr Val Arg Lys Gly Asp Thr Arg Leu
1               5                   10


<210>  135
<211>  13
<212>  PRT
<213>  Homo sapiens

<400>  135

Asp Ile Tyr Lys Asp Pro Asp Tyr Val Arg Lys Gly Asp
1               5                   10


<210>  136
<211>  13
<212>  PRT
<213>  Homo sapiens

<400>  136

Arg Phe Arg Gln Gly Lys Asp Tyr Val Gly Ala Ile Pro
1               5                   10


<210>  137
<211>  13
<212>  PRT
<213>  Homo sapiens

<400>  137

Asp Ile Tyr Lys Asp Pro Asp Tyr Val Arg Lys Gly Ser
1               5                   10

<210>  138
<211>  13
<212>  PRT
<213>  Homo sapiens

<400>  138

Lys Ser Phe Leu Asp Ser Gly Tyr Arg Ile Leu Gly Ala
1               5                   10

<210>  139
<211>  13
<212>  PRT
<213>  Homo sapiens

<400>  139

Ala Leu Gly Ala Asp Asp Ser Tyr Tyr Thr Ala Arg Ser
1               5                   10

<210>  140
<211>  13
<212>  PRT
<213>  Homo sapiens

<400>  140

Leu Arg Thr His Asn Gly Ala Ser Pro Tyr Gln Cys Thr
1               5                   10

<210>  141
<211>  15
<212>  PRT
<213>  Homo sapiens

<400>  141

His Lys Leu Gly Gly Gly Gln Tyr Gly Glu Val Tyr Glu Gly Val
1               5                   10                  15

<210>  142
<211>  15
<212>  PRT
<213>  Homo sapiens

<400>  142

Arg Thr Ser Ser Glu Ser Ile Tyr Ser Arg Pro Gly Ser Ser Ile
1               5                   10                  15

<210>  143
<211>  15
<212>  PRT
<213>  Homo sapiens

<400>  143

Gly Ser Ile Asn Ser Pro Val Tyr Ser Arg His Ser Tyr Thr Pro
1               5                   10                  15

<210>  144
<211>  15
<212>  PRT
<213>  Homo sapiens

<400>  144

Ser Arg Thr Thr Asp Gly Val Tyr Glu Gly Val Ala Ile Gly Gly
1               5                   10                  15


<210>  145
<211>  15
<212>  PRT
<213>  Homo sapiens

<400>  145

Val Thr His Thr Val Pro Ile Tyr Glu Gly Tyr Ala Leu Pro His
1               5                   10                  15


<210>  146
<211>  15
<212>  PRT
<213>  Homo sapiens

<400>  146

Pro Ala Arg Pro Ala Pro Ala Pro Pro Leu Tyr Ser Ser Leu Thr
1               5                   10                  15


<210>  147
<211>  15
<212>  PRT
<213>  Homo sapiens

<400>  147

Pro Asn Gln Phe Val Pro Leu Tyr Thr Asp Pro Gln Glu Val Leu
1               5                   10                  15


<210>  148
<211>  15
<212>  PRT
<213>  Homo sapiens

<400>  148

Lys Gln Thr Ile Pro Asp Pro Tyr Glu Asp Phe Met Tyr Arg His
1               5                   10                  15


<210>  149
<211>  15
<212>  PRT
<213>  Homo sapiens

<400>  149

Arg Lys Asn Leu Glu Ala Thr Tyr Gly Thr Val Arg Thr Gly Asn
1               5                   10                  15


<210>  150
<211>  15

<212>  PRT
<213>  Homo sapiens

<400>  150

Asp Gln Asp Ala Arg Asp Leu Tyr Asp Ala Gly Val Lys Arg Lys
1               5                   10                  15


<210>  151
<211>  15
<212>  PRT
<213>  Homo sapiens

<400>  151

Arg Tyr Lys Ser Tyr Ser Pro Tyr Asp Met Leu Glu Ser Ile Arg
1               5                   10                  15


<210>  152
<211>  15
<212>  PRT
<213>  Homo sapiens

<400>  152

Phe Lys Ala Tyr Gln Phe Tyr Tyr Gly Ser Asn Arg Cys Thr Asn
1               5                   10                  15


<210>  153
<211>  15
<212>  PRT
<213>  Homo sapiens

<400>  153

Ser Lys Met Gln Gln Asn Gly Tyr Glu Asn Pro Thr Tyr Lys Phe
1               5                   10                  15


<210>  154
<211>  15
<212>  PRT
<213>  Homo sapiens

<400>  154

Ala Leu Glu Glu Asp Val Ile Tyr Asp Asp Val Pro Cys Glu Ser
1               5                   10                  15


<210>  155
<211>  15
<212>  PRT
<213>  Homo sapiens

<400>  155

Tyr Gln Thr His Gln Pro Val Tyr His Lys Ile Gln Gly Asp Asp
1               5                   10                  15


<210>  156
<211>  15
<212>  PRT
<213>  Homo sapiens

<400>  156

Ala Pro Gly Pro Gln Asp Ile Tyr Asp Val Pro Pro Val Arg Gly
1               5                   10                  15


<210>  157
<211>  15
<212>  PRT
<213>  Homo sapiens

<400>  157

Pro Leu Leu Arg Glu Glu Thr Tyr Asp Val Pro Pro Ala Phe Ala
1               5                   10                  15


<210>  158
<211>  15
<212>  PRT
<213>  Homo sapiens

<400>  158

Ser Pro Pro Ala Glu Asp Val Tyr Asp Val Pro Pro Pro Ala Pro
1               5                   10                  15


<210>  159
<211>  15
<212>  PRT
<213>  Homo sapiens

<400>  159

Pro Pro Pro Ala Pro Asp Leu Tyr Asp Val Pro Pro Gly Leu Arg
1               5                   10                  15


<210>  160
<211>  15
<212>  PRT
<213>  Homo sapiens

<400>  160

Glu Gly Gly Trp Met Glu Asp Tyr Asp Tyr Val His Leu Gln Gly
1               5                   10                  15


<210>  161
<211>  15
<212>  PRT
<213>  Homo sapiens

<400>  161

Arg Pro Gly Pro Gly Thr Leu Tyr Asp Val Pro Arg Glu Arg Val
1               5                   10                  15


<210>  162
<211>  15
<212>  PRT
<213>  Homo sapiens

<400>  162

Ser Cys Gly Val Asp Gly Asp Tyr Glu Asp Ala Glu Leu Asn Pro

1               5                   10                  15


<210>  163
<211>  15
<212>  PRT
<213>  Homo sapiens

<400>  163

Trp Asn Ser Val Ala Arg Arg Tyr Pro Asn Leu Phe Thr Asn Met
1               5                   10                  15


<210>  164
<211>  15
<212>  PRT
<213>  Homo sapiens

<400>  164

Glu Asp Glu Gly Trp Gln Val Tyr Arg Leu Gly Ala Arg Asp Ala
1               5                   10                  15


<210>  165
<211>  15
<212>  PRT
<213>  Homo sapiens

<400>  165

Val Lys Ser Leu Glu Asp Pro Tyr Ser Gln Gln Ile Arg Leu Gln
1               5                   10                  15


<210>  166
<211>  15
<212>  PRT
<213>  Homo sapiens

<400>  166

Cys Pro Phe Ile Asp Asn Thr Tyr Ser Cys Ser Gly Lys Leu Leu
1               5                   10                  15


<210>  167
<211>  15
<212>  PRT
<213>  Homo sapiens

<400>  167

Asp Glu Ser Gln Gly Glu Ile Tyr Arg Ser Ile Glu His Leu Asp
1               5                   10                  15


<210>  168
<211>  15
<212>  PRT
<213>  Homo sapiens

<400>  168

Arg Ala Tyr Ala Asp Ser Tyr Tyr Tyr Glu Asp Gly Gly Met Lys
1               5                   10                  15

<210>  169
<211>  15
<212>  PRT
<213>  Homo sapiens

<400>  169

Thr Asp Gly Glu Asp Ala Asp Tyr Thr His Phe Thr Asn Gln Gln
1               5                   10                  15


<210>  170
<211>  15
<212>  PRT
<213>  Homo sapiens

<400>  170

Ala Pro Gly Gly Arg His Pro Gly Gly Gly Asp Tyr Glu Glu Leu
1               5                   10                  15


<210>  171
<211>  15
<212>  PRT
<213>  Homo sapiens

<400>  171

Thr Thr Gln Asn Gly Arg Phe Tyr Ala Ile Ser Ala Arg Phe Lys
1               5                   10                  15


<210>  172
<211>  15
<212>  PRT
<213>  Homo sapiens

<400>  172

Val Asp Ser Glu Gly His Leu Tyr Thr Val Pro Ile Arg Glu Gln
1               5                   10                  15


<210>  173
<211>  15
<212>  PRT
<213>  Homo sapiens

<400>  173

Ser Ser Ser Ala Asn Ala Ile Tyr Ser Leu Ala Ala Arg Pro Leu
1               5                   10                  15


<210>  174
<211>  15
<212>  PRT
<213>  Homo sapiens

<400>  174

Thr Asn Arg Thr Ser Gln Asp Tyr Asp Gln Leu Pro Ser Cys Ser
1               5                   10                  15


<210>  175
<211>  15
<212>  PRT

<213>  Homo sapiens

<400>  175

Glu Gly Thr Tyr Gly Val Val Tyr Lys Ala Lys Asn Arg Glu Thr
1               5                   10                  15


<210>  176
<211>  15
<212>  PRT
<213>  Homo sapiens

<400>  176

Met Arg Glu Ser Glu Gly Leu Tyr Gly Gln Pro Gly Asp Glu Thr
1               5                   10                  15


<210>  177
<211>  15
<212>  PRT
<213>  Homo sapiens

<400>  177

Gly Gln Thr Val Asp Asp Pro Tyr Ala Thr Phe Val Lys Met Leu
1               5                   10                  15


<210>  178
<211>  15
<212>  PRT
<213>  Homo sapiens

<400>  178

Arg Pro Tyr Pro Lys Pro Ala Pro Ser Ser Gly Lys Asp Tyr Val
1               5                   10                  15


<210>  179
<211>  15
<212>  PRT
<213>  Homo sapiens

<400>  179

Ser Gln Arg Asn Arg Ser Asn Tyr Tyr Asp Ala Tyr Gln Ala Gln
1               5                   10                  15


<210>  180
<211>  15
<212>  PRT
<213>  Homo sapiens

<400>  180

Ser Arg His Tyr Glu Asp Gly Tyr Pro Gly Gly Ser Asp Asn Tyr
1               5                   10                  15


<210>  181
<211>  15
<212>  PRT
<213>  Homo sapiens

<400>  181

Arg Leu Pro Ser Ser Pro Val Tyr Glu Asp Ala Ala Ser Phe Lys
1               5                   10                  15


<210>  182
<211>  15
<212>  PRT
<213>  Homo sapiens

<400>  182

Glu Gly Thr Tyr Gly Val Val Tyr Lys Gly Arg His Lys Thr Thr
1               5                   10                  15


<210>  183
<211>  15
<212>  PRT
<213>  Homo sapiens

<400>  183

Lys Lys Lys Thr Glu Gly Thr Tyr Asp Leu Pro Tyr Trp Asp Arg
1               5                   10                  15


<210>  184
<211>  15
<212>  PRT
<213>  Homo sapiens

<400>  184

Pro Ala Pro Asp Glu Leu Val Tyr Gln Val Pro Gln Ser Thr Gln
1               5                   10                  15


<210>  185
<211>  15
<212>  PRT
<213>  Homo sapiens

<400>  185

Arg Tyr Gly Arg Arg Lys Gln Tyr Pro Ile Ser Leu Val Leu Ala
1               5                   10                  15


<210>  186
<211>  15
<212>  PRT
<213>  Homo sapiens

<400>  186

Arg Leu Lys Ser Thr Cys Ile Tyr Gly Gly Ala Pro Lys Gly Pro
1               5                   10                  15


<210>  187
<211>  15
<212>  PRT
<213>  Homo sapiens

<400>  187

Arg Val Ser Thr Asp Leu Lys Tyr Arg Lys Gln Pro Trp Gly Leu
1               5                   10                  15

<210>  188
<211>  15
<212>  PRT
<213>  Homo sapiens

<400>  188

Ala Thr Gln Pro Glu Pro Ile Tyr Ala Glu Ser Thr Lys Arg Lys
1               5                   10                  15


<210>  189
<211>  15
<212>  PRT
<213>  Homo sapiens

<400>  189

Cys Pro Ser Gln Asp Ser Leu Tyr Ser Asp Pro Leu Asp Ser Thr
1               5                   10                  15


<210>  190
<211>  15
<212>  PRT
<213>  Homo sapiens

<400>  190

Tyr Asn Pro Ala Thr Asp Asp Tyr Ala Val Pro Pro Pro Arg Ser
1               5                   10                  15


<210>  191
<211>  15
<212>  PRT
<213>  Homo sapiens

<400>  191

Glu Asn Asn Tyr Cys Pro His Tyr Glu Lys Val Ser Gly Asp Tyr
1               5                   10                  15


<210>  192
<211>  15
<212>  PRT
<213>  Homo sapiens

<400>  192

Thr Leu Asp Thr Leu Ser Ile Tyr Glu Thr Pro Ser Met Gly Leu
1               5                   10                  15


<210>  193
<211>  15
<212>  PRT
<213>  Homo sapiens

<400>  193

Trp Ser Leu Pro Asn Pro Glu Tyr Tyr Thr Leu Arg Tyr Ala Asp
1               5                   10                  15


<210>  194

<211>  15
<212>  PRT
<213>  Homo sapiens

<400>  194

Ile Pro Lys Glu Pro Ser Ser Tyr Asp Phe Val Tyr His Tyr Gly
1               5                   10                  15


<210>  195

<400>  195
000

<210>  196
<211>  15
<212>  PRT
<213>  Homo sapiens

<400>  196

Asp Asp Thr Ser Asp Pro Thr Tyr Thr Ser Ala Leu Gly Gly Lys
1               5                   10                  15


<210>  197
<211>  15
<212>  PRT
<213>  Homo sapiens

<400>  197

Val Tyr Ile Asp Pro Phe Thr Tyr Glu Asp Pro Asn Glu Ala Val
1               5                   10                  15


<210>  198
<211>  15
<212>  PRT
<213>  Homo sapiens

<400>  198

Glu Asn Ser Ser Asp Pro Thr Tyr Thr Ser Ser Leu Gly Gly Lys
1               5                   10                  15


<210>  199
<211>  15
<212>  PRT
<213>  Homo sapiens

<400>  199

Pro Thr Ala Glu Asn Pro Glu Tyr Leu Gly Leu Asp Val Pro Val
1               5                   10                  15


<210>  200
<211>  15
<212>  PRT
<213>  Homo sapiens

<400>  200

Glu Glu Glu Asp Val Asn Gly Tyr Val Met Pro Asp Thr His Leu
1               5                   10                  15

<210>  201
<211>  15
<212>  PRT
<213>  Homo sapiens

<400>  201

Lys Val Ser Ser Thr His Tyr Tyr Leu Leu Pro Glu Arg Pro Pro
1               5                   10                  15


<210>  202
<211>  15
<212>  PRT
<213>  Homo sapiens

<400>  202

Thr Ser Ser Lys Lys Val Ile Tyr Ser Gln Pro Ser Ala Arg Ser
1               5                   10                  15


<210>  203
<211>  15
<212>  PRT
<213>  Homo sapiens

<400>  203

Ile Ile Asp Glu Glu Asp Thr Tyr Thr Met Pro Ser Thr Arg Asp
1               5                   10                  15


<210>  204
<211>  15
<212>  PRT
<213>  Homo sapiens

<400>  204

Pro Ile Gly Asn Gln His Ile Tyr Gln Pro Val Gly Lys Pro Asp
1               5                   10                  15


<210>  205
<211>  15
<212>  PRT
<213>  Homo sapiens

<400>  205

Asp Arg Ser Asn Asp Lys Val Tyr Glu Asn Val Thr Gly Leu Val
1               5                   10                  15


<210>  206
<211>  15
<212>  PRT
<213>  Homo sapiens

<400>  206

Glu Tyr Glu Asn Ile Arg His Tyr Glu Glu Ile Pro Glu Tyr Glu
1               5                   10                  15


<210>  207
<211>  15

<212>  PRT
<213>  Homo sapiens

<400>  207

Gly Thr Gly Glu Asp Asp Asp Tyr Val Asp Asp Phe Asn Ser Thr
1               5                   10                  15


<210>  208
<211>  10
<212>  PRT
<213>  Homo sapiens

<400>  208

Glu Phe Gly Thr Tyr Gly Thr Leu Ser Lys
1               5                   10


<210>  209
<211>  15
<212>  PRT
<213>  Homo sapiens

<400>  209

Gly Asp Val Ser Gln Phe Pro Tyr Val Glu Phe Thr Gly Arg Asp
1               5                   10                  15


<210>  210
<211>  15
<212>  PRT
<213>  Homo sapiens

<400>  210

Arg His Gly Glu Gln Ser Leu Tyr Ser Pro Gln Thr Pro Ala Tyr
1               5                   10                  15


<210>  211
<211>  15
<212>  PRT
<213>  Homo sapiens

<400>  211

Leu Asn Gly Ala Glu Pro Asn Tyr His Ser Leu Pro Ser Ala Arg
1               5                   10                  15


<210>  212
<211>  15
<212>  PRT
<213>  Homo sapiens

<400>  212

Pro Pro Asp Tyr Asn Ser Val Val Leu Tyr Ser Thr Pro Pro Ile
1               5                   10                  15


<210>  213
<211>  15
<212>  PRT
<213>  Homo sapiens

<400>  213

Arg Lys Leu Asp Asn Gly Gly Tyr Tyr Ile Thr Thr Arg Ala Gln
1               5                   10                  15


<210>  214
<211>  15
<212>  PRT
<213>  Homo sapiens

<400>  214

Ala Ser Val Asp Ser Ser Leu Tyr Asn Leu Pro Arg Ser Tyr Ser
1               5                   10                  15


<210>  215
<211>  15
<212>  PRT
<213>  Homo sapiens

<400>  215

Gln Gln Asn Thr Asp Leu Ile Tyr Gln Thr Gly Pro Lys Ser Thr
1               5                   10                  15


<210>  216
<211>  15
<212>  PRT
<213>  Homo sapiens

<400>  216

Lys Val Pro Ser Glu Gly Ala Tyr Asp Ile Ile Leu Pro Arg Ala
1               5                   10                  15


<210>  217
<211>  15
<212>  PRT
<213>  Homo sapiens

<400>  217

Asp Gly Phe Asp Pro Ser Asp Tyr Ala Glu Pro Met Asp Ala Val
1               5                   10                  15


<210>  218
<211>  15
<212>  PRT
<213>  Homo sapiens

<400>  218

Pro Gln Gly Pro Pro Glu Ile Tyr Ser Asp Thr Gln Phe Pro Ser
1               5                   10                  15


<210>  219
<211>  15
<212>  PRT
<213>  Homo sapiens

<400>  219

Lys Ser Glu Val Asp Glu Thr Tyr Ala Leu Pro Ala Thr Lys Ile

1               5                   10                  15


<210>  220
<211>  15
<212>  PRT
<213>  Homo sapiens

<400>  220

Arg Val Ile Glu Asp Asn Glu Tyr Thr Ala Arg Glu Gly Ala Lys
1               5                   10                  15


<210>  221
<211>  11
<212>  PRT
<213>  Homo sapiens

<400>  221

Ala Ala Glu Glu Ile Tyr Ala Ala Arg Arg Gly
1               5                   10


<210>  222
<211>  15
<212>  PRT
<213>  Homo sapiens

<400>  222

Ser Arg Asn Met Gly Gly Pro Tyr Gly Gly Gly Asn Tyr Gly Pro
1               5                   10                  15


<210>  223
<211>  15
<212>  PRT
<213>  Homo sapiens

<400>  223

Lys Ala Thr Glu Asn Asp Ile Tyr Asn Phe Phe Ser Pro Leu Asn
1               5                   10                  15


<210>  224
<211>  15
<212>  PRT
<213>  Homo sapiens

<400>  224

Gly Met Asp Asn Gln Gly Gly Tyr Gly Ser Val Gly Arg Met Gly
1               5                   10                  15


<210>  225
<211>  15
<212>  PRT
<213>  Homo sapiens

<400>  225

Gly Gln Arg Gln Ala Leu Asp Tyr Val Glu Leu Ser Pro Leu Thr
1               5                   10                  15

<210>  226
<211>  15
<212>  PRT
<213>  Homo sapiens

<400>  226

Ser Thr Ser Pro Glu Ser Pro Tyr Thr His Trp Lys Gln Thr Val
1               5                   10                  15


<210>  227
<211>  15
<212>  PRT
<213>  Homo sapiens

<400>  227

Asp Asp Ile Thr Gln Glu Glu Tyr Gly Glu Phe Tyr Lys Ser Leu
1               5                   10                  15


<210>  228
<211>  15
<212>  PRT
<213>  Homo sapiens

<400>  228

Lys Glu Thr Gln Lys Ser Ile Tyr Tyr Ile Thr Gly Glu Ser Lys
1               5                   10                  15


<210>  229
<211>  15
<212>  PRT
<213>  Homo sapiens

<400>  229

Gln Ser Tyr Asn Gln Ser Pro Tyr Ser Asn Tyr Gly Pro Pro Gln
1               5                   10                  15


<210>  230
<211>  15
<212>  PRT
<213>  Homo sapiens

<400>  230

Asp Ile Tyr Glu Thr Asp Tyr Tyr Arg Lys Gly Gly Lys Gly Leu
1               5                   10                  15


<210>  231
<211>  15
<212>  PRT
<213>  Homo sapiens

<400>  231

Gln Arg Val Asp Pro Asn Gly Tyr Met Met Met Ser Pro Ser Gly
1               5                   10                  15


<210>  232
<211>  15
<212>  PRT

<213>  Homo sapiens

<400>  232

Cys Gly Gly Asp Ser Asp Gln Tyr Val Leu Met Ser Ser Pro Val
1               5                   10                  15


<210>  233
<211>  15
<212>  PRT
<213>  Homo sapiens

<400>  233

Gly Ala Gln Gly Glu Gly Pro Tyr Ser Ser Leu Val Ser Cys Arg
1               5                   10                  15


<210>  234
<211>  15
<212>  PRT
<213>  Homo sapiens

<400>  234

Arg Glu Glu Pro Glu Ala Leu Tyr Ala Ala Val Asn Lys Lys Pro
1               5                   10                  15


<210>  235
<211>  15
<212>  PRT
<213>  Homo sapiens

<400>  235

Val Arg Arg Glu Val Gly Asp Tyr Gly Gln Leu His Glu Thr Glu
1               5                   10                  15


<210>  236
<211>  15
<212>  PRT
<213>  Homo sapiens

<400>  236

Asp Ile Tyr Lys Asp Pro Asp Tyr Val Arg Lys Gly Asp Ala Arg
1               5                   10                  15


<210>  237
<211>  15
<212>  PRT
<213>  Homo sapiens

<400>  237

Glu Glu Ala Pro Glu Asp Leu Tyr Lys Asp Phe Leu Thr Leu Glu
1               5                   10                  15


<210>  238
<211>  15
<212>  PRT
<213>  Homo sapiens

<400>  238

Pro Asn Pro Cys Ser Ala Thr Tyr Ser Asn Leu Gly Gln Ser Arg
1               5                   10                  15


<210>  239
<211>  15
<212>  PRT
<213>  Homo sapiens

<400>  239

Ala Tyr Asp Asn Leu Ala Ile Tyr Lys Ser Phe Leu Gly Thr Ser
1               5                   10                  15


<210>  240
<211>  15
<212>  PRT
<213>  Homo sapiens

<400>  240

Thr Arg Val Met Lys Ala Ile Tyr Ser Ser Phe Lys Asp Asp Val
1               5                   10                  15


<210>  241
<211>  15
<212>  PRT
<213>  Homo sapiens

<400>  241

Ser Gly Leu Glu Arg Thr Pro Tyr Glu Ala Tyr Asp Pro Ile Gly
1               5                   10                  15


<210>  242
<211>  15
<212>  PRT
<213>  Homo sapiens

<400>  242

Asp His Ala Glu Ala Ala Leu Tyr Lys Asn Leu Leu His Ser Lys
1               5                   10                  15


<210>  243
<211>  15
<212>  PRT
<213>  Homo sapiens

<400>  243

Cys Ser Asp Ser Thr Asn Glu Tyr Met Asp Met Lys Pro Gly Val
1               5                   10                  15


<210>  244
<211>  15
<212>  PRT
<213>  Homo sapiens

<400>  244

Ser Glu Ser Thr Asn His Ile Tyr Ser Asn Leu Ala Asn Cys Ser
1               5                   10                  15

<210>  245
<211>  15
<212>  PRT
<213>  Homo sapiens

<400>  245

Ser Gly Gly Tyr Gly Gly Gly Tyr Gly Gly Val Leu Thr Ala Ser
1               5                   10                  15


<210>  246
<211>  15
<212>  PRT
<213>  Homo sapiens

<400>  246

Glu Asp Glu Glu Ser Glu Asp Tyr Gln Asn Ser Ala Ser Ile His
1               5                   10                  15


<210>  247
<211>  15
<212>  PRT
<213>  Homo sapiens

<400>  247

Lys Met Val Val Glu Ser Ala Tyr Glu Val Ile Lys Leu Lys Gly
1               5                   10                  15


<210>  248
<211>  15
<212>  PRT
<213>  Homo sapiens

<400>  248

Ser Ile Tyr Ala Pro Ser Thr Tyr Ala His Leu Ser Pro Ala Lys
1               5                   10                  15


<210>  249
<211>  15
<212>  PRT
<213>  Homo sapiens

<400>  249

Lys Val Gly Cys Phe Asp Pro Tyr Ser Asp Asp Pro Arg Leu Gly
1               5                   10                  15


<210>  250
<211>  15
<212>  PRT
<213>  Homo sapiens

<400>  250

Arg Ser Trp Ala Ser Pro Val Tyr Thr Glu Ala Asp Gly Thr Phe
1               5                   10                  15


<210>  251

<211>  15
<212>  PRT
<213>  Homo sapiens

<400>  251

Ile Asp Ala Thr Ser Gly Ile Tyr Asn Ser Glu Lys Ser Ser Asn
1               5                   10                  15


<210>  252
<211>  15
<212>  PRT
<213>  Homo sapiens

<400>  252

Arg Ser Glu Asn Glu Asp Ile Tyr Tyr Lys Ser Met Pro Asn Leu
1               5                   10                  15


<210>  253
<211>  15
<212>  PRT
<213>  Homo sapiens

<400>  253

Arg Gly Gly Met Asp Tyr Ala Tyr Ile Pro Pro Pro Gly Leu Gln
1               5                   10                  15


<210>  254
<211>  15
<212>  PRT
<213>  Homo sapiens

<400>  254

Gly Arg Tyr Tyr Glu Gly Tyr Tyr Ala Ala Gly Pro Gly Tyr Gly
1               5                   10                  15


<210>  255
<211>  15
<212>  PRT
<213>  Homo sapiens

<400>  255

Arg Ser Leu Asp Asn Gly Gly Tyr Tyr Ile Ser Pro Arg Ile Thr
1               5                   10                  15


<210>  256
<211>  15
<212>  PRT
<213>  Homo sapiens

<400>  256

Asp Asp Phe Tyr Thr Ala Thr Glu Gly Gln Tyr Gln Gln Gln Pro
1               5                   10                  15


<210>  257
<211>  15
<212>  PRT
<213>  Homo sapiens

<400>  257

Pro Asp Glu Glu Asp Tyr Asp Tyr Glu Ser Tyr Glu Lys Thr Thr
1               5                   10                  15


<210>  258
<211>  15
<212>  PRT
<213>  Homo sapiens

<400>  258

Thr Ser Phe Met Met Thr Pro Tyr Val Val Thr Arg Tyr Tyr Arg
1               5                   10                  15


<210>  259
<211>  15
<212>  PRT
<213>  Homo sapiens

<400>  259

Ser Leu Asn Ser Glu Asn Ser Tyr Val Ser Pro Arg Ile Leu Thr
1               5                   10                  15


<210>  260
<211>  15
<212>  PRT
<213>  Homo sapiens

<400>  260

Ser Arg Asn Arg Asp Arg Arg Tyr Asp Glu Val Pro Ser Asp Leu
1               5                   10                  15


<210>  261
<211>  15
<212>  PRT
<213>  Homo sapiens

<400>  261

Gly Tyr Tyr Asp Arg Met Asp Tyr Glu Asp Asp Arg Leu Arg Asp
1               5                   10                  15


<210>  262
<211>  15
<212>  PRT
<213>  Homo sapiens

<400>  262

Lys Ala Asp Gly Gly Ala Glu Tyr Ala Thr Tyr Gln Thr Lys Ser
1               5                   10                  15


<210>  263
<211>  15
<212>  PRT
<213>  Homo sapiens

<400>  263

Phe Gly Leu Ala Arg Asp Met Tyr Asp Lys Glu Tyr Tyr Ser Val
1               5                   10                  15


<210>  264
<211>  15
<212>  PRT
<213>  Homo sapiens

<400>  264

Pro Thr Arg Pro Val Pro Ser Tyr Ile Gln Arg Pro Asp Tyr Ala
1               5                   10                  15


<210>  265
<211>  15
<212>  PRT
<213>  Homo sapiens

<400>  265

Thr Gly Lys Leu Arg Pro His Tyr Pro Leu Met Pro Thr Arg Pro
1               5                   10                  15


<210>  266
<211>  15
<212>  PRT
<213>  Homo sapiens

<400>  266

Thr Ala Ala Asp Glu Glu Glu Asp Glu Tyr Ser Gly Gly Leu Cys
1               5                   10                  15


<210>  267
<211>  15
<212>  PRT
<213>  Homo sapiens

<400>  267

Tyr Thr Ile Gly Thr Gly Ser Tyr Gly Arg Cys Gln Lys Ile Arg
1               5                   10                  15


<210>  268
<211>  15
<212>  PRT
<213>  Homo sapiens

<400>  268

Phe Gly Lys Phe Asp Gln Ile Tyr Asp Val Gly Tyr Gln Tyr Gly
1               5                   10                  15


<210>  269
<211>  15
<212>  PRT
<213>  Homo sapiens

<400>  269

Arg Arg Leu Leu Lys Pro Ala Tyr Gly Thr Ser Gly Gly Leu Pro
1               5                   10                  15

<210>  270
<211>  15
<212>  PRT
<213>  Homo sapiens

<400>  270

Ile Val Ala Thr Lys Pro Leu Tyr Val Ala Leu Ala Gln Arg Lys
1               5                   10                  15


<210>  271
<211>  15
<212>  PRT
<213>  Homo sapiens

<400>  271

Leu Thr Val Pro Glu Ser Thr Tyr Thr Ser Ile Gln Gly Asp Pro
1               5                   10                  15


<210>  272
<211>  15
<212>  PRT
<213>  Homo sapiens

<400>  272

Pro Ser Ser Cys Asn Asp Leu Tyr Ala Thr Val Lys Asp Phe Glu
1               5                   10                  15


<210>  273
<211>  15
<212>  PRT
<213>  Homo sapiens

<400>  273

Leu Val Pro Lys Glu Asn Asp Tyr Glu Ser Ile Ser Asp Leu Gln
1               5                   10                  15


<210>  274
<211>  15
<212>  PRT
<213>  Homo sapiens

<400>  274

Ile Gly Gly Ser Ala Pro Ile Tyr Val Lys Asn Ile Leu Pro Arg
1               5                   10                  15


<210>  275
<211>  15
<212>  PRT
<213>  Homo sapiens

<400>  275

Asn Ser Ser Gly Lys Thr Glu Tyr Met Ala Phe Pro Lys Pro Phe
1               5                   10                  15


<210>  276
<211>  15

<212>  PRT
<213>  Homo sapiens

<400>  276

Glu Lys Leu Gly Glu Gly Thr Tyr Ala Thr Val Tyr Lys Gly Arg
1               5                   10                  15


<210>  277
<211>  15
<212>  PRT
<213>  Homo sapiens

<400>  277

Pro Asp Gly His Glu Tyr Ile Tyr Val Asp Pro Met Gln Leu Pro
1               5                   10                  15


<210>  278
<211>  15
<212>  PRT
<213>  Homo sapiens

<400>  278

Leu Tyr Asp Arg Pro Ala Ser Tyr Lys Lys Lys Ser Met Leu Asp
1               5                   10                  15


<210>  279
<211>  15
<212>  PRT
<213>  Homo sapiens

<400>  279

Pro Asn Glu Gly Asp Asn Asp Tyr Ile Ile Pro Leu Pro Asp Pro
1               5                   10                  15


<210>  280

<400>  280
000

<210>  281
<211>  15
<212>  PRT
<213>  Homo sapiens

<400>  281

Ser Ala Thr Lys Ile Ala Leu Tyr Glu Thr Pro Thr Gly Trp Lys
1               5                   10                  15


<210>  282
<211>  15
<212>  PRT
<213>  Homo sapiens

<400>  282

Leu Arg Lys Thr Arg Asp Gln Tyr Leu Met Trp Leu Thr Gln Lys
1               5                   10                  15

<210>  283
<211>  15
<212>  PRT
<213>  Homo sapiens

<400>  283

Lys Asn Glu Thr Glu Asp Gln Tyr Ala Leu Met Glu Asp Glu Asp
1               5                   10                  15


<210>  284
<211>  15
<212>  PRT
<213>  Homo sapiens

<400>  284

Ala Leu Asn Thr Thr Ala Thr Tyr Ala Glu Pro Tyr Arg Pro Ile
1               5                   10                  15


<210>  285
<211>  15
<212>  PRT
<213>  Homo sapiens

<400>  285

Glu Lys Phe Thr Ile His Asn Tyr Ser His Ser Arg Glu Arg Pro
1               5                   10                  15


<210>  286
<211>  15
<212>  PRT
<213>  Homo sapiens

<400>  286

Arg Ala Arg Gln Glu Glu Leu Tyr Ser Glu Leu Gln Ala Arg Glu
1               5                   10                  15


<210>  287
<211>  15
<212>  PRT
<213>  Homo sapiens

<400>  287

Pro Pro Arg Ser Glu Asp Ile Tyr Ala Asp Pro Ala Ala Tyr Val
1               5                   10                  15


<210>  288
<211>  15
<212>  PRT
<213>  Homo sapiens

<400>  288

Ser Leu Ser Phe Pro Glu Asn Tyr Gln Thr Leu Pro Lys Ser Thr
1               5                   10                  15


<210>  289
<211>  15
<212>  PRT

<213>  Homo sapiens

<400>  289

Ser Ser Glu Pro Val Gly Ile Tyr Gln Gly Phe Glu Lys Lys Thr
1               5                   10                  15


<210>  290
<211>  15
<212>  PRT
<213>  Homo sapiens

<400>  290

Met Gln Asp Asn Ser Gly Thr Tyr Gly Lys Ile Trp Glu Gly Ser
1               5                   10                  15


<210>  291
<211>  15
<212>  PRT
<213>  Homo sapiens

<400>  291

Thr Lys His Ile Gly Leu Val Tyr Ser Gly Met Gly Pro Asp Tyr
1               5                   10                  15


<210>  292
<211>  15
<212>  PRT
<213>  Homo sapiens

<400>  292

Gly Asp Phe Gly Leu Ser Arg Tyr Met Glu Asp Ser Thr Tyr Tyr
1               5                   10                  15


<210>  293
<211>  15
<212>  PRT
<213>  Homo sapiens

<400>  293

Glu Leu Thr Ala Asp Phe Leu Tyr Glu Glu Val His Pro Lys Gln
1               5                   10                  15


<210>  294
<211>  15
<212>  PRT
<213>  Homo sapiens

<400>  294

Arg Glu Asp Ser Ala Arg Val Tyr Glu Asn Val Gly Leu Met Gln
1               5                   10                  15


<210>  295
<211>  15
<212>  PRT
<213>  Homo sapiens

<400>  295

Val Pro Ala Ser Asp Asp Ala Tyr Pro Glu Ile Glu Lys Phe Phe
1               5                   10                  15

<210>  296
<211>  15
<212>  PRT
<213>  Homo sapiens

<400>  296

Ser Arg Pro Gly Gly Tyr Gly Tyr Gly Tyr Gly Arg Ser Arg Asp
1               5                   10                  15

<210>  297
<211>  15
<212>  PRT
<213>  Homo sapiens

<400>  297

Tyr Ala Pro Pro Pro Arg Asp Tyr Thr Tyr Arg Asp Tyr Gly His
1               5                   10                  15

<210>  298
<211>  15
<212>  PRT
<213>  Homo sapiens

<400>  298

Arg Tyr Pro Asn Asp Ser Val Tyr Ala Asn Trp Met Leu Ser Pro
1               5                   10                  15

<210>  299
<211>  15
<212>  PRT
<213>  Homo sapiens

<400>  299

Gln Tyr Asp Asn Leu Glu Asp Tyr His Ser Leu Pro Gln His Gln
1               5                   10                  15

<210>  300
<211>  15
<212>  PRT
<213>  Homo sapiens

<400>  300

Ser Arg Lys Ala Gln Asp Cys Tyr Phe Met Lys Leu His His Cys
1               5                   10                  15

<210>  301
<211>  15
<212>  PRT
<213>  Homo sapiens

<400>  301

Ala Lys Leu Glu His Leu Ile Tyr Leu Lys Asn Arg Leu Ser Ser
1               5                   10                  15

<210>  302
<211>  15
<212>  PRT
<213>  Homo sapiens

<400>  302

Glu Pro Lys Asn Tyr Gly Ser Tyr Ser Thr Gln Ala Ser Ala Ala
1               5                   10                  15


<210>  303
<211>  15
<212>  PRT
<213>  Homo sapiens

<400>  303

Gln Pro Asp Thr Glu Glu Glu Tyr Glu Asp Ser Ser Gly Asn Val
1               5                   10                  15


<210>  304
<211>  15
<212>  PRT
<213>  Homo sapiens

<400>  304

Gly Gln Met Pro Glu Asn Pro Tyr Ser Glu Val Gly Lys Ile Ala
1               5                   10                  15


<210>  305
<211>  15
<212>  PRT
<213>  Homo sapiens

<400>  305

Lys Val Thr Ile Ala Asp Asp Tyr Ser Asp Pro Phe Asp Ala Lys
1               5                   10                  15


<210>  306
<211>  15
<212>  PRT
<213>  Homo sapiens

<400>  306

Glu Pro Pro Asp His Gln Tyr Tyr Asn Asp Phe Pro Gly Lys Glu
1               5                   10                  15


<210>  307
<211>  15
<212>  PRT
<213>  Homo sapiens

<400>  307

Ser Thr Pro Gly Ser Ala Thr Tyr Val Arg Tyr Lys Pro Ser Pro
1               5                   10                  15


<210>  308

<211>  15
<212>  PRT
<213>  Homo sapiens

<400>  308

Lys Pro Ala Gln Glu Ser Asn Tyr Arg Leu Leu Arg Arg Asn Asn
1               5                   10                  15


<210>  309
<211>  15
<212>  PRT
<213>  Homo sapiens

<400>  309

Ile Tyr Arg Lys Glu Pro Leu Tyr Ala Ala Phe Pro Gly Ser His
1               5                   10                  15


<210>  310
<211>  15
<212>  PRT
<213>  Homo sapiens

<400>  310

Arg Ser Gly Lys Pro Arg Gly Tyr Ala Phe Ile Glu Tyr Glu His
1               5                   10                  15


<210>  311
<211>  15
<212>  PRT
<213>  Homo sapiens

<400>  311

Pro Gln Asn Leu Asn Asp Ala Tyr Gly Pro Pro Ser Asn Phe Leu
1               5                   10                  15


<210>  312
<211>  15
<212>  PRT
<213>  Homo sapiens

<400>  312

Arg Ala Glu Pro Lys Ser Ile Tyr Glu Tyr Gln Pro Gly Lys Ser
1               5                   10                  15


<210>  313
<211>  15
<212>  PRT
<213>  Homo sapiens

<400>  313

Ser Lys Ser Gln Thr Asp Val Tyr Asn Asp Ser Thr Asn Leu Ala
1               5                   10                  15


<210>  314
<211>  15
<212>  PRT
<213>  Homo sapiens

<400>  314

Lys His Asp Tyr Asn Tyr Pro Tyr Val Asp Ser Ser Asp Phe Gly
1               5                   10                  15


<210>  315
<211>  15
<212>  PRT
<213>  Homo sapiens

<400>  315

Thr Ser His Val Glu Asn Asp Tyr Ile Asp Asn Pro Ser Leu Ala
1               5                   10                  15


<210>  316
<211>  15
<212>  PRT
<213>  Homo sapiens

<400>  316

Lys Leu Asp Ser Gly Gly Phe Tyr Ile Thr Ser Arg Thr Gln Phe
1               5                   10                  15


<210>  317
<211>  15
<212>  PRT
<213>  Homo sapiens

<400>  317

Glu Asp Pro Met Tyr Ser Met Tyr Ala Lys Leu Gln Asn Gln Pro
1               5                   10                  15


<210>  318
<211>  15
<212>  PRT
<213>  Homo sapiens

<400>  318

His Thr Glu Thr Lys Ser Leu Tyr Pro Ser Ser Glu Ile Gln Leu
1               5                   10                  15


<210>  319
<211>  15
<212>  PRT
<213>  Homo sapiens

<400>  319

Leu Val Asn Glu Ala Pro Val Tyr Ser Val Tyr Ser Lys Leu His
1               5                   10                  15


<210>  320
<211>  15
<212>  PRT
<213>  Homo sapiens

<400>  320

Ala Asp Pro Gly Ala Ala Pro Tyr Leu Lys Thr Lys Phe Ile Cys
1               5                   10                  15


<210>  321
<211>  15
<212>  PRT
<213>  Homo sapiens

<400>  321

Gly Pro Leu Asp Gly Ser Pro Tyr Ala Gln Val Gln Arg Pro Pro
1               5                   10                  15


<210>  322
<211>  15
<212>  PRT
<213>  Homo sapiens

<400>  322

Lys Leu Lys Pro Met Leu Ile Tyr His Ser Glu Asn Pro Arg Ala
1               5                   10                  15


<210>  323
<211>  15
<212>  PRT
<213>  Homo sapiens

<400>  323

Ala Gln Lys His Pro Asp Ile Tyr Ala Val Pro Ile Lys Thr His
1               5                   10                  15


<210>  324
<211>  15
<212>  PRT
<213>  Homo sapiens

<400>  324

Ser Pro Glu Asp Glu Ala Ile Tyr Gly Pro Asn Thr Lys Met Val
1               5                   10                  15


<210>  325
<211>  15
<212>  PRT
<213>  Homo sapiens

<400>  325

Arg Glu Ser Ser Tyr Asp Ile Tyr Arg Val Pro Ser Ser Gln Ser
1               5                   10                  15


<210>  326
<211>  15
<212>  PRT
<213>  Homo sapiens

<400>  326

Met Glu Gly Pro Pro Pro Thr Tyr Ser Glu Val Ile Gly His Tyr
1               5                   10                  15

<210>  327
<211>  15
<212>  PRT
<213>  Homo sapiens

<400>  327

Gly Pro Val Asp Gly Ser Leu Tyr Ala Lys Val Arg Lys Lys Ser
1               5                   10                  15


<210>  328
<211>  15
<212>  PRT
<213>  Homo sapiens

<400>  328

Lys Val Val Tyr Glu Asn Ala Tyr Gly Gln Phe Ile Gly Pro His
1               5                   10                  15


<210>  329
<211>  15
<212>  PRT
<213>  Homo sapiens

<400>  329

Asn Lys Gly Lys Glu Lys Ile Tyr Ser Ala Glu Arg Phe Leu Ile
1               5                   10                  15


<210>  330
<211>  15
<212>  PRT
<213>  Homo sapiens

<400>  330

Met Glu Thr Arg Tyr Asn Leu Lys Ser Pro Ala Val Lys Arg Leu
1               5                   10                  15


<210>  331
<211>  15
<212>  PRT
<213>  Homo sapiens

<400>  331

Ala Phe Ser Arg Val Gln Ile Tyr His Asn Pro Thr Ala Asn Ser
1               5                   10                  15


<210>  332
<211>  15
<212>  PRT
<213>  Homo sapiens

<400>  332

Thr Glu Asn Asp Asp Asp Val Tyr Arg Ser Leu Glu Glu Leu Ala
1               5                   10                  15


<210>  333
<211>  15

<212>  PRT
<213>  Homo sapiens

<400>  333

Ala Arg Gly Gly Gln Val Phe Tyr Asn Ser Glu Tyr Gly Glu Leu
1               5                   10                  15


<210>  334
<211>  15
<212>  PRT
<213>  Homo sapiens

<400>  334

Gln Val Phe Tyr Asn Ser Glu Tyr Gly Glu Leu Ser Glu Pro Ser
1               5                   10                  15


<210>  335

<400>  335
000

<210>  336
<211>  15
<212>  PRT
<213>  Homo sapiens

<400>  336

Gly Ser Ser Trp Glu Ala Pro Tyr Ser Arg Ser Lys Leu Arg Pro
1               5                   10                  15


<210>  337
<211>  15
<212>  PRT
<213>  Homo sapiens

<400>  337

Gly Val Val Gly Gly Glu Asp Tyr Glu Glu Val Asp Arg Tyr Ser
1               5                   10                  15

## Description

### FIELD OF THE INVENTION

The present invention is concerned with a method for pharmacologically profiling compounds using an array of substrates, in particular kinase substrates, immobilized on a porous matrix. More particularly, a method useful in the prediction of drug response, i.e. to enable the distinction between responders and non-responders in the treatment of cells, tissues, organs or warm-blooded animals with the compound to be tested, and in compound differentiation, is disclosed.

### BACKGROUND OF THE INVENTION

The cost of drug discovery and development is increasing, while the rate of new drug approvals is declining. In contrast to major technological advances with in silico and in vitro primary screening tools, there are only limited advances in the tools available for establishing the actions of agents in the complex biochemical networks characteristic of fully assembled living systems. Recent advances in genomics and proteomic technologies have begun to address this challenge by providing the tools that allow analyzing variations in gene transcription and protein expression at the protein level, in samples of high biological complexity. For instance, genomic and proteomic signatures have provided insights into the molecular mechanisms of a range of physiological and pathological processes, such as for example the pathology of cancer (Vogelstein, B., Kinzler, K.W., Nat. Med. 10(8), 789-99 (2004)), including tumor invasion (Gupta, G.P., et al. Cold Spring Harb Symp Quant Biol. 70:149-58 (2005)).

However, the general application of these methods to establish the actions of agents in complex biological systems, is hampered by the given that these methods rely on changes in protein abundance to deduce their role in cellular processes and, therefore, provide only an indirect estimate of dynamics in protein function. Indeed, several important forms of post-transcriptional regulation, including protein-protein and protein-small-molecule interactions, determine protein function and may or may not be directly reflected in gene expression signatures. To address this issue, a chemical proteomic strategy referred to as activity-based protein profiling (ABPP) has emerged that utilizes active site-directed probes to profile the functional state of enzyme families directly in complex proteomes (Jessani, N., Cravatt, B.F., Curr Opin Chem Biol. Feb;8(1):54-9 (2004); Evans, M.J., Cravatt, B.F. Chem. Rev. Aug;106(8):3279-301 (2006)). By development of chemical probes that capture fractions of the proteome based on shared functional properties, rather than mere abundance, ABPP interrogates portions of the biomolecular space that are inaccessible to other large-scale profiling methods. More than a dozen enzyme classes are now addressable by ABPP, including all major classes of proteases, kinases, phosphatases, glycosidases, and oxidoreductases. The application of ABPP to a number of cell and animal models has succeeded in identifying enzyme activities associated with a range of diseases, including cancer, malaria, and metabolic disorders. The ABPP method also facilitates the generation of selective inhibitors for disease-linked enzymes, including enzymes of uncharacterized function. In summary, ABPP constitutes a powerful *hypothesis-generating technology engine*, illuminating which members of enzyme superfamilies are associated with specific physiological or pathological processes and, at the same time, facilitating the creation of selective chemical reagents to test the functions of these proteins.

The human genome encompasses some 2,000 proteins that utilize adenosine 5'-triphosphate (ATP) in one way or another and some 500 of these encode for protein kinases, i.e the protein-tyrosine and protein-serine/threonine kinases, that share a catalytic domain conserved in sequence and structure but which are notably different in how their catalysis is regulated. Substrate phosphorylation by these enzymes is nature's predominant molecular way of organizing cellular signal transduction and regulating biochemical processes in general. It is not surprising, therefore, that abnormal phosphorylation of cellular proteins is a hallmark of disease and that there is a growing interest in the use of kinase inhibitors as drugs for therapeutic intervention in many disease states such as cancer, diabetes, inflammation and arthritis. It is accordingly understandable that a number of ABPP approaches to interrogate the kinase proteome have been developed. In one method the kinase substrates are fluorescently labeled and a phosphorylation-induced fluorescence change allows real-time visualization of protein kinase activity in both cell lysates and living cells (Chen, CA., et al. Biochim. Biophys. Acta. 1697(1-2):39-51 (2004)). In another method the phosphorylation of the peptide substrates is determined using fluorescently labeled anti-phosphotyrosine antibodies (van Beuningen, R., et al. Clinical Chemistry 47:1931-1933 (2001); Schuller A. et al., 11th Annual World Congres of Drug Discovery Technology (8-10 Aug 2006), Boston, MA, USA).

Protein tyrosine kinases (PTKs) are enzymes that catalyze the transfer of phosphate from ATP to tyrosine residues in polypeptides. The human genome contains about 90 functional PTKs that regulate cellular proliferation, survival, differentiation and motility. Many members of the PTK family have been identified as oncogenes over the last 25 years. More recently, inhibition of tyrosine kinase activity has become an important new route to treat cancer. Imatinib (Gleevec) is a very effective drug to treat patients with Philadelphia-chromosome (Ph) positive chronic myeloid leukemia (CML); imatinib inhibits the constitutive tyrosine kinase activity of BCR-ABL, the gene product encoded by the bcr-abl fusion gene, a result of the Ph translocation. Imatinib is also effective as a treatment for gastro-intestinal tumors (GIST). The underlying mechanism is that GISTs are often the result of overactivation of the receptor tyrosine kinases cKIT or PDGFRalpha, both of which are also inhibited by imatinib. However, a significant problem associated with imatinib treatment is the occurrence of resistance upon prolonged treatment, both in CML and in GIST. Two other approved molecules, erlotinib (Tarceva) and gefitinib (Iressa), are tyrosine kinase inhibitors primarily targeted at the EGFR receptor. The clinical efficacy of erlotinib and gefitinib is by far not as impressive as imatinib: the response rate of erlotinib and gefitinib in an unselected non-small-cell lung cancer (NSCLC) patient population is between 10 and 20%. In addition, acquired resistance occurs frequently and rapidly during treatment with EGFR inhibitors. Hence, the early detection of emerging resistance during treatment, and the stratification of drug-naive patients that are likely to respond to a kinase inhibitor from those that are not, will save patients the trouble and the precious time undergoing useless therapy (and provide the opportunity to timely undergo alternative treatments), will save money for patients and reimbursement agencies, and will increase the chances of approval of a drug in a more limited but well-defined patient population. Additionally, during the clinical development of a drug, these stratification tools can be employed to probe for indications with a particularly high fraction of likely responders.

Several examples of such prediction tools have been developed and some are in clinical use. Trastuzumab (Herceptin), a monoclonal antibody directed against the extracellular domain of Her2, is only administered to metastatic breast cancer patients that overexpress Her2. Activating mutations in EGFR have been correlated with exquisite sensitivity to EGFR inhibitors, both *in vitro* and in the clinic. Conversely, secondary mutations in EGFR have been associated with acquired resistance to erlotinib and/or gefitinib; likewise, mutations in the kinase domains of BCR-ABL, cKIT and PDGRalpha have been associated with resistance to imatinib. Also mutations in key downstream molecules in the receptor tyrosine kinase signaling cascades that allow for RTK-independent signaling, such as constitutive overactive RAS, and inactivated or deleted PTEN (the PI3 phosphatase), sometimes inversely correlate with response to RTK inhibitors. With the advent of genome-wide profiling technology, and instigated by the realization that many kinase inhibitors have multiple targets and that the relevant targets critical for effectiveness of a treatment are not always clear, unbiased methods to stratify responders and non-responders have been suggested. The most advertised of these methods is the use of gene expression signatures. In such cases, a whole-genome expression profile (before treatment) of several responders is compared to that of several non-responders, and genes that are differentially expressed between the two groups can be used to predict the likelihood of unknown cases. A critical disadvantage of this method is that it is not directly related to the mechanism of action of kinase inhibitors and hence relies on indirect parameters to assess responsiveness of a sample. Moreover, this method (and most other response-prediction methods) are based on parameters (in this case gene expression) in the untreated sample only. As a consequence, these methods are very sensitive to tumor heterogeneity and sample quality.

It has now been found that the ABPP approach is particular useful in pharmacologically profiling a compound with the objective to determine patient response to a given drug or regimen. In particular in oncology, the use of ABPP has proven useful to provide "fingerprints" that can predict response to therapeutics in cell lysates prepared from cancer cell lines, xenograft tumors or cancer patient biopsies.

### AIMS OF THE INVENTION

The aim of the present invention is to provide a method for providing a pharmacological profile of kinase inhibitors.

A further aim is to provide a use for said method for reliably predicting a patient's response to a specific kinase inhibitor.

Another aim of the present invention is to provide a tool for providing a pharmacological profile of kinase inhibitors

### SUMMARY OF THE INVENTION

The present invention concerns a method for obtaining a pharmacological profile of a kinase inhibitor using a first and a second array of substrates immobilized on a porous matrix, said method comprising the subsequent steps of;
(i) preparing a cell lysate from a cell line, including cancer cell lines; primary and immortalized tissue cell lines; non-human animal model biopsies and patient biopsies;
(ii) filtering said cell lysate over a filter in the 10 to 0.1 micrometer range to obtain a filtered cell lysate;
(iii) contacting said first array of substrates in the presence of the kinase inhibitor with a first fraction of said filtered cell lysate and determining the response of said first array
(iv) contacting said second array of substrates in the absence of the kinase inhibitor with a second fraction of said filtered cell lysate and determining the response of said second array; and
obtaining the pharmacological profile as the ratio of the array substrate response in step (iii) *over* the array substrate response in step (iv).

Preferably, said substrates are selected from the group consisting of hormone receptors, peptides, enzymes, oligonucleotides, monoclonal antibodies, haptens and aptamers., More preferably, said substrates are kinase substrates.

Advantageously, said substrates are peptide kinase substrates.

In a preferred embodiment, said substrates are at least two peptide kinase substrates selected from the group consisting of the peptide kinase substrates with sequence numbers 1 to 337. Advantageously, said substrates consist either of the peptide kinase substrates with sequence numbers 15, 16, 22, 34, 62, 83, 86, 87, 100, 105, 108, 110, 113, 125, 129, and 133; of the peptide kinase substrates with sequence numbers 15, 16, 21, 23, 38, 42, 53, 62, 69, 77, 83, 86, 91, 94, 103, 112, 114, 129, 133, 136 and 138; of the peptide kinase substrates with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138; or of the peptide kinase substrates with sequence numbers 5, 10, 38, 30, 54, 57, 68, 72, 73, 74, 82, 91, 98, 99, 102, 104, 110, 135, 118, 119, 71, 138.

In the method of the present invention, the cell lysate is preferably prepared from a cancer cell line; xenograft tumor or cancer patient biopsy, including tumor and normal tissue.

It is preferred that the response of the array of substrates is determined using a detectable signal, said signal resulting from the interaction of the sample with the array of substrates, advantageously using detectably labeled antibodies, more advantageously using fluorescently labeled anti-phosphotyrosine antibodies.

Another aspect of the present invention includes the use of the pharmacological profile determined according to the method of the present invention, to enable the distinction between responders and non-responders in the treatment of cells, tissues, organs or warm-blooded animals for a kinase inhibitor.

Preferably, the kinase inhibitor to be tested is selected from the group consisting of MTKII, 605 and erlotinib.

Use according to any one of claims 14 or 15 wherein pharmacological profile is determined using an array of substrates comprising at least 2 peptides selected from the peptide kinase substrates with sequence numbers 15, 16, 22, 34, 62, 83, 86, 87, 100, 105, 108, 110, 113, 125, 129, and 133.

Use according to any one of claims 14 or 15 wherein pharmacological profile is determined using an array of substrates comprising at least 2 peptides selected either from the peptide kinase substrates with sequence numbers 15, 16, 22, 34, 62, 83, 86, 87, 100, 105, 108, 110, 113, 125, 129, and 133; from the peptide kinase substrates with sequence numbers 15, 16, 21, 23, 38, 42, 53, 62, 69, 77, 83, 86, 91, 94, 103, 112, 114, 129, 133, 136 and 138; from the peptide kinase substrates with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138; or from the peptide kinase substrates with sequence numbers 5, 10, 38, 30, 54, 57, 68, 72, 73, 74, 82, 91, 98, 99, 102, 104, 110, 135, 118, 119, 71, 138.

The present invention also concerns a method to enable the distinction of responders from non-responders cell lines and tumors to the treatment with 4,6-ethanediylidenepyrimido[4,5-b][6,1,12] benzoxadiazacyclopentadecine, 17-bromo-8,9,10,11,12,13,14,19-octahydro-20-methoxy-13-methyl- (***MTKI 1***); or the pharmaceutically acceptable acid or base addition salts thereof; or erlotinib, or compound 605, said method comprising;
providing a sample from said cell lines and/or tumors;
contacting an array of substrates with said sample in the presence of MTKI1, erlotinib or 605;
contacting an array of substrates with said sample in the absence of MTKI1, erlotinib or 605;
determine the response of said array to the sample in step (ii);
determine the response of said array to the sample in step (iii); and
obtain the pharmacological profile as the ratio in response of the array in steps (iv) over step (v); characterized in that the array of substrates comprises at least 2 peptides selected from the group consisting of the peptide kinase substrates with sequence numbers 15, 16, 22, 34, 62, 83, 86, 87, 100, 105, 108, 110, 113, 125, 129, and 133; and wherein a responder is identified as an inhibition (ratio < 1.0) in response for at least two of the peptides selected from the peptide kinase substrates with sequence numbers 15, 16, 22, 34, 62, 83, 86, 87, 100, 105, 108, 110, 113, 125, 129, and 133.

A responder is preferably identified as an inhibition in response (ratio at least < 0.80) for at least two of the peptides selected from the group consisting of the peptide kinase substrates with sequence numbers 15, 16, 22, 34, 62, 83, 86, 87, 100, 105, 108, 110, 113, 125, 129, and 133.

Preferably, the array of substrates comprising at least 3 peptides selected from the group consisting of the peptide kinase substrates with sequence numbers 15, 16, 22, 34, 62, 83, 86, 87, 100, 105, 108, 110, 113, 125, 129, and 133; in particular the array of substrates comprises the peptide kinase substrates with sequence numbers 15, 16, 22, 34, 62, 83, 86, 87, 100, 105, 108, 110, 113, 125, 129, and 133; more in particular the array of substrates consists of the peptide kinase substrates with sequence numbers 15, 16, 22, 34, 62, 83, 86, 87, 100, 105, 108, 110, 113, 125, 129, and 133.

In the method above, the group consisting of the peptide kinase substrates with sequence numbers 15, 16, 22, 34, 62, 83, 86, 87, 100, 105, 108, 110, 113, 125, 129, and 133, can be replaced with the group consisting of the peptide kinase substrates with sequence numbers 15, 16, 21, 23, 38, 42, 53, 62, 69, 77, 83, 86, 91, 94, 103, 112, 114, 129, 133, 136 and 138; the group consisting of the peptide kinase substrates with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138; or the group consisting of the peptide kinase substrates with sequence numbers 5, 10, 38, 30, 54, 57, 68, 72, 73, 74, 82, 91, 98, 99, 102, 104, 110, 135, 118, 119, 71, 138.

In any method according to the present invention, the response of the array of substrates is preferably determined using antibodies in a solution free of antifungals, in particular using an azide free solution.

In the methods of the present invention, an overall accuracy of 60%, 70%, preferably 80 % or advantageously 90 % or higher can be obtained.

Another embodiment of the present invention concerns an array of substrates comprising peptides selected from the group consisting of the peptide kinase substrates with sequence numbers 1 to 337, with the proviso that said array does not consist of peptide kinase substrates with sequence numbers 1-140.

Said array of substrates can consist of the peptide kinase substrates with sequence numbers 1 to 337, or the peptide kinase substrates with sequence numbers 15, 16, 22, 34, 62, 83, 86, 87, 100, 105, 108, 110, 113, 125, 129, and 133, or the peptide kinase substrates with sequence numbers 15, 16, 21, 23, 38, 42, 53, 62, 69, 77, 83, 86, 91, 94, 103, 112, 114, 129, 133, 136 and 138, or the peptide kinase substrates with sequence numbers142, 2, 163, 173, 177, 190, 161, 197, 207, 208, 213, 241, 73, 252, 255, 258, 262, 79, 87, 266, 86, 269, 95, 296, 303, 305, 308, and 138, or the peptide kinase substrates with sequence numbers 5, 10, 38, 30, 54, 57, 68, 72, 73, 74, 82, 91, 98, 99, 102, 104, 110, 135, 118, 119, 71, 138.

Another aspect of the present invention concers a method for predicting possible kinase inhibitor response in a patient in the treatment of cancer, comprising the steps of:
(i) Preparing a cell lysate from a cancer patient biopsy, including normal and tumor tissue,
(ii) Contacting a first array of substrates according to the present invention with a fraction of the cell lysate of step (i) in the presence of a kinase inhibitor;
(iii) Contacting a second array of substrates identical to said first array of substrates with a fraction of the cell lysate of step (i) in the absence of the kinase inhibitor; and

Obtaining a pharmacological profile for said patient as the ratio of the first array of substrates response over the second array of substrates response, wherein said pharmacological profile predicts said possible kinase inhibitor response.

The invention will be further clarified in the following drawings and examples, which are to be considered non-limiting to the scope of protection conferred by the claims.

### BRIEF DESCRIPTION OF THE DRAWINGS

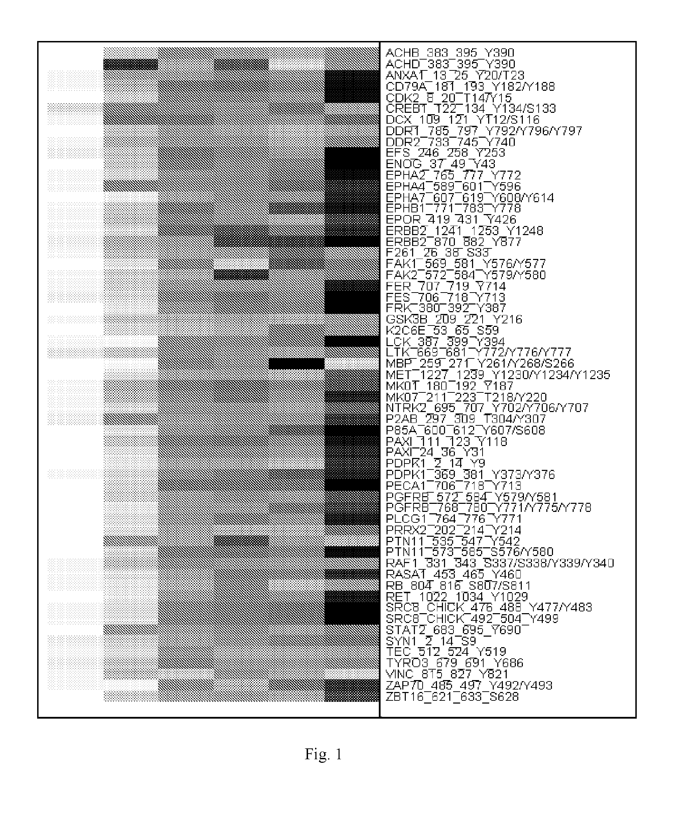

Figure 1: Ratio of initial velocities of the phosphorylation of detectable peptides in treated over untreated lysates from cell line NCI-H3255. From left to right: DMSO, lapatinib/Tykerb, MTKI1/MTKI, gefitinib/Iressa, erlotinib/Tarceva, ZD6474/zactima. Scale: white =1 (no inhibition); black=0.1 (10-fold inhibition).

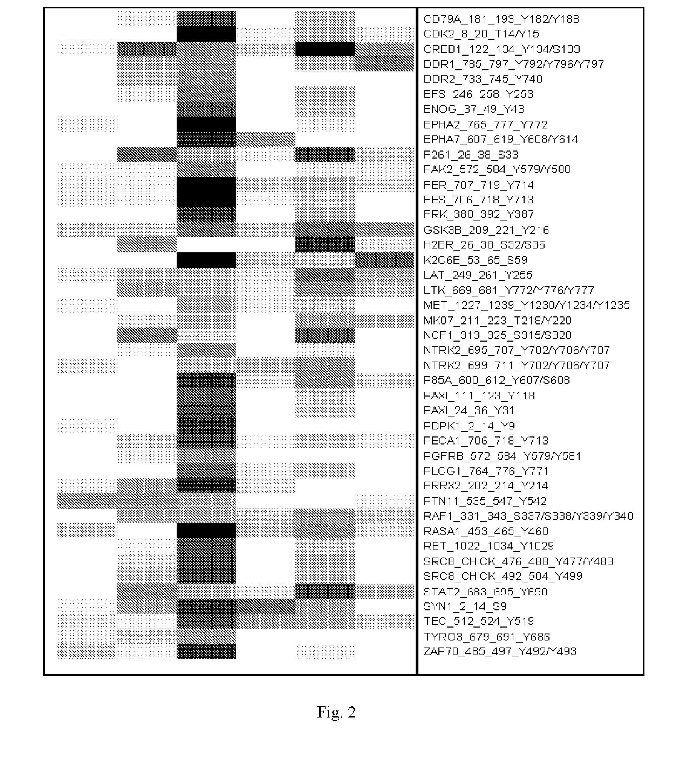

Figure 2: Ratio of initial velocities of the phosphorylation in treated over untreated DU145 samples: left three profiles are derived from cellular treatment with DMSO (outer left lane), MTKI1 (2^{nd} lane) or a histone deacetylase inhibitor (3^{rd} lane); rightmost three lanes are derived from lysate treatment of untreated DU145 cells with DMSO (1^{st} lane), MTKI1 (2^{nd} lane), and the histone deacetylase inhibitor (3^{rd} lane). Scale: white =1 (no inhibition); black=0.1 (10-fold inhibition).

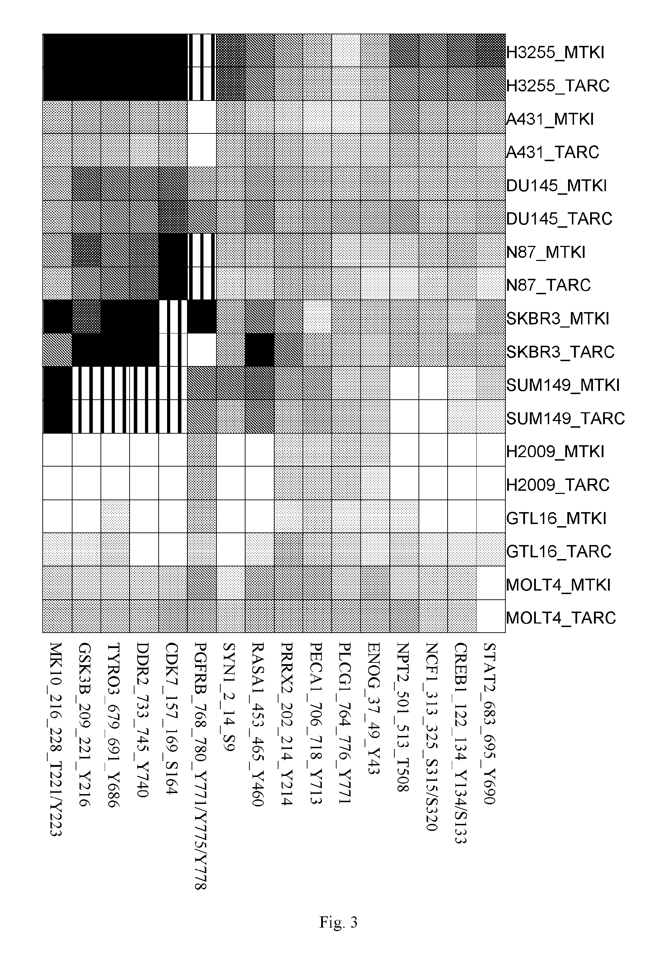

Figure 3: Ratio of initial velocity of peptide phosphorylation in the presence of solvent (DMSO) over the initial velocity of peptide phosphorylation in the presence of MTKI or Tarceva; Scale: white =1 (no inhibition); black=0.25 (4-fold inhibition)

### DETAILED DESCRIPTION OF THE INVENTION

The present invention describes a method to measure the inhibition of tyrosine kinase activity by small molecule compounds in lysates, prepared from cell lines or tumor samples. Importantly, this method directly measures the relevant mechanism of action of a kinase inhibitor and the parameter that correlates with response status is a relative measure (i.e. the *ratio* of kinase activity in the absence and in the presence of compound). It is demonstrated for a multi-targeted kinase inhibitor, MTKI-1, that responder cell lines can be discriminated from non-responder cell lines based on these phosphopeptide profiles with an overall accuracy of 82 % in 27 cell lines. The kinases that phosphorylate these peptides are closely linked to the mechanism of action of the inhibitor. In addition, it is shown that also in xenograft tumor lysates or for similar kinase inhibitors, many of the same peptides can discriminate responders from non-responders, indicating that this technology should be applicable to patient stratification.

In one embodiment the present invention provides a method for pharmacologically profiling of compounds using an array of substrates immobilized on a porous matrix, said method comprising;
(i) contacting the array of substrates with either an untreated sample or a sample pretreated with the compound to be tested;
(ii) determine the response of said array to the untreated sample;
(iii) determine the response of said array to the pretreated sample; and
(iv) obtain the pharmacological profile as the difference in response of the array in steps (ii) and step (iii).

The substrates as used herein, are meant to include hormone receptors, peptides, enzymes, oligonucleotides, monoclonal antibodies, haptens and aptamers. In particular the substrates used or kinase substrates, more in particular peptide kinase substrates, even more particular the peptide kinase substrates in Table 1, most particular using at least 2, 6, 12, 40, 50, 60, 70, 80, 90, 100, 110, 120 or 130 peptides of the peptide kinase substrates of Table 1. In an even further embodiment the array of substrates comprising at least 2 peptides selected from the group consisting of the peptides with sequence numbers 4, 19, 24, 69, 113, 114, 136 and 138; more in particular the array of substrates consist of the peptides with sequence numbers 4, 19, 24, 69, 113, 114, 136 and 138.

In an alternative further embodiment, the array of substrates comprising at least 2 peptides selected from the group consisting of the peptides with sequence numbers 15, 16, 22, 34, 62, 83, 86, 87, 100, 105, 108, 110, 113, 125, 129 and 133, more in particular the array of substrates consist of the peptides with sequence numbers 15, 16, 22, 34, 62, 83, 86, 87, 100, 105, 108, 110, 113, 125, 129 and 133.

In another alternative further embodiment, the array of substrates comprising at least 2 peptides selected from the group consisting of the peptides with sequence numbers 5, 10, 38, 30, 54, 57, 68, 72, 73, 74, 82, 91, 98, 99, 102, 104, 110, 135, 118, 119, 71, 138, more in particular the array of substrates consist of the peptides with sequence numbers 5, 10, 38, 30, 54, 57, 68, 72, 73, 74, 82, 91, 98, 99, 102, 104, 110, 135, 118, 119, 71, 138.

**Table 1: list of 140 peptides used in the PAMCHIP96 profiling analysis, their sequence and Seq.Id.No.**

| Seq. | Name | Sequence |
|---|---|---|
| 1 | 41_653_665_Y627 | RLDGENIYIRHSN |
| 2 | ACHB_383_395_Y390 | WGRGTDEYFIRKP |
| 3 | ACHD_383_395_Y390 | YISKAEEYFLLKS |
| 4 | AMPE_5_17_Y12 | EREGSKRYCIQTK |
| 5 | ANXA1_13_25_Y20/T23 | IENEEQEYVQTVK |
| 6 | ANXA2_16_28_Y23/S25 | HSTPPSAYGSVKA |
| 7 | B3AT_39_51_Y46/S50 | TEATATDYHTTSH |
| 8 | C1R_199_211_S206 | TEASGYISSLEYP |
| 9 | CALM_93_105_Y99/S101 | FDKDGNGYISAAE |
| 10 | CALM_95_107_Y99/S101 | KDGNGYISAAELR |
| 11 | CBL_693_705_Y700 | EGEEDTEYMTPSS |
| 12 | CD3Z_146_158_Y153 | STATKDTYDALHM |
| 13 | CD79A_181_193_Y182/Y188 | EYEDENLYEGLNL |
| 14 | CDK2_8_20_T14/Y15 | EKIGEGTYGVVYK |
| 15 | CDK7_157_169_S164 | GLAKSFGSPNRAY |
| 16 | CREB1_122_134_Y134/S133 | QKRREILSRRPSY |
| 17 | CRK_214_226_Y221 | GPPEPGPYAQPSV |
| 18 | CTNB1_79_91_Y86 | VADIDGQYAMTRA |
| 19 | DCX_109_121_Y112/S116 | GIVYAVSSDRFRS |
| 20 | DDR1_506_518_Y513 | LLLSNPAYRLLLA |
| 21 | DDR1_785_797_Y792/Y796/Y797 | FGMSRNLYAGDYY |
| 22 | DDR2_733_745_Y740 | RNLYSGDYYRIQG |
| 23 | DYR1A_212_224_Y219 | KHDTEMKYYIVHL |
| 24 | DYR1A_312_324_Y319/Y321 | CQLGQRIYQYIQS |
| 25 | EFS_246_258_Y253 | GGTDEGIYDVPLL |
| 26 | EFS_246_258_Y253F | GGTDEGIFDVPLL |
| 27 | EGFR_1062_1074_Y1069 | EDSFLQRYSSDPT |
| 28 | EGFR_1103_1115_Y1110 | GSVQNPVYHNQPL |
| 29 | EGFR_1118_1130_Y1125 | APSRDPHYQDPHS |
| 30 | EGFR_1165_1177_Y1172 | ISLDNPDYQQDFF |
| 31 | EGFR_1190_1202_Y1197 | STAENAEYLRVAP |
| 32 | EGFR_862_874_Y869 | LGAEEKEYHAEGG |
| 33 | EGFR_908_920_Y915 | MTFGSKPYDGIPA |
| 34 | ENOG_37_49_Y43 | SGASTGIYEALEL |
| 35 | EPHA1_774_786_Y781 | LDDFDGTYETQGG |
| 36 | EPHA2_581_593_Y588 | QLKPLKTYVDPHT |
| 37 | EPHA2_765_777_Y772 | EDDPEATYTTSGG |
| 38 | EPHA4_589_601_Y596 | LNQGVRTYVDPFT |
| 39 | EPHA4_921_933_Y928 | QAIKMDRYKDNFT |
| 40 | EPHA7_607_619_Y608/Y614 | TYIDPETYEDPNR |
| 41 | EPHB 1_771_783_Y778 | DDTSDPTYTSSLG |
| 42 | EPHB1_921_933_Y928 | SAIKMVQYRDSFL |
| 43 | EPHB4_583_595_Y590 | IGHGTKVYIDPFT |
| 44 | EPOR 361 373 Y368 | SEHAQDTYLVLDK |
| 45 | EPOR_419_43_Y426 | ASAASFEYTILDP |
| 46 | ERBB2_1241_1253_Y1248 | PTAENPEYLGLDV |
| 47 | ERBB2_870_882_Y877 | LDIDETEYHADGG |
| 48 | ERBB2_945_957_Y952 | PISTIDVYMIMVK |
| 49 | ERBB4_1181_1193_Y1188 | QALDNPEYHNASN |
| 50 | ERBB4_1277_1289_Y1284 | IVAENPEYLSEFS |
| 51 | F261-26-38-S33 | RLQRRRGSSIPQF |
| 52 | FABH 13 25 Y19 | DSKNFDDYMKSLG |
| 53 | FAK1_569_581_Y576/Y577 | RYMEDSTYYKASK |
| 54 | FAK2_572_584_Y579/Y580 | RYIEDEDYYKASV |
| 55 | FER_707_719_Y714 | RQEDGGVYSSSGL |
| 56 | FES_706_718_Y713 | REEADGVYAASGG |
| 57 | FGFR1_759_771_Y766 | ALTSNQEYLDLSM |
| 58 | FGFR2_762_774_Y769 | TLTTNEEYLDLSQ |
| 59 | FGFR3_641_653_Y648 | DVHNLDYYKKTTN |
| 60 | FGFR3_753_765_Y760 | TVTSTDEYLDLSA |
| 61 | FRK_380_392_Y387 | KVDNEDIYESRHE |
| 62 | GSK3B_209_221_Y216 | RGEPNVSYICSRY |
| 63 | H2BR_26_38_S32/S36 | DGKKRKRSRKESY |
| 64 | INSR_1348_1360_S1354/Y1355 | SLGFKRSYEEHIP |
| 65 | INSR_993_1005_Y993/Y999 | YASSNPEYLSASD |
| 66 | IRS1_1222_1234_Y1230 | SSEDLSAYASISF |
| 67 | IRS2_535_545_Y540 | GGGGGEFYGYMTM |
| 68 | JAK1_1015_1027_Y1022/Y1023 | AIETDKEYYTVKD |
| 69 | K2C6E_53_65_S59 | GAGFGSRSLYGLG |
| 70 | K2C8_425_437_S431 | SAYGGLTSPGLSY |
| 71 | KSYK_518_530_Y525/Y526 | ALRADENYYKAQT |
| 72 | LAT_194_206_Y200 | MESIDDYVNVPES |
| 73 | LAT_249_261_Y255 | EEGAPDYENLQEL |
| 74 | LCK_387_399_Y394 | RLIEDNEYTAREG |
| 75 | LTK_669_681_Y772/Y776/Y777 | RDIYRASYYRRGD |
| 76 | MBP_198_210_Y203 | ARTAHYGSLPQKS |
| 77 | MBP_259_271_Y261/Y268/S266 | FGYGGRASDYKSA |
| 78 | MBP_263_275_Y268/S266/S270 | GRASDYKSAHKGF |
| 79 | MET_1227_1239_Y1230/Y1234/Y1235 | RDMYDKEYYSVHN |
| 80 | MK01_180_192_Y187 | HTGFLTEYVATRW |
| 81 | MK01_198_210_Y205 | IMLNSKGYTKSID |
| 82 | MK07_211_223_T218/Y220 | AEHQYFMTEYVAT |
| 83 | MK10_216_228_T221/Y223 | TSFMMTPYVVTRY |
| 84 | MK12_178_190_T183/Y185 | ADSEMTGYVVTRW |
| 85 | MK14_173_185_T180/Y182 | RHTDDEMTGYVAT |
| 86 | NCF1_313_325_S315/S320 | QRSRKRLSQDAYR |
| 87 | NPT2_501_513_T508 | AKALGKRTAKYRW |
| 88 | NTRK1_489_501_Y496 | HIIENPQYFSDAC |
| 89 | NTRK2_509_521_Y516 | PVIENPQYFGITN |
| 90 | NTRK2_695_707_Y702/Y706/Y707 | FGMSRDVYSTDYY |
| 91 | NTRK2_699_711_Y702/Y706/Y707 | RDVYSTDYYRVGG |
| 92 | ODBA_340_352_S345 | DDSSAYRSVDEVN |
| 93 | ODPAT_291_303_S291/S293 | SMSDPGVSYRTRE |
| 94 | P2AB_297_309_T304/Y307 | EPHVTRRTPDYFL |
| 95 | P85A_600_612_Y607/S608 | NENTEDQYSLVED |
| 96 | PAXI_111_123_Y118 | VGEEEHVYSFPNK |
| 97 | PAXI_24_36_Y31 | FLSEETPYSYPTG |
| 98 | PDPK1_2_14_Y9 | ARTTSQLYDAVPI |
| 99 | PDPK1_369_381_Y373/Y376 | DEDCYGNYDNLLS |
| 100 | PECA1_706_718_Y713 | KKDTETVYSEVRK |
| 101 | PERI_459_471_Y471 | QRSELDKSSAHSY |
| 102 | PGFRB_1002_1014_Y1009 | LDTSSVLYTAVQP |
| 103 | PGFRB_572_584_Y579/Y581 | VSSDGHEYIYVDP |
| 104 | PGFRB_709_721_Y716 | RPPSAELYSNALP |
| 105 | PGFRB_768_780_Y771/Y775/Y778 | SSNYMAPYDNYVP |
| 106 | PGFRB_771_783_Y771/Y775/Y778 | YMAPYDNYVPSAP |
| 107 | PLCG1_1246_1258_S1248/Y1253 | EGSFESRYQQPFE |
| 108 | PLCG1_764_776_Y771 | IGTAEPDYGALYE |
| 109 | PLCG1_776_788_Y783 | EGRNPGFYVEANP |
| 110 | PRRX2_202_214_Y214 | WTASSPYSTVPPY |
| 111 | PTN11 535 547 Y542 | SKRKGHEYTNIKY |
| 112 | RAF1_331_343_S337/S338/Y339/Y340 | RPRGQRDSSYYWE |
| 113 | RASA1_453 465 Y460 | TVDGKEIYNTIRR |
| 114 | RB_804_816_S807/S811 | IYISPLKSPYKIS |
| 115 | RBL2_99_111_Y111/5103 | VPTVSKGTVEGNY |
| 116 | RET_1022_1034_Y1029 | TPSDSLIYDDGLS |
| 117 | RET_680_692_Y687 | AQAFPVSYSSSGA |
| 118 | RON_1346_1358_Y1353 | SALLGDHYVQLPA |
| 119 | RON_1353_1365_Y1356/Y1360 | YVQLPATYMNLGP |
| 120 | SRC8_CHICK_470_482_Y477 | VSQREAEYEPETV |
| 121 | SRC8_CHICK_476_488_Y477/Y483 | EYEPETVYEVAGA |
| 122 | SRC8_CHICK_492_504_Y499 | YQAEENTYDEYEN |
| 123 | STA5A_687_699_Y694 | LAKAVDGYVKPQI |
| 124 | STAT1_694_706_Y701 | DGPKGTGYIKTEL |
| 125 | STAT2_683_695_Y690 | NLQERRKYLKHRL |
| 126 | STAT3_698_710_Y705 | DPGSAAPYLKTKF |
| 127 | STAT4_686_698_Y693 | TERGDKGYVPSVF |
| 128 | STAT4_714_726_Y725 | PSDLLPMSPSVYA |
| 129 | SYN1_2_14_S9 | NYLRRRLSDSNFM |
| 130 | TAU_512_524_Y514/T522 | SGYSSPGSPGTPG |
| 131 | TEC_512_524_Y519 | RYFLDDQYTSSSG |
| 132 | TNNT1_2_14_Y9 | SDTEEQEYEEEQP |
| 133 | TYRO3_679_691_Y686 | KIYSGDYYRQGCA |
| 134 | VEGFR1_1049_1061_Y1053 | KNPDYVRKGDTRL |
| 135 | VEGFR2 1052 1064 Y1059 | DIYKDPDYVRKGD |
| 136 | VEGFR2_944_956_Y951 | RFRQGKDYVGAIP |
| 137 | VGFR3_1061_1073_Y1063/Y1068/S1073 | DIYKDPDYVRKGS |
| 138 | VINC_815_827_Y821 | KSFLDSGYRILGA |
| 139 | ZAP70_485_497_Y492/Y493 | ALGADDSYYTARS |
| 140 | ZBT16_621_633_S628 | LRTHNGASPYQCT |

In an alternative embodiment, the substrates used are kinase substrates, more in particular peptide kinase substrates, even more particular the peptide kinase substrates in Table 2, most particular using at least 2, 6, 12, 16, 20, 24, 28, 32, 36, 40, 50, 60, 70, 80, 90, 100, 110, 120, 130, 150, 180, 200, 220 or 240 peptides of the peptide kinase substrates of Table 2. In an even further embodiment the array of substrates comprising at least 2 peptides selected from the group consisting of peptides with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138; more in particular the array of substrates consist of the peptides with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138.

**Table 2: list of 256 peptides used in an alternative profiling analysis, their sequence en Seq.Id.No.**

| Seq. | Name | Sequence |
|---|---|---|
| 1 | 41_653_665_Y627 | RLDGENIYIRHSN |
| 141 | ABL Y272 | HKLGGGQYGEVYEGV |
| 142 | ABLIM1_350_364_Y357 | RTSSESIYSRPGSSI |
| 143 | ABLIM1_454_468_Y461 | GSINSPVYSRHSYTP |
| 2 | ACHB_383_395_Y390 | WGRGTDEYFIRKP |
| 3 | ACHD_383_395_Y390 | YISKAEEYFLLKS |
| 144 | ACLY_675_689_Y682 | SRTTDGVYEGVAIGG |
| 145 | ACTB_159_173_Y166 | VTHTVPIYEGYALPH |
| 146 | ADAM9_805_819_Y815 | PARPAPAPPLYSSLT |
| 147 | ADD2_482_496_Y489 | PNQFVPLYTDPQEVL |
| 148 | AGBL2_10_24_Y17 | KQTIPDPYEDFMYRH |
| 149 | ANKRD26_289_303_Y296 | RKNLEATYGTVRTGN |
| 150 | ANXA2_192_206_Y199 | DQDARDLYDAGVKRK |
| 151 | ANXA2_231_245_Y238 | RYKSYSPYDMLESIR |
| 152 | APCDD1_103_117_Y110 | FKAYQFYYGSNRCTN |
| 153 | APP_750_764_Y757 | SKMQQNGYENPTYKF |
| 154 | ARHGEF10L_124_138_Y131 | ALEEDVIYDDVPCES |
| 155 | BAG3_240_254_Y247 | YQTHQPVYHKIQGDD |
| 156 | BCAR1_242_256_Y249 | APGPQDIYDVPPVRG |
| 157 | BCAR1_320_334_Y327 | PLLREETYDVPPAFA |
| 158 | BCAR1_355_369_Y362 | SPPAEDVYDVPPPAP |
| 159 | BCAR1_365_379_Y372 | PPPAPDLYDVPPGLR |
| 160 | BCAR1_657_671_Y664 | EGGWMEDYDYVHLQG |
| 161 | BCAR1_380_394_Y387 | RPGPGTLYDVPRERV |
| 162 | BCR_239_253_Y246 | SCGVDGDYEDAELNP |
| 163 | C11 orf35_217_231_Y224 | WNSVARRYPNLFTNM |
| 164 | C19orf21_88_102_Y95 | EDEGWQVYRLGARDA |
| 165 | C1orf73_928_942_Y935 | VKSLEDPYSQQIRLQ |
| 166 | C20orf18_281_295_Y288 | CPFIDNTYSCSGKLL |
| 167 | C2orf4_203_217_Y210 | DESQGEIYRSIEHLD |
| 168 | C3orf6_138_152_Y145 | RAYADSYYYEDGGMK |
| 169 | C3orf6_272_286_Y279 | TDGEDADYTHFTNQQ |
| 170 | C9orf86_672_686_Y683 | APGGRHPGGGDYEEL |
| 171 | CALR3_68_82_Y75 | TTQNGRFYAISARFK |
| 172 | CAV1_7_21_Y14 | VDSEGHLYTVPIREQ |
| 173 | CBL_667_681_Y674 | SSSANAIYSLAARPL |
| 11 | CBL_693_705_Y700 | EGEEDTEYMTPSS |
| 174 | CBLB_882_896_Y889 | TNRTSQDYDQLPSCS |
| 13 | CD79A_181_193_Y182/Y188 | EYEDENLYEGLNL |
| 14 | CDK2_8_20_T14/Y15 | EKIGEGTYGVVYK |
| 175 | CDK3_12_26_Y19 | EGTYGVVYKAKNRET |
| 15 | CDK7_157_169_S164 | GLAKSFGSPNRAY |
| 176 | CENTB2_735_749_Y742 | MRESEGLYGQPGDET |
| 177 | CFL1_61_75_Y68 | GQTVDDPYATFVKML |
| 178 | CLDN1_197_211_Y210 | RPYPKPAPSSGKDYV |
| 179 | CLDN2_187_201_Y194 | SQRNRSNYYDAYQAQ |
| 16 | CREB1_122_134_Y134/S133 | QKRREILSRRPSY |
| 180 | CTNND1_214_228_Y221 | SRHYEDGYPGGSDNY |
| 181 | CTTN_414_428_Y421 | RLPSSPVYEDAASFK |
| 182 | CDC2_12_26_Y19 | EGTYGVVYKGRHKTT |
| 183 | DCBLD2 558 572 Y565 | KKKTEGTYDLPYWDR |
| 184 | DCBLD2 743 757 Y750 | PAPDELVYQVPQSTQ |
| 19 | DCX_109_121_Y112/S116 | GIVYAVSSDRFRS |
| 20 | DDR1_506_518_Y513 | LLLSNPAYRLLLA |
| 22 | DDR2_733_745_Y740 | RNLYSGDYYRIQG |
| 185 | DDX3X_259_273_Y266 | RYGRRKQYPISLVLA |
| 186 | DDX5_195_209_Y202 | RLKSTCIYGGAPKGP |
| 187 | DKFZp434C0328_451_465_Y458 | RVSTDLKYRKQPWGL |
| 188 | DKFZp761P0423_404_418_Y411 | ATQPEPIYAESTKRK |
| 189 | DOK1_308_322_Y315 | CPSQDSLYSDPLDST |
| 190 | DOK1_402_416_Y409 | YNPATDDYAVPPPRS |
| 24 | DYR1A_312_324_Y319/Y321 | CQLGQRIYQYIQS |
| 191 | EFNB1_310_324_Y317 | ENNYCPHYEKVSGDY |
| 30 | EGFR_1165_1177_Y1172 | ISLDNPDYQQDFF |
| 192 | EIF3S9_442_456_Y449 | TLDTLSIYETPSMGL |
| 193 | ELMO2_41_55_Y48 | WSLPNPEYYTLRYAD |
| 194 | ELMO2_706_720_Y717 | IPKEPSSYDFVYHYG |
| 195 | | |
| 35 | EPHA1_774_786_Y781 | LDDFDGTYETQGG |
| 37 | EPHA2_765_777_Y772 | EDDPEATYTTSGG |
| 38 | EPHA4_589_601_Y596 | LNQGVRTYVDPFT |
| 42 | EPHB1_921_933_Y928 | SAIKMVQYRDSFL |
| 196 | EPHB2_774_788_Y781 | DDTSDPTYTSALGGK |
| 197 | EPHB3_607_621_Y614 | VYIDPFTYEDPNEAV |
| 198 | EPHB4_767_781_Y774 | ENSSDPTYTSSLGGK |
| 44 | EPOR 361 373 Y368 | SEHAQDTYLVLDK |
| 199 | ERBB2_1241_1255_Y1248 | PTAENPEYLGLDVPV |
| 47 | ERBB2_870_882_Y877 | LDIDETEYHADGG |
| 200 | ERBB3_1152_1166_Y1159 | EEEDVNGYVMPDTHL |
| 201 | ERRFI1_388_402_Y395 | KVSSTHYYLLPERPP |
| 202 | F11R_273_287_Y280 | TSSKKVIYSQPSARS |
| 203 | FAK_Y407 | IIDEEDTYTMPSTRD |
| 204 | FAK_Y861 | PIGNQHIYQPVGKPD |
| 205 | FAK_Y925 | DRSNDKVYENVTGLV |
| 53 | FAK1_569_581_Y576/Y577 | RYMEDSTYYKASK |
| 55 | FER_707_719_Y714 | RQEDGGVYSSSGL |
| 206 | FGD6_747_761_Y754 | EYENIRHYEEIPEYE |
| 207 | FGFR1OP_330_344_Y337 | GTGEDDDYVDDFNST |
| 208 | FKS | EFGTYGTLSK |
| 209 | FLJ11273_43_57_Y50 | GDVSQFPYVEFTGRD |
| 210 | FLJ12747_469_483_Y476 | RHGEQSLYSPQTPAY |
| 211 | FLJ20625_33_47_Y40 | LNGAEPNYHSLPSAR |
| 212 | FLT1_Y1333 | PPDYNSVVLYSTPPI |
| 213 | FYN_206_220_Y213 | RKLDNGGYYITTRAQ |
| 214 | GAB1_252_266_Y259 | ASVDSSLYNLPRSYS |
| 215 | GOLGA5_47_61_Y54 | QQNTDLIYQTGPKST |
| 216 | GPRC5C_3392 406 Y399 | KVPSEGAYDIILPRA |
| 217 | GRLF1 1081 1095 Y1088 | DGFDPSDYAEPMDAV |
| 62 | GSK3B_209_221_Y216 | RGEPNVSYICSRY |
| 63 | H2BR_26_38_S32/S36 | DGKKRKRSRKESY |
| 218 | H41_183_197_Y190 | PQGPPEIYSDTQFPS |
| 219 | HCFC2_553_567_Y560 | KSEVDETYALPATKI |
| 220 | HCK_404_418_Y411 | RVIEDNEYTAREGAK |
| 221 | HKS1 | AAEEIYAARRG |
| 222 | HNRPA2B1_312_326_Y319 | SRNMGGPYGGGNYGP |
| 223 | HNRPF_299_313_Y306 | KATENDIYNFFSPLN |
| 224 | HNRPH3 289 303 Y296 | GMDNQGGYGSVGRMG |
| 225 | HRIHFB2122_166_180_Y173 | GQRQALDYVELSPLT |
| 226 | HRMT1L2_292_306_Y299 | STSPESPYTHWKQTV |
| 227 | HSPCB_294_308_Y301 | DDITQEEYGEFYKSL |
| 228 | HSPCB_477_491_Y484 | KETQKSIYYITGESK |
| 229 | ILF3_757_771_Y764 | QSYNQSPYSNYGPPQ |
| 230 | INSR_1183_1197_Y1190 | DIYETDYYRKGGKGL |
| 231 | IRS1_655_669_Y662 | QRVDPNGYMMMSPSG |
| 232 | IRS2_816_830_Y823 | CGGDSDQYVLMSSPV |
| 233 | ITGB4_1200_1214_Y1207 | GAQGEGPYSSLVSCR |
| 234 | ITSN2_960_974_Y967 | REEPEALYAAVNKKP |
| 235 | JAK2_563_577_Y570 | VRREVGDYGQLHETE |
| 69 | K2C6E_53_65_S59 | GAGFGSRSLYGLG |
| 236 | KDR Y1059 | DIYKDPDYVRKGDAR |
| 237 | KDR Y996 | EEAPEDLYKDFLTLE |
| 238 | KIAA2002_1100_1114_Y1107 | PNPCSATYSNLGQSR |
| 239 | KIAA2002_634_648_Y641 | AYDNLAIYKSFLGTS |
| 240 | KIRREL_401_415_Y408 | TRVMKAIYSSFKDDV |
| 241 | KIRREL 550 564 Y557 | SGLERTPYEAYDPIG |
| 242 | KIT Y703 | DHAEAALYKNLLHSK |
| 243 | KIT Y721 | CSDSTNEYMDMKPGV |
| 244 | KIT_Y936 | SESTNHIYSNLANCS |
| 245 | KRT19_58_72_Y65 | SGGYGGGYGGVLTAS |
| 73 | LAT_249_261_Y255 | MESIDDYVNVPES |
| 246 | LAT2_186_200_Y193 | EDEESEDYQNSASIH |
| 247 | LDHB_233_247_Y240 | KMVVESAYEVIKLKG |
| 248 | LISCH7 302 316 Y309 | SIYAPSTYAHLSPAK |
| 249 | LLGL1_502_516_Y509 | KVGCFDPYSDDPRLG |
| 250 | LMO7_341_355_Y348 | RSWASPVYTEADGTF |
| 251 | LMO7_801_815_Y808 | IDATSGIYNSEKSSN |
| 252 | LPHN2 1343 1357 Y1350 | RSENEDIYYKSMPNL |
| 253 | LPP_268_282_Y275 | RGGMDYAYIPPPGLQ |
| 254 | LPP_294_308_Y301 | GRYYEGYYAAGPGYG |
| 255 | LYN_186_200_Y193 | RSLDNGGYYISPRIT |
| 256 | LYN_498_512_Y508 | DDFYTATEGQYQQQP |
| 257 | MAP1B_1882 1896 Y1889 | PDEEDYDYESYEKTT |
| 258 | MAPK8_178_192_Y185 | TSFMMTPYVVTRYYR |
| 259 | MARCH7 308 322 Y315 | SLNSENSYVSPRILT |
| 260 | MARVELD2 7 21 Y14 | SRNRDRRYDEVPSDL |
| 261 | MATR3_212_226_Y219 | GYYDRMDYEDDRLRD |
| 76 | MBP_198_210_Y203 | ARTAHYGSLPQKS |
| 77 | MBP_259_271_Y261/Y268/S266 | FGYGGRASDYKSA |
| 262 | MCP_362_376_Y369 | KADGGAEYATYQTKS |
| 79 | MET_1227_1239_Y1230/Y1234/Y1235 | RDMYDKEYYSVHN |
| 263 | MET_Y1230 | FGLARDMYDKEYYSV |
| 87 | NPT2_501_513_T508 | AKALGKRTAKYRW |
| 264 | METAP1_103_117_Y110 | PTRPVPSYIQRPDYA |
| 265 | METAP1_92_106_Y99 | TGKLRPHYPLMPTRP |
| 80 | MK01_180_192_Y187 | HTGFLTEYVATRW |
| 83 | MK10_216_228_T221/Y223 | TSFMMTPYVVTRY |
| 266 | NAPG_298_312_Y307 | TAADEEEDEYSGGLC |
| 86 | NCF1_313_325_S315/S320 | QRSRKRLSQDAYR |
| 129 | SYN1_2_14_S9 | NYLRRRLSDSNFM |
| 267 | NEK2_12_26_Y19 | YTIGTGSYGRCQKIR |
| 268 | NTE_1188_1202_Y1195 | FGKFDQIYDVGYQYG |
| 91 | NTRK2_699_711_Y702/Y706/Y707 | RDVYSTDYYRVGG |
| 94 | P2AB_297_309_T304/Y307 | EPHVTRRTPDYFL |
| 269 | P2RY2_223_237_Y230 | RRLLKPAYGTSGGLP |
| 95 | P85A_600_612_Y607/S608 | NENTEDQYSLVED |
| 270 | PABPC1_357_371_Y364 | IVATKPLYVALAQRK |
| 271 | PAG1_334_348_Y341 | LTVPESTYTSIQGDP |
| 272 | PAG1_352_366_Y359 | PSSCNDLYATVKDFE |
| 273 | PAG1_410_424_Y417 | LVPKENDYESISDLQ |
| 274 | PARD3_482_496_Y489 | IGGSAPIYVKNILPR |
| 96 | PAXI_111_123_Y118 | VGEEEHVYSFPNK |
| 97 | PAXI_24_36_Y31 | FLSEETPYSYPTG |
| 275 | PCM1_1169_1183_Y1176 | NSSGKTEYMAFPKPF |
| 276 | PCTK2 196 210 Y203 | EKLGEGTYATVYKGR |
| 277 | PDFGA_Y574 | PDGHEYIYVDPMQLP |
| 278 | PDFGA_Y768 | LYDRPASYKKKSMLD |
| 279 | PDFGB_Y1021 | PNEGDNDYIIPLPDP |
| 98 | PDPK1_2_14_Y9 | ARTTSQLYDAVPI |
| 99 | PDPK1_369_381_Y373/Y376 | DEDCYGNYDNLLS |
| 123 | STA5A_687_699_Y694 | LAKAVDGYVKPQI |
| 280 | | |
| 281 | PGM1_346_360_Y353 | SATKIALYETPTGWK |
| 282 | PIK3R1_303_317_Y310 | LRKTRDQYLMWLTQK |
| 283 | PIK3R2_598_612_Y605 | KNETEDQYALMEDED |
| 284 | PKP4_471_485_Y478 | ALNTTATYAEPYRPI |
| 285 | PLAGL1_17_31_Y24 | EKFTIHNYSHSRERP |
| 108 | PLCG1_764_776_Y771 | IGTAEPDYGALYE |
| 286 | PLEC1_3245 3259 Y3252 | RARQEELYSELQARE |
| 287 | PLEKHA6_485_499_Y492 | PPRSEDIYADPAAYV |
| 288 | PLEKHA7_463_477_Y470 | SLSFPENYQTLPKST |
| 289 | PRKCD_306 320 Y313 | SSEPVGIYQGFEKKT |
| 290 | PRKCD_327 341 Y334 | MQDNSGTYGKIWEGS |
| 110 | PRRX2_202_214_Y214 | WTASSPYSTVPPY |
| 291 | PSMA2_69_83_Y76 | TKHIGLVYSGMGPDY |
| 292 | PTK2_585_599_Y592 | GDFGLSRYMEDSTYY |
| 293 | PTK9_336_350_Y343 | ELTADFLYEEVHPKQ |
| 111 | PTN11_535 547 Y542 | SKRKGHEYTNIKY |
| 294 | PTPN11_573 587 Y580 | REDSARVYENVGLMQ |
| 295 | PTTG1_104_118_Y111 | VPASDDAYPEIEKFF |
| 112 | RAF1_331_343_S337/S338/Y339/Y340 | RPRGQRDSSYYWE |
| 113 | RASA1_453 465 Y460 | TVDGKEIYNTIRR |
| 114 | RB_804_816_S807/S811 | IYISPLKSPYKIS |
| 296 | RBM3_120_134_Y127 | SRPGGYGYGYGRSRD |
| 297 | RBMX_234_248_Y241 | YAPPPRDYTYRDYGH |
| 116 | RET_1022_1034_Y1029 | TPSDSLIYDDGLS |
| 298 | RET_Y1096 | RYPNDSVYANWMLSP |
| 299 | RICS_1673_1687_Y1680 | QYDNLEDYHSLPQHQ |
| 300 | RIPK2_374_388_Y381 | SRKAQDCYFMKLHHC |
| 118 | RON_1346_1358_Y1353 | SALLGDHYVQLPA |
| 126 | STAT3_698_710_Y705 | DPGSAAPYLKTKF |
| 301 | SACS_3345_3359_Y3352 | AKLEHLIYLKNRLSS |
| 302 | SCAMP3_79_93_Y86 | EPKNYGSYSTQASAA |
| 303 | SF3A3_472_486_Y479 | QPDTEEEYEDSSGNV |
| 304 | SHANK2_387_401_Y394 | GQMPENPYSEVGKIA |
| 305 | SHB_326_340_Y333 | KVTIADDYSDPFDAK |
| 306 | SHC1_233_247_Y240 | EPPDHQYYNDFPGKE |
| 307 | SIPA1L3_1162_1176_Y1169 | STPGSATYVRYKPSP |
| 308 | SLC20A2 370 384 Y377 | KPAQESNYRLLRRNN |
| 309 | SNIP_257_271_Y264 | IYRKEPLYAAFPGSH |
| 310 | SNRP70_139_153_Y146 | RSGKPRGYAFIEYEH |
| 311 | SNX3_15_29_Y22 | PQNLNDAYGPPSNFL |
| 312 | SORBS1_319_333_Y326 | RAEPKSIYEYQPGKS |
| 313 | SPAST_205_219_Y212 | SKSQTDVYNDSTNLA |
| 314 | SPRED2_261_275_Y268 | KHDYNYPYVDSSDFG |
| 315 | SPRY4_68_82_Y75 | TSHVENDYIDNPSLA |
| 316 | SRC Y215 | KLDSGGFYITSRTQF |
| 121 | SRC8_CHICK_476_488_Y477/Y483 | EYEPETVYEVAGA |
| 317 | STAM_377_391_Y384 | EDPMYSMYAKLQNQP |
| 318 | STAM2_185_199_Y192 | HTETKSLYPSSEIQL |
| 319 | STAM2_364_378_Y371 | LVNEAPVYSVYSKLH |
| 320 | STAT3_697_711_Y704 | ADPGAAPYLKTKFIC |
| 131 | TEC_512_524_Y519 | RYFLDDQYTSSSG |
| 321 | TENC1_486_500_Y493 | GPLDGSPYAQVQRPP |
| 322 | TIGD1_232_246_Y239 | KLKPMLIYHSENPRA |
| 323 | TJP2_1111_1125_Y1118 | AQKHPDIYAVPIKTH |
| 324 | TJP2_499_513_Y506 | SPEDEAIYGPNTKMV |
| 325 | TJP3_390_404_Y397 | RESSYDIYRVPSSQS |
| 326 | TMEPAI_225_239_Y232 | MEGPPPTYSEVIGHY |
| 327 | TNS3_347_361_Y354 | GPVDGSLYAKVRKKS |
| 328 | TXNRD1_124_138_Y131 | KVVYENAYGQFIGPH |
| 329 | TXNRD1_145_159_Y152 | NKGKEKIYSAERFLI |
| 133 | TYR03_679_691_Y686 | KIYSGDYYRQGCA |
| 330 | UBE2J1_1_15_Y5 | METRYNLKSPAVKRL |
| 100 | PECA1_706_718_Y713 | KKDTETVYSEVRK |
| 331 | VASP_32_46_Y39 | AFSRVQIYHNPTANS |
| 332 | VAV2_135_149_Y142 | TENDDDVYRSLEELA |
| 134 | VEGFR1_1049_1061_Y1053 | KNPDYVRKGDTRL |
| 136 | VEGFR2_944_956_Y951 | RFRQGKDYVGAIP |
| 333 | VEGFR3_Y1333 | ARGGQVFYNSEYGEL |
| 334 | VEGFR3_Y1337 | QVFYNSEYGELSEPS |
| 138 | VINC_815_827_Y821 | KSFLDSGYRILGA |
| 335 | | |
| 139 | ZAP70_485_497_Y492/Y493 | ALGADDSYYTARS |
| 140 | ZBT16_621_633_S628 | LRTHNGASPYQCT |
| 336 | ZNF326_129_143_Y136 | GSSWEAPYSRSKLRP |
| 337 | ZNF598_299_313_Y306 | GVVGGEDYEEVDRYS |

As used herein the peptide substrates are named as follows; SwissProt Entry Name for the Human protein_Start position of the peptide within the full length protein _ Stop position within the full length protein _ Position of the tyrosine or serine that can be phosphorylated.

The porous matrix as used herein, could be any material having oriented through-going channels as are generally known in the art, such as for example described in PCT patent publication WO 01/19517 and is typically made from a metal oxide, glass, silicon oxide or cellulose. In a particular embodiment the porous material is made of a metal oxide selected from the group consisting of zinc oxide, zirconium oxide, tin oxide, aluminum oxide, titanium oxide and thallium; in a more particular embodiment the metal oxide consists of aluminum oxide.

The samples used in the methods of the invention could in principle be any biological sample, such as for example blood, urine, saliva, tissue biopsy or autopsy material and then in particular cell lysates thereof, but would typically consist of cell lysates prepared from cell lines, including cancer cell lines; primary and immortalized tissue cell lines; non-human animal model biopsies and patient biopsies. In one embodiment of the invention, the cell lysates are prepared from cancer cell lines; xenograft tumors or cancer patient biopsies, including tumor and normal tissue.

It will be appreciated that the preferred method for the pretreatment of the samples will depend on the particular compound to be tested, and the type of sample used. The optimum method can be readily determined by those skilled in the art using conventional methods and in view of the information set out herein. For example, in the treatment of cell lysates, the compound to be tested may be added to the cell lysate directly; in non-human animal models the compound to be tested will typically be administered at a therapeutically effective amount of the particular compound, optionally in addition salt form, and/or combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous, or parenteral administration; or topical administration such as via inhalation, a nose spray, eye drops or via a cream, gel, shampoo or the like. The optimum method and order of administration and the dosage amounts and regime can be readily determined by those skilled in the art and as provided in more detail in the examples hereinafter.

In those embodiments of the present invention where the methods according to the invention are used to pharmacologically profile kinase inhibitors, the array of substrates is contacted with the pretreated sample in the presence of the compound to be tested. It is accordingly an object of the present invention to provide a method for obtaining a pharmacological profile of a kinase inhibitor using an array of substrates immobilized on a porous matrix, said method comprising;
(i) contacting the array of substrates with a sample in the presence of the kinase inhibitor;
(ii) contacting the array of substrates with a sample in the absence of the kinase inhibitor;
(iii) determine the response of said array to the sample in step (i);
(iv) determine the response of said array to the sample in step (ii); and
obtain the pharmacological profile as the difference in response of the array in steps (iii) and step (iv).

The response of the array of substrates is determined using a detectable signal, said signal resulting from the interaction of the sample with the array of substrates. As mentioned hereinbefore, in determining the interaction of the sample with the array of substrates the signal is either the result of a change in a physical or chemical property of the detectably labeled substrates, or indirectly the result of the interaction of the substrates with a detectably labeled molecule capable of binding to the substrates. For the latter, the molecule that specifically binds to the substrates of interest (e.g., antibody or polynucleotide probe) can be detectably labeled by virtue of containing an atom (e.g., radionuclide), molecule (e.g., fluorescein), or complex that, due to a physical or chemical property, indicates the presence of the molecule. A molecule may also be detectably labeled when it is covalently bound to or otherwise associated with a "reporter" molecule (*e.g*. , a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labeled avidin or streptavidin conjugate, magnetic beads (*e.g*., Dynabeads'), fluorescent dyes (*e.g*., fluorescein, fluorescein-isothiocyanate (FITC), Texas red, rhodamine, green fluorescent protein, enhanced green fluorescent protein, lissamine, phycoerythrin, Cy2, Cy3, Cy3.5, Cy5, Cy5.5, Cy7, FluorX [Amersham], SyBR Green I & II [Molecular Probes], and the like), radiolabels (*e.g*., ³H, ¹²⁵I, ³⁵S ¹⁴C, or ³²P), enzymes (*e.g*., hydrolases, particularly phosphatases such as alkaline phosphatase, esterases and glycosidases, or oxidoreductases, particularly peroxidases such as horse radish peroxidase, and the like), substrates, cofactors, inhibitors, chemilluminescent groups, chromogenic agents, andcolorimetric labels such as colloidal gold or colored glass or plastic (e. g., polystyrene, polypropylene, latex, etc.) beads. Patents teaching the use of such labels include U. S. Pat. Nos. 3,817, 837; 3,850, 752; 3,939, 350; 3,996, 345; 4,277, 437; 4,275, 149; and 4,366, 241.

Means of detecting such labels are well known to those of skill in the art. Thus, for example, chemiluminescent and radioactive labels may be detected using photographic film or scintillation counters, and fluorescent markers may be detected using a photodetector to detect emitted light (*e.g.*, as in fluorescence-activated cell sorting). Enzymatic labels are typically detected by providing the enzyme with a substrate and detecting a colored reaction product produced by the action of the enzyme on the substrate. Colorimetric labels are detected by simply visualizing the colored label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the color associated with the label. Fluorescence resonance energy transfer has been adapted to detect binding of unlabeled ligands, which may be useful on arrays.

In a particular embodiment of the present invention the response of the array of substrates to the sample is determined using detectably labeled antibodies; more in particular fluorescently labeled antibodies. In those embodiments of the invention where the substrates consist of kinase substrates, the response of the array of substrates is determined using fluorescently labeled anti-phosphotyrosine antibodies. As outlined in more detail in the examples hereinafter, the use of fluorescently labeled anti-phosphotyrosine antibodies allows real-time determination of the substrate activity and accordingly provides the possibility to express the array activity as the initial kinase velocity. In this embodiment the pharmacological profile is determined as the ratio of the array substrate response to the (pre)treated samples *over* the array substrate response to the untreated samples.

Hence, in a particular embodiment the present invention provides a method for obtaining a pharmacological profile of a kinase inhibitor using an array of substrates immobilized on a porous matrix, said method comprising;
(i) contacting the array of substrates with a sample in the presence of the kinase inhibitor;
(ii) contacting the array of substrates with a sample in the absence of the kinase inhibitor;
(iii) determine the response of said array to the sample in step (i);
(iv) determine the response of said array to the sample in step (ii); and
obtain the pharmacological profile as the ratio of the array substrate response in step (iii) *over* the array substrate response in step (iv).

In a further aspect of this embodiment, and any of the embodiments of the invention;
- the samples consist of cell lysates obtainable from any biological sample (supra), in particular cell lysates prepared from cancer cell lines; xenograft tumors or cancer patient biopsies, including tumor and normal tissue.
- the array of substrates consist of kinase substrates, in particular peptide kinase substrates, more in particular comprising at least 2, 6, 12, 40, 50, 60, 70, 80, 90, 100, 110, 120, 130 or 140 peptides of the peptide kinase substrates of Table 1. In an even further embodiment the array of substrates comprising at least 2 peptides selected from the group consisting of peptides with sequence numbers 4, 19, 24, 69, 113, 114, 136 and 138, or from the group consisting of peptides with sequence numbers 15, 16, 22, 34, 62, 83, 86, 87, 100, 105, 108, 110, 113, 125, 129, and 133 or from the group consisting of peptides with sequence numbers 15, 16, 21, 23, 38, 42, 53, 62, 69, 77, 83, 86, 91, 94, 103, 112, 114, 129, 133, 136 and 138, or from the group consisting of peptides with sequence numbers 5, 10, 38, 30, 54, 57, 68, 72, 73, 74, 82, 91, 98, 99, 102, 104, 110, 135, 118, 119, 71, 138. Alternatively, the array of substrates consist of kinase substrates, more in particular peptide kinase substrates, even more particular the peptide kinase substrates in Table 2, most particular using at least 2, 6, 12, 16, 20, 24, 28, 32, 36, 40, 50, 60, 70, 80, 90, 100, 110, 120, 130, 150, 180, 200, 220 or 240 peptides of the peptide kinase substrates of Table 2. In an even further embodiment the array of substrates the array of substrates consist of the peptides with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138.
- the response of the array of substrates is determined using antibodies, in particular fluorescently labeled antibodies, more in particular fluorescently labeled anti-phosphotyrosine antibodies; most particular using the commercially available PY20-FITC antibodies. It has been observed that the presence of antifungal compound, such as azoles or azide, in the sample could influence the array response, thus in a particular embodiment the response of the array of substrates is done using antibodies, in any of the above embodiments, in a solution free of antifungals, in particular using an azide free solution.
- the samples are filtered prior to the incubation on the porous matrix, i.e. prior to the contacting of the substrate array with the samples in both steps (i) and (ii). Filtering of the samples is done using art known procedures, such as for example but not limited to filter membranes made from regenerated cellulose, cellulose esters, nylon, polypropylene, glass, anopore or teflon, typically the filter range should match the oriented through-going channels of the porous matrix, and is for example in the 10 to 0.1 micrometer range. In particular using an inorganic 0.2 micrometer filter, more in particular using an anopore 0.2 micrometer filter, as provided in the examples hereinafter.
- the cell lysates used in step (i) are pre-incubated with the kinase inhibitor. It will be appreciated that the preferred method for the pre-incubation of the cell lysates will depend on the particular compound to be tested, and the type of cell lysates used. The optimum method can be readily determined by those skilled in the art using conventional methods, but would typically consist of incubating the cell lysates in the appropriate buffer, for example M-PER buffer (PIERCE) containing phosphatase and protease inhibitors, with the compound to be tested for a period in the range of 30 min - 60 min. Again, the incubation is typically done on ice.

Hence, in a further embodiment the present invention provides a method for obtaining a pharmacological profile of a kinase inhibitor using an array of substrates immobilized on a porous matrix, said method comprising;
(i) obtain cell lysates prepared from cancer cell lines; xenograft tumors or cancer patient biopsies, including tumor and normal tissue;
(ii) filter the cell lysates over a filter in the 10 to 0.1 micrometer range;
(iii) incubate a fraction of said cell lysates with the kinase inhibitor to be tested;
(iv) contact the array of substrates in the presence of the kinase inhibitor to be tested, with the incubated fraction of step (iii);
(v) contact the array of substrates in the absence of the kinase inhibitor to be tested, with the fraction of cell lysates which was not incubated with the kinase inhibitor;
(vi) determine the response of said array in step (iv);
(vii) determine the response of said array in step (v); and
obtain the pharmacological profile as the ratio of the array substrate response in step (iv) *over* the array substrate response in step (v).

In this embodiment, and any of the embodiments of the invention, one or more of the following further restrictions may apply;
- the array of substrates consist of kinase substrates, in particular peptide kinase substrates, more in particular comprising at least 2, 6, 12, 40, 50, 60, 70, 80, 90, 100, 110, 120, 130 or 140 peptides of the peptide kinase substrates of Table 1. In an even further embodiment the array of substrates comprising at least 2 peptides selected from the group consisting of peptides with sequence numbers 4, 19, 24, 69, 113, 114, 136 and 138; from the group consisting of 15, 16, 21, 23, 38, 42, 53, 62, 69, 77, 83, 86, 91, 94, 103, 112, 114, 129, 133, 136 and 138, from the group consisting of 15, 16, 22, 34, 62, 83, 86, 87, 100, 105, 108, 110, 113, 125, 129, and 133 or from the group consisting of 5, 10, 38, 30, 54, 57, 68, 72, 73, 74, 82, 91, 98, 99, 102, 104, 110, 135, 118, 119, 71, 138. Alternatively, the array of substrates consist of kinase substrates, more in particular peptide kinase substrates, even more particular the peptide kinase substrates in Table 2, most particular using at least 2, 6, 12, 16, 20, 24, 28, 32, 36, 40, 50, 60, 70, 80, 90, 100, 110, 120, 130, 150, 180, 200, 220 or 240 peptides of the peptide kinase substrates of Table 2. In an even further embodiment the array of substrates consist of the peptides with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138.
- the response of the array of substrates is determined using antibodies, in particular fluorescently labeled antibodies, more in particular fluorescently labeled anti-phosphotyrosine antibodies; most particular using the commercially available PY20-FITC antibodies. It has been observed that the presence of antifungal compound, such as azoles or azide, in the sample could influence the array response, thus in a particular embodiment the response of the array of substrates is done using antibodies, in any of the above embodiments, in a solution free of antifungals, in particular using an azide free solution.
- Filtering of the samples is done using art known procedures, such as for example but not limited to filter membranes made from regenerated cellulose, cellulose esters, nylon, polypropylene, glass, anopore or teflon, typically the filter range should match the oriented through-going channels of the porous matrix, and is for example in the 10 to 0.1 micrometer range. In particular using an inorganic 0.2 micrometer filter, more in particular using an anopore 0.2 micrometer filter, as provided in the examples hereinafter.

In another embodiment, the present invention provides the use of the pharmacological profile determined using the methods of the invention, to enable the distinction between responders and non-responders in the treatment of cells, tissues, organs or warm-blooded animals with the compound to be tested. In particular to enable the distinction of responders from non-responders cell lines and tumors to the treatment with the compound to be tested.

As outlined in the examples hereinafter, it has also been an object of the present invention to provide the identification of specific sets of substrates, herein also referred to as "molecular signature" or "fingerprint', on the substrate array of Table 1, that enables the distinction of responders from non-responders cell lines and tumors to the treatment with a class of macrocyclic quinazoline derivatives (I) below, described as multi targeted kinase inhibitors (MTKI) in PCT patent publication WO 2004/10765. (PRD2071)

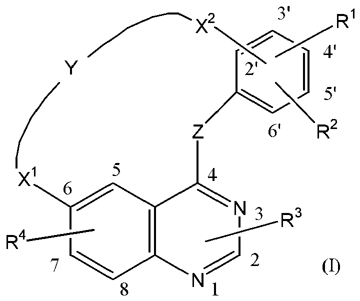

In particular to the treatment with a compound of formula (I) wherein;
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, - C₁₋₃alkyl-NH-CO-Het²⁰-, or -Het²²-CH₂-CO-NH-C₁₋₃alkyl-;
X¹ represents O, or -O-C₁₋₂alkyl-;
X² represents a direct bond, -C₁₋₂alkyl-, O, -O-C₁₋₂alkyl-, NR¹² or NR¹²-C₁₋₂alkyl-;
R¹ represents hydrogen, cyano, halo or hydroxy, preferably halo;
R² represents hydrogen, cyano, halo, or hydroxy;
R³ represents hydrogen;
R⁴ represents C₁₋₄alkyloxy-;
R¹² represents hydrogen, or C₁₋₄alkyl-;
R¹³ represents hydrogen or C₁₋₄alkyl;
R¹⁴ represents hydrogen or C₁₋₄alkyl;
Het²⁰ represents pyrrolidinyl, piperazinyl or piperidinyl; and
Het²² represents pyrrolidinyl, piperazinyl or piperidinyl.

In particular to enable the distinction of responders from non-responders cell lines and tumors to the treatment with 26483327-AAA 4,6-ethanediylidenepyrimido[4,5-b][6,1,12]benzoxadiazacyclopentadecine, 17-bromo-8,9,10,11,12,13,14,19-octahydro-20-methoxy-13-methyl- (***MTKI1***); or the pharmaceutically acceptable acid or base addition salts thereof.

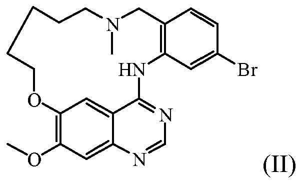

As used in the foregoing definitions and hereinafter,
- halo is generic to fluoro, chloro, bromo and iodo;
- C₁₋₂alkyl defines methyl or ethyl;
- C₁₋₃alkyl defines straight and branched chain saturated hydrocarbon radicals having from 1 to 3 carbon atoms such as, for example, methyl, ethyl, propyl and the like;
- C₁₋₄alkyl defines straight and branched chain saturated hydrocarbon radicals having from 1 to 4 carbon atoms such as, for example, methyl, ethyl, propyl, butyl, 1-methylethyl, 2-methylpropyl, 2,2-dimethylethyl and the like;
- C₁₋₅alkyl defines straight and branched chain saturated hydrocarbon radicals having from 1 to 5 carbon atoms such as, for example, methyl, ethyl, propyl, butyl, pentyl, 1-methylbutyl, 2,2-dimethylpropyl, 2,2-dimethylethyl and the like;
- C₃₋₉alkyl defines straight and branched chain saturated hydrocarbon radicals having from 3 to 9 carbon atoms such as propyl, butyl, pentyl, hexyl, heptyl, octyl, nonyl and the like;
- C₁₋₄alkyloxy defines straight or branched saturated hydrocarbon radicals such as methoxy, ethoxy, propyloxy, butyloxy, 1-methylethyloxy, 2-methylpropyloxy and the like;
- the term "CO" refers to a carbonyl group.

The pharmaceutically acceptable acid or base addition salts as mentioned hereinabove are meant to comprise the therapeutically active non-toxic acid and non-toxic base addition salt forms which MTKI 1 is able to form. The basic properties can be converted in their pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e*. butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

The acidic properties may be converted in their pharmaceutically acceptable base addition salts by treating said acid form with a suitable organic or inorganic base. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The terms acid or base addition salt also comprise the hydrates and the solvent addition forms which MTKI 1 is able to form. Examples of such forms are e.g. hydrates, alcoholates and the like.

Using the pharmacological profiling methods of the present invention (supra) with the substrates of Table 1, the "molecular signature" for MTKI1 was found to comprise at least 2 peptides selected from the group consisting of peptides with sequence numbers 15, 16, 22, 34, 62, 83, 86, 87, 100, 105, 108, 110, 113, 125, 129, and 133.

Using the pharmacological profiling methods of the present invention (supra) with the substrates of Table 2, the "molecular signature" for MTKI1 was found to comprise at least 2 peptides selected from the group consisting of peptides with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138.

It is accordingly an object of the present invention to provide a method to enable the distinction of responders from non-responders cell lines and tumors to the treatment with 4,6-ethanediylidenepyrimido[4,5-b][6,1,12] benzoxadiazacyclopentadecine, 17-bromo-8,9,10,11,12,13,14,19-octahydro-20-methoxy-13-methyl- (***MTKI 1***); or the pharmaceutically acceptable acid or base addition salts thereof; said method comprising;
(i) obtaining a sample from said cell lines and/or tumors;
(ii) contacting an array of substrates with said sample in the presence of MTKI1;
(iii) contacting an array of substrates with said sample in the absence of MTKI1;
(iv) determine the response of said array to the sample in step (ii);
(v) determine the response of said array to the sample in step (iii); and
obtain the pharmacological profile as the ratio in response of the array in steps (iv) over step (v); characterized in that the array of substrates comprises at least 2 peptides selected from the group consisting of peptides with sequence numbers 4, 19, 24, 69, 113, 114, 136 and 138; and wherein a responder is identified as an inhibition (ratio < 1.0) in response for at least two of the peptides selected from peptides with sequence numbers 15, 16, 22, 34, 62, 83, 86, 87, 100, 105, 108, 110, 113, 125, 129, and 133.

In this embodiment, one or more of the following further restrictions may apply;
- the array of substrates is immobilized on a matrix, more in particular on a porous matrix, such as for example, but not limited to any material having oriented through-going channels as are generally known in the art, such as for example described in PCT patent publication WO 01/19517 and is typically made from a metal oxide, glass, silicon oxide or cellulose. In a particular embodiment the porous material is made of a metal oxide selected from the group consisting of zinc oxide, zirconium oxide, tin oxide, aluminum oxide, titanium oxide and thallium; in a more particular embodiment the metal oxide consists of aluminum oxide;
- the sample could in principle be any biological sample, such as for example blood, urine, saliva, tissue biopsy or autopsy material and then in particular cell lysates thereof, but would typically consist of cell lysates prepared from cell lines, including cancer cell lines; primary and immortalized tissue cell lines; non-human animal model biopsies and patient biopsies. In one embodiment of the invention, the cell lysates are prepared from cancer cell lines; xenograft tumors or cancer patient biopsies, including tumor and normal tissue;
- the array of substrates comprising at least 3, 4, 5, 6 or 7 peptides selected from the group consisting of peptides with sequence numbers 15, 16, 22, 34, 62, 83, 86, 87, 100, 105, 108, 110, 113, 125, 129, and 133.; in particular the array of substrates comprises the peptides with sequence numbers 15, 16, 22, 34, 62, 83, 86, 87, 100, 105, 108, 110, 113, 125, 129, and 133; more in particular the array of substrates consists of the peptides with sequence numbers 15, 16, 22, 34, 62, 83, 86, 87, 100, 105, 108, 110, 113, 125, 129, and 133;
- the response of the array of substrates is determined using a detectable signal, said signal resulting from the interaction of the sample with the array of substrates. As mentioned hereinbefore, in determining the interaction of the sample with the array of substrates the signal is either the result of a change in the physical or chemical property of the detectably labeled substrates, or indirectly the result of the interaction of the substrates with a detectably labeled molecule capable of binding to the substrates. In an even further embodiment, the response of the array of substrates is determined using antibodies, in particular fluorescently labeled antibodies, more in particular fluorescently labeled anti-phosphotyrosine antibodies; most particular using the commercially available PY20-FITC antibodies. It has been observed that the presence of antifungal compound, such as azoles or azide, in the sample could influence the array response, thus in a particular embodiment the response of the array of substrates is determined using antibodies, in any of the above embodiments, in a solution free of antifungals, in particular using an azide free solution.
- a responder is identified as an inhibition in response (ratio at least < 0.80) for at least three of the peptides selected from peptides with sequence numbers 15, 16, 22, 34, 62, 83, 86, 87, 100, 105, 108, 110, 113, 125, 129, and 133.

The aforementioned method could optionally comprise one or more of the following additional steps;
- an additional step in which a fraction of the sample is preincubated with MTKI1 prior to its application on the array of subtrates.
- an additional step in which the samples are filtrated prior to their application on the array of substrates. Filtering of the samples is done using art known procedures, such as for example but not limited to filter membranes made from regenerated cellulose, cellulose esters, nylon, polypropylene, glass, anopore or teflon, typically the filter range should match the oriented through-going channels of the porous matrix, and is for example in the 10 to 0.1 micrometer range. In particular using an inorganic 0.2 micrometer filter, more in particular using an anopore 0.2 micrometer filter, as provided in the examples hereinafter.

### EXPERIMENTAL DATA AND EXAMPLES

### Materials and Methods:

### The use of PAMCHIPs in prediction of drug response and in compound differentiation.

PAMGENE technology using the tyrosine peptide PAMCHIP96 allows the kinetic detection of the phosphorylation of 144 tyrosine peptides spotted onto a 3-dimensional porous well of a 96-well plate (www.pamgene.com). This technology can be used to measure the activity of purified kinases and the effects of kinase inhibitors thereon.

Phosphotyrosine peptides are detected using a mix of fluorescently labeled anti-phosphotyrosine antibodies. Phosphorylation of peptides can be followed in real time due to the possibility of removing the reaction mix for a few seconds during the assay by a pumping mechanism to allow CCD camera detection of phosphopeptides bound to the fluorescent antibody. These consecutive datapoints (quantified spot images) generate a kinetic curve for each individual peptide, and allow for the calculation of initial velocities and endpoints associated with each individual peptide.

This technology has been modified to enable profiling of tyrosine kinase activities in untreated and compound-treated lysates (lysates prepared from cancer cell lines, xenograft tumors and patient biopsies, tumor and normal tissue, can all be used), as well as treated and untreated cell lines. The use of these profiles was demonstrated in stratifying compound responder from non responders cell lines and tumors, as well as in the differentiation of tyrosine kinase inhibitors based on their target selectivity and ATP competitiveness, as well as in characterizing the indirect effect of non-kinase inhibitors (such as histone deacetylase inhibitors) on kinase activity.

### Cultured cell lysates

Cells cultured in appropriate medium in T175 flasks, treated with compound if necessary, are harvested at 70-90% confluency.

Media is removed, cells are washed with10ml ice cold PBS containing 0.1mM NaVO₄ and lysed in 1ml of M-PER buffer (PIERCE) containing phosphatase and protease inhibitors (HALT, PIERCE). Cells are collected by scraping, and centrifuged for 10 minutes at 12 000xg. Supernatant lysates are filtered through a 0.2 micrometer filter (Anotop 0.2 µm anopore filter; Whatmann). Lysates are flash frozen in liquid nitrogen and stored at-80C.

### Tumor and tissue lysates

100 mg of frozen tumor or tissue blocks, or sections, are lysed in 1ml of M-PER buffer (PIERCE) containing phosphatase and protease inhibitors (HALT, PIERCE). The suspension is mixed using a ULTRA-TURRAX blender (IKA werke), and centrifuged twice for 10 minutes at 12 000xg. Supernatant lysates are filtered through a 0.2 micrometer filter (Anotop 0.2 µm anopore filter; Whatmann). Lysates are flash frozen in liquid nitrogen and stored at-80C.

### PAMCHIP procedure

Just prior to the PAMCHIP experiment, total protein content is quantitated in the lysates and lysates are diluted to 1 mg/ml in a total volume of 200 µl, with M-PER phosphatase and protease inhibitors (HALT, PIERCE). 10 µg of total lysate (corresponding to 10 µl) will be used per well on the PAMCHIP96.

To all 200 µl lysates 10 units of benzonase (Novagen) is added and lysates are further incubated for 30 min on ice and next centrifuged for 10 minutes at 12 000xg. The supernatant is then 1:1 diluted with compound or DMSO diluted to the appropriate concentration in water, and lysates are further incubated exactly 60 min on ice. Meanwhile, a PAMCHIP96 tyrosine peptide chip (PAMGENE) is blocked with 2% BSA (filtered through a 0.2 µm anopore filter; Whatmann) using the standard setting of a PAMSTATION96 (PAMGENE).

The composition of the kinase buffer is as follows: 1x ABL buffer (NEB), 0.5mg/ml BSA (from 10 mg/ml NEB stock), 100 µM ATP (dissolved and diluted in 1x ABL buffer), 5 µg/ml PY20-FITC (ExAlpha Biologicals, azide-free preparation). A twofold concentrated kinase buffer is prepared chilled on ice, DMSO or compound is added as appropriate, and the solution is added 1:1 to the (compound treated) ice cold lysates, and immediately applied to a tyrosine peptide PAMCHIP96. The assay is run on a PAMSTATION96 according to standard procedures: after loading of the plate, solution is pumped once onto the wells, a first exposure is read (150 mSec) and then a kinetic reading program (reading every 5 min for 150 mSec) is run for 60 min. Lysates are run in 6 technical replicates for each condition; replicates are always applied horizontally next to each other to avoid vertical strip effects of the chip.

### Data analysis

### Preprocessing.

The data (spot images) are further quantified and linked to the peptide identities using EVOLVE PAMGRID software (PAMGENE). The quantified data are then fit to a curve using CurveFitHT software (PAMGENE) using the Vinip2 curve fitting algorithm, applied on the first 30 min of the assay; the initial velocity is derived at the first kinetic time point read. All subsequent data analyses were done on initial velocities (Vini) using the software packages Omniviz and R (Ihaka, R. and Gentlemen, R. (1996) R: a language for data analysis and graphics. J. Comput. Graph. Stat., 5, 299-314).

### Testing for differentially inhibited peptides.

After replacing the Vini's lower than 0.1 by 0.1, all Vini's were log2-transformed to approach normality. Subsequently, it was tested whether the compound-induced reduction in Vini's differed significantly between responding and non-responding tumors. This test was done for each peptide separately using a mixed model (Littell, R.C., Milliken, G.A., Stroup, W.W., Wolfinger, R.D. 1996. SAS system for mixed models. Cary, North Carolina: SAS Institute Inc.) allowing the incorporation of both technical variation (i.e., the variation across replicates) and biological variation (i.e., the variation among the various responding and non-responding cell lines or tumors). Variation between cell lines or tumors in Vini and in treatment effect on Vini was modeled respectively by random intercepts and random compound effects per cell line or tumor, nested in response status. The difference in compound-induced reduction in Vini between responding and non-responding cell lines or tumors was tested through the interaction between compound treatment and response status, both modeled as fixed effects. The p-values for the test for this interaction were corrected for multiple testing using False Discovery Rate procedure (Benjamini, Y. and Hochberg, Y. 1995. Controlling the False Discovery Rate: A Practical and Powerful Approach to Multiple Testing. J. Roy. Stat. Soc. B, 57(1): 289-300), resulting in so-called q-values.

### Example 1: Generation of signature peptides in a 27-cell lines panel for MTKI.

It was tested whether the Pamchip peptide arrays are a useful tool to predict the response of a cell line to the multi-targeted kinase inhibitor, MTKI. MTKI inhibits the proliferation of a range of cell lines *in vitro* with widely varying IC₅₀'s. The lysates prepared from these 27 cell lines, representing diverse cancer cell types, were profiled on the peptide arrays in the absence or presence of 5µM MTKI, in 6 replicates for each condition. In this panel, 13 cell lines were defined as responder cell lines (IC₅₀ < 3µM; cell lines are ordered according to increasing IC50 in Example 1, whereas 14 cell lines are classified as 'non-responder' cell lines (IC₅₀ > 10 µM). Intermediary cell lines and cell lines with highly variable IC₅₀ measurements were omitted from the analysis. The experiment included the highly responsive H3255 lung cancer cell line, which overexpresses mutant EGFR L858R. This mutant variant is highly responsive to MTKI, which is an ATP-competitive compound that binds to the active conformation of EGFR (similar to erlotinib and gefitinib). Accordingly, the most profound changes induced by MTKI in lysate kinase activity can be observed for this highly responsive cell line.

To identify those peptides for which the inhibition of phosphorylation by MTKI correlates well with the response status of the cell line from which the lysate was prepared, a statistical mixed-model was set up to identify those peptides that are most likely to change after MTKI treatment in responder cell lysates compared to non-responder lysates, taken into account the inter-replicate variability (see the material and methods section for a more detailed description of the statistical test). The 16 peptides that are most likely to change by MTKI treatment in responders versus non-responders are listed in Example 1, using a cut-off p-value of 0.01 (correction for the large number of repeated measurements in this statistical analysis indicates that this p-value translates to a q value of 0.1). To illustrate that the inhibition of the phosphorylation of these peptides in a given lysate does indeed correlate with the response of the corresponding cell line to MTKI, the following cut-off was chosen to classify responders and non-responders: minimally 4 peptides out of the 16 peptide list should be inhibited with a ratio of less than 0.8 (more than 20% inhibition) to classify as a responder. Applying this rule to all 27 cell lines indicates an overall classification accuracy of 82% (with 78% sensitivity, and 86% specificity). Interestingly, two out of three responders that are falsely predicted as non-responders (Colo699 and H2009) have the highest IC50 of all 13 responders (ie these are 'borderline' responders). Note that the use of these 20 peptides as a classification tool can be optimized according to the required sensitivity and specificity.

Results represented by the ratio of initial velocity of peptide phosphorylation in the presence of solvent (DMSO) over the initial velocity of peptide phosphorylation in the presence of MTKI are shown in Tables 3 A to D .; 'NaN indicates that there is no detectable phosphorylation in the presence of DMSO

In order to adapt the sensitivity or specificity to specific needs (e.g. to eliminate false positives or alternatively false negatives as much as possible), the skilled person can adapt the prediction statistics easily, e.g. by changing the Responder prediction ratio.

**Table 3A**

| peptides | H3255 MTKI | NCI-N87 MTKI | H322 MTKI | SNU484 MTKI | H2122 MTKI | SKBR3 MTKI | A431 MTKI |
|---|---|---|---|---|---|---|---|
| CDK7_157_169_S164 | **0.55** | 0.82 | 0.88 | **0.67** | 0.90 | 0.97 | **0.53** |
| CREB1_122_134_Y134/S133 | **0.73** | 0.82 | 0.94 | **0.70** | 0.85 | 0.99 | **0.63** |
| DDR2_733_745_Y740 | **0.44** | 0.95 | 0.85 | **0.75** | 0.81 | 0.93 | **0.56** |
| ENOG_37_49_Y43 | **0.76** | 0.91 | **0.76** | **0.68** | **0.71** | **0.**77 | 0.83 |
| GSK3B_209_221_Y216 | **0.60** | 0.83 | 0.94 | **0.78** | 0.84 | 0.89 | **0.56** |
| MK 10_216_228_T221/Y223 | **0.47** | **0.73** | **0.76** | **0.72** | **0.79** | **0.68** | **0.46** |
| NCF1_313_325_S315/S320 | **0.59** | 0.89 | 0.88 | 0.80 | 0.94 | 0.94 | **0.57** |
| NPT2_501_513_T508 | **0.68** | 0.88 | 0.88 | 0.85 | 0.90 | **0.**77 | **0.53** |
| PECA1_706_718_Y713 | **0.68** | 0.82 | **0.73** | **0.64** | **0.67** | **0.68** | **0.72** |
| PGFRB_768_780_Y771/Y77 5/Y778 | **0.12** | **0.32** | 0.92 | NaN | NaN | NaN | 0.97 |
| PLCG1_764_776_Y771 | 0.97 | **0.78** | **0.79** | **0.70** | **0.76** | **0.75** | 0.82 |
| PRRX2_202_214_Y214 | **0.67** | 0.82 | 0.85 | **0.67** | 0.84 | **0.75** | **0.75** |
| RASA1_453 465 Y460 | **0.65** | **0.59** | 0.83 | **0.58** | **0.78** | 0.88 | **0.62** |
| STAT2_683_695_Y690 | **0.72** | 0.83 | 0.89 | **0.73** | 0.90 | 0.81 | **0.57** |
| SYN1_2_14_S9 | **0.70** | **0.75** | 0.91 | **0.67** | 0.91 | 1.03 | **0.58** |
| TYR03_679_691_Y686 | **0.47** | 0.85 | 0.96 | 0.85 | 0.95 | 0.90 | 0.52 |
| **status** | R | R | R | R | R | R | R |
| **prediction** | R | R | R | R | R | R | R |

**Table 3B**

| peptides | SUM159 MTKI | BT474OD MTKI | DU145 MTKI | SUM149 MTKI | H2009 MTKI | Colo699 MTKI | HT1373 MTKI |
|---|---|---|---|---|---|---|---|
| CDK7_157_169_S164 | 0.83 | **0.74** | **0.63** | **0.72** | 0.89 | 0.93 | 1.10 |
| CREB1_122_134_Y134/S1 33 | 1.03 | 0.90 | 0.93 | 0.88 | 0.97 | 0.86 | 0.95 |
| DDR2_733_745_Y740 | 0.83 | 0.83 | **0.71** | 0.83 | 0.95 | 1.20 | 0.92 |
| ENOG_37_49_Y43 | 0.82 | **0.55** | **0.65** | 0.91 | 0.81 | 0.86 | 0.87 |
| GSK3B_209_221_Y216 | 1.13 | 0.87 | 0.94 | **0.41** | 1.00 | 1.03 | 0.97 |
| MK 10_216_228_T221/Y22 3 | 0.89 | **0.76** | **0.66** | **0.79** | 0.89 | 0.86 | 0.87 |
| NCF1_313_325_S315/S320 | 0.99 | 0.88 | 0.87 | 1.06 | 0.89 | 0.93 | 1.02 |
| NPT2_501_513_T508 | 0.98 | **0.77** | 0.90 | **0.78** | **0.76** | 0.95 | 1.00 |
| PECA1_706_718_Y713 | 0.87 | **0.51** | **0.73** | 0.87 | **0.79** | 0.83 | 0.86 |
| PGFRB_768_780_Y771/Y7 75/Y778 | NaN | NaN | **0.39** | **0.58** | **0.43** | **0.23** | NaN |
| PLCG1_764_776_Y771 | **0.79** | **0.68** | **0.76** | 0.95 | 0.81 | **0.78** | **0.76** |
| PRRX2_202_214_Y214 | 0.84 | **0.56** | **0.68** | **0.71** | 0.90 | **0.76** | 0.91 |
| RASA1_453 465 Y460 | 0.82 | **0.61** | **0.69** | 0.93 | 0.83 | 0.81 | **0.78** |
| STAT2_683_695_Y690 | 1.09 | 0.91 | 0.93 | 0.89 | 0.84 | 0.93 | 0.97 |
| SYN1_2_14_S9 | 1.03 | 0.94 | 0.92 | 1.02 | 0.93 | 0.95 | 0.92 |
| TYRO3_679_691_Y686 | 0.87 | 0.88 | **0.68** | **0.76** | 0.89 | 1.18 | 0.87 |
| **status** | R | R | R | R | R | R | NR |
| **prediction** | NR | R | R | R | NR | NR | NR |

**Table 3C**

| peptides | MDA MB435s MTKI | RSJSA1 MTKI | HEK239 MTKI | H23 MTKI | SHSY5 Y MTKI | H1650 MTKI | MDA231PAR MTKI |
|---|---|---|---|---|---|---|---|
| CDK7_157_169_S164 | 1.08 | 0.81 | 1.20 | 0.92 | 0.97 | 0.82 | 1.13 |
| CREB1_122_134_Y134/S133 | 1.09 | 0.99 | 1.08 | 1.02 | 1.17 | 0.88 | 1.04 |
| DDR2_733_745_Y740 | 1.07 | 1.02 | 1.00 | 0.81 | 1.01 | 0.85 | 1.11 |
| ENOG_37_49_Y43 | 0.84 | 0.95 | 0.96 | 0.80 | 0.99 | **0.73** | 0.93 |
| GSK3B_209_221_Y216 | 1.14 | 1.11 | 1.18 | 1.09 | 0.87 | **0.79** | 0.98 |
| MK10_216_228_T221/Y223 | 1.29 | 0.96 | **0.75** | 0.98 | 1.10 | **0.69** | 0.83 |
| NCF1_313_325_S315/S320 | 1.09 | 1.00 | 0.96 | 0.86 | 1.26 | 0.92 | 1.03 |
| NPT2_501_513_T508 | 1.05 | 0.96 | 0.89 | 0.91 | 1.17 | 0.91 | 1.09 |
| PECA1_706_718_Y713 | **0.76** | 0.85 | 1.00 | **0.62** | 0.96 | **0.70** | 0.86 |
| PGFRB_768_780_Y771/Y775 /Y778 | NaN | NaN | NaN | NaN | NaN | **0.32** | NaN |
| PLCG1_764_776_Y771 | 0.98 | 0.90 | 1.02 | 0.84 | 0.96 | 0.84 | 0.93 |
| PRRX2_202_214_Y214 | 1.03 | 1.03 | 1.30 | **0.67** | 0.92 | 0.91 | 0.97 |
| RASA1_453 465 Y460 | 0.88 | 1.17 | 0.89 | **0.71** | 0.87 | 0.98 | 0.80 |
| STAT2_683_695_Y690 | 1.09 | 1.03 | 1.03 | 0.93 | 1.17 | 0.85 | 1.00 |
| SYN1_2_14_S9 | 1.12 | 1.05 | 0.92 | 1.01 | 1.11 | 0.83 | 0.92 |
| TYRO3_679_691_Y686 | 0.93 | 1.22 | 0.93 | 1.10 | 0.95 | 0.90 | 0.99 |
| **status** | NR | NR | NR | NR | NR | NR | NR |
| **prediction** | NR | NR | NR | NR | NR | R | NR |

**Table 3D**

| peptides | MOLT4 | MCF7 | MKN45 | SNU5 | GTL16 | U118MG |
|---|---|---|---|---|---|---|
| | MTKI | MTKI | MTKI | MTKI | MTKI | MTKI |
| CDK7_157_169_S164 | 0.91 | 0.89 | 0.90 | 0.98 | 0.98 | 0.85 |
| CREB1_122_134_Y134/S133 | 1.02 | 0.85 | 0.94 | 1.01 | 0.91 | 0.99 |
| DDR2_733_745_Y740 | **0.78** | 1.15 | 0.94 | 0.95 | 1.00 | 1.00 |
| ENOG_37_49_Y43 | **0.**77 | 0.83 | 0.91 | 0.81 | 0.80 | 0.85 |
| GSK3B_209_221_Y216 | 1.19 | 0.89 | 0.99 | 0.98 | 1.11 | 1.06 |
| MK10_216_228_T221/Y223 | 0.85 | 1.03 | 0.95 | 0.99 | 0.96 | 0.97 |
| NCF1_313_325_S315/S320 | 1.18 | 0.90 | 0.95 | 0.99 | 0.96 | 1.05 |
| NPT2_501_513_T508 | 0.96 | 0.90 | 0.97 | 0.98 | 0.89 | 0.94 |
| PECA1_706_718_Y713 | **0.**77 | **0.74** | 0.83 | 0.81 | 0.95 | 0.92 |
| PGFRB_768_780_Y771/Y775 /Y778 | **0.17** | NaN | 1.04 | 0.89 | **0.75** | NaN |
| PLCG1_764_776_Y771 | 0.80 | **0.78** | 0.92 | 0.84 | 0.90 | 0.91 |
| PRRX2_202_214_Y214 | **0.72** | 0.90 | 1.04 | 0.89 | 0.97 | 1.03 |
| RASA1_453 465 Y460 | **0.79** | 0.81 | 0.92 | 0.92 | 0.81 | 0.94 |
| STAT2_683_695_Y690 | 0.95 | 0.91 | 0.97 | 0.91 | 1.06 | 0.97 |
| SYN1_2_14_S9 | 1.22 | 0.92 | 0.94 | 1.01 | 0.91 | 1.02 |
| TYRO3_679_691_Y686 | 0.97 | 0.98 | 1.00 | 0.89 | 0.92 | 0.91 |
| **status** | NR | NR | NR | NR | NR | NR |
| **prediction** | R | NR | NR | NR | NR | NR |

R=Responder to MTKI

NR=Non-Responder to MTKI

A responder is predicted when minimally 4 peptides have a ratio <0.8 (indicated in **bold**)

Overall prediction accuracy: : 82% (22/27)

Sensitivity (number of predicted Responders over number of actual Responders): 78% (10/13)

Specificity (number of predicted Non-Responders over number of actual Non-Responders): 86% (12/14)

### Example 2: Generation of signature peptides in xenograft tumors for MTKI.

To explore whether a similar approach allows for the classification of responder and non-responder tumors, the same strategy as above was applied to 12 different xenograft tumors, which were subcutaneously grown in nude immuno-compromised mice from human cancer cell lines. These tumors can be divided in 6 responsive tumors and 6 non-responsive tumors. The responsive tumors are arranged according to their responsiveness to MTKI in vivo, ranging from abrupt tumor regression of H3255, A431 and H322 tumors, to potent inhibition of tumor growth for DU145, SUM149 and BT474. The non-responsive tumors (H460 and H441, HT29, PC3, SKOV3, H1703) are all non-responsive to MTKI in vivo. Lysates were made from homogenized frozen tumor blocks and analyzed in the absence or presence of 5 µM MTKI, as before. Overall, the phosphorylation rate of most peptides was significantly faster in tumor lysates compared to the corresponding cell lines. Nevertheless, inhibition of peptide phosphorylation profiles indicated that there is a subset of peptides for which the inhibition of phosphorylation correlated with responsiveness of the tumors to MTKI. A statistical test was applied to this data set -similar as before for the 27 cell lines- and the 21 peptides that best discriminate between responders and non-responders is shown in Tables 4A and B. The cut-off p-value used for this set is 0.1. Note that the p-values for these peptides to discriminate between responders and non-responder tumors are significantly lower than those derived for the cell lines because the number of tumors (12) is far lower then the number of cell lines in Example 1 (27). Note that 7 of the peptides in this tumor signature set were also found in the 16-peptide cell line signature set (Example 1) indicating that inhibition of some of the same activities are critical to reduce tumor growth in vivo as compared to inhibition of growth in vitro, but also pointing to the importance of other kinases in the *in vivo* setting. Importantly, for many signature peptides the extent of inhibition correlated with the responsiveness of the tumor to MTKI.

Results represented by the ratio of initial velocity of peptide phosphorylation in the presence of solvent (DMSO) over the initial velocity of peptide phosphorylation in the presence of MTKI are shown in Tables 4A-B.; 'NaN indicates that there is no detectable phosphorylation in the presence of DMSO

In order to adapt the sensitivity or specificity to specific needs (e.g. to eliminate false positives or alternatively false negatives as much as possible), the skilled person can adapt the prediction statistics easily, e.g. by changing the Responder prediction ratio.

**Table 4A**

| Peptide | H3255 | A431 | H322 | DU145 | SUM149 | BT474 |
|---|---|---|---|---|---|---|
| | MTKI | MTKI | MTKI | MTKI | MTKI | MTKI |
| CDK7_157_169_S164 | **0.45** | **0.60** | **0.77** | 0.96 | 0.93 | 0.88 |
| CREB1_122_134_Y134/S133 | **0.61** | **0.67** | 0.80 | 0.93 | 1.02 | 1.02 |
| DDR1_785_797_Y792/Y796/Y797 | **0.48** | **0.58** | **0.71** | **0.64** | 0.88 | 0.94 |
| DYR1A_212_224_Y219 | **0.62** | **0.03** | **0.59** | 0.90 | **0.10** | **0.07** |
| EPHA4_589_601_Y596 | **0.44** | **0.51** | **0.63** | **0.77** | 1.03 | **0.79** |
| EPHB1_921_933_Y928 | **0.44** | **0.39** | **0.75** | 0.81 | 0.85 | **0.70** |
| FAK1_569_581_Y576/Y577 | **0.48** | **0.58** | **0.82** | **0.72** | 0.94 | 0.89 |
| GSK3B_209_221_Y216 | **0.57** | **0.67** | **0.77** | 1.07 | 0.91 | 0.89 |
| K2C6E_53_65_S59 | **0.56** | **0.54** | 0.80 | **0.77** | 0.86 | 0.80 |
| MBP_259_271_Y261/Y268/S266 | **0.47** | **0.60** | **0.65** | 0.81 | 1.04 | 0.85 |
| MK 10_216_228_T221/Y223 | **0.31** | **0.60** | **0.68** | **0.77** | **0.75** | 0.83 |
| NCF1_313_325_S315/S320 | **0.73** | **0.66** | **0.78** | 0.97 | 0.94 | 1.02 |
| NTRK2_699_711_Y702/Y706/Y707 | **0.48** | **0.46** | **0.76** | **0.**77 | 0.85 | **0.**77 |
| P2AB_297_309_T304/Y307 | **0.66** | **0.65** | **0.77** | 0.81 | 0.87 | **0.62** |
| PGFRB_572_584_Y579/Y581 | **0.66** | **0.64** | **0.67** | **0.72** | **0.69** | **0.75** |
| RAF1_331_343_S337/S338/Y339/Y3 40 | **0.53** | **0.58** | **0.79** | 0.83 | 0.99 | 0.94 |
| RB_804_816_S807/S811 | **0.52** | **0.53** | **0.75** | **0.76** | 0.83 | 0.80 |
| SYN1_2_14_S9 | **0.50** | **0.76** | **0.78** | 0.88 | 1.01 | 0.96 |
| TYRO3_679_691_Y686 | **0.51** | **0.55** | 0.80 | 0.83 | 1.03 | 0.84 |
| VEGFR2_944_956_Y951 | **0.52** | **0.58** | **0.75** | 0.95 | 0.86 | 0.84 |
| VINC_815_827_Y821 | **0.40** | **0.52** | **0.76** | 0.84 | **0.75** | 0.85 |
| response status | R | R | R | R | R | R |
| response prediction | R | R | R | R | NR | NR |

**Table 4B**

| Peptide | SKOV 3MTKI | PC3 MTKI | H460 MTKI | H441 MTKI | HT29 MTKI | H1703 MTKI |
|---|---|---|---|---|---|---|
| CDK7_157_169_S164 | 0.89 | 0.82 | 0.98 | 0.93 | 0.96 | 0.77 |
| CREB1_122_134_Y134/S133 | 1.03 | 0.86 | 1.02 | 0.95 | 1.00 | 0.93 |
| DDR1_785_797_Y792/Y796/Y797 | 0.87 | 0.83 | 1.01 | **0.77** | 0.85 | 0.96 |
| DYR1A_212_224_Y219 | **0.67** | **0.44** | NaN | 0.83 | **0.50** | NaN |
| EPHA4_589_601_Y596 | 1.20 | 0.83 | 2.10 | 0.86 | 1.08 | 0.87 |
| EPHB1_921_933_Y928 | **0.**77 | 0.81 | 1.01 | 0.85 | 1.03 | **0.64** |
| FAK1_569_581_Y576/Y577 | 0.88 | 0.83 | 1.12 | 0.82 | **0.**77 | 0.73 |
| GSK3B_209_221_Y216 | 1.06 | **0.**77 | 0.95 | 0.97 | 0.98 | 0.98 |
| K2C6E_53_65_S59 | 0.85 | **0.71** | 0.97 | **0.79** | 0.82 | 0.94 |
| MBP_259_271_Y261/Y268/S266 | 1.16 | 0.84 | 1.02 | **0.79** | 0.89 | 0.89 |
| MK10_216_228_T221/Y223 | 0.84 | **0.67** | 0.96 | 0.85 | **0.73** | 0.84 |
| NCF 1_313_325_S315/5320 | 0.91 | 0.83 | 1.01 | 0.93 | 0.92 | 0.95 |
| NTRK2_699_711_Y702/Y706/Y707 | **0.**77 | **0.73** | 0.99 | 0.80 | 0.87 | 0.88 |
| P2AB_297_309_T304/Y307 | 1.37 | 0.84 | 1.06 | 0.92 | 1.00 | 0.88 |
| PGFRB_572_584_Y579/Y581 | **0.61** | **0.62** | 1.12 | **0.74** | **0.68** | **0.**77 |
| RAF1_331_343_S337/S338/Y339/Y3 40 | 1.01 | 0.85 | 1.03 | 0.88 | 0.97 | 0.94 |
| RB_804_816_S807/S811 | 0.94 | **0.61** | 0.96 | 0.82 | **0.75** | 0.86 |
| SYN1_2_14_S9 | 1.00 | **0.75** | 1.07 | 0.98 | 1.01 | 0.91 |
| TYRO3_679_691_Y686 | 0.98 | 0.81 | 1.03 | 0.83 | 0.92 | 0.95 |
| VEGFR2_944_956_Y951 | 0.90 | **0.**77 | 0.91 | 0.91 | 1.01 | 0.82 |
| VINC_815_827_Y821 | 0.**79** | **0.66** | 0.85 | 0.88 | 0.89 | 0.80 |
| response status | NR | NR | NR | NR | NR | NR |
| response prediction | NR | R | NR | NR | NR | NR |

R=Responder to MTKI

NR=Non-Responder to MTKI

A Responder is predicted when minimally 7 peptides have a ratio < 0.8 (indicated in bold font).

Overall prediction accuracy: 9/12 (75%)

Sensitivity (number of predicted Responders over number of actual Responders): 4/6 (67%).

Specificity (number of predicted Non-Responders over number of actual Non-Responders): 5/6 (83%).

### Example 3: Human frozen tissue and tumor samples.

The same approach as for the xenograft tumor lysates was applied to human tissue (frozen tissue purchased from Proteogenex). Snap-frozen lung tumors and normal matched tissue were tested. It was found that all samples gave robust signals, and that different tumors resulted in different responses to MTKI1 treatment, suggesting that this technology is applicable to frozen human tissue in general and may predict response of tumors (and other tissue) to MTKI1 or other tyrosine kinase inhibitors.

### Example 4: Compound differentiation

PAMCHIPS can also be used to profile and differentiate compounds. Five tyrosine kinase inhibitors (from left to right: DMSO, lapatinib/Tykerb, MTKI1/MTKI, gefitinib/Iressa, erlotinib/Tarceva, ZD6474/zactima) result in a distinct inhibition profile in lysates prepared from the responsive cell line NCI-H3255. Likewise, profiles have been generated from less responsive cell lines to differentiate compounds. Results are represented in fig. 1

It was found that also non-kinase inhibitors can have profound effects on the PAMCHIP profile, when lysates are prepared from compound treated cells. An example is given where either a histone deacetylase inhibitor (JNJ-26481585) or MTKI1 is applied on DU-145 prostate cancer cells at 5µM for 20 hours. A robust effect of the histone deacetylase inhibitor is observed on the PAMCHIP profile after cellular treatment, whereas little effect can be seen when lysates are treated with a histone deacetylase inhibitor, consistent with the idea that this compound indirectly inhibits kinases in a cellular context. This illustrates that any compound, when applied to cells, can potentially be profiled using PAMCHIPS. Results are represented in fig. 2.

### Example 5: Design of a novel 256-peptide array- Generation of signature peptides in xenograft tumors for MTKI.

To improve the specificity of the standard 140 peptide array and therefore the ability of peptide sets to discriminate between responders and non-responders, a novel set of 256 peptides was selected. The following strategy was used: first, phosphotyrosine peptides were identified in lysates of two cancer cell lines, DU145 and NCI-N87, by mass spectrometry (in collaboration with Cell Signalling Technologies). This yielded 937 phosphopeptides, many of which were never described earlier. 63 peptides of particular interest were added: known autophosphorylation sites or other substrates of tyrosine kinases that were underrepresented in the 937 peptides. All of these 1000 peptides were then synthesized using the SPOT methodology (a fast, cost-effective peptide synthesis procedure by JPT, Berlin, Germany), and spotted on 7 different 144 peptide arrays. These arrays were incubated with 32 different cell line and tumor lysates, in the absence and presence of MTKI. From these peptides 194 peptides were selected based on (a) detectability in at least one of the lysates, (b) differential phosphorylation rates across the lysates, and (c) differential inhibition by MTKI in various lysates. In addition 62 peptides were added from the standard 140-peptide array that were found useful in previous experiments (such as those in Examples 1 to 4). The selected 256 peptides were then synthesized using standard high quality procedures by JPT (custom synthesis) and spotted as a single array by Pamgene. This new array (256-peptides) was used to profile 12 xenograft tumor lysates as before in the absence or presence of MTKI. The same mixed-model statistical analysis as before was used to select peptides that discriminate responder versus non-responder tumors. Results of that analysis are shown in Example 5 (28 peptides with P value < 0.1). The fold inhibition of many of these peptides by MTKI is higher compared to the peptides selected from the 140 peptide array (see Example 2), indicating that these peptides are indeed more selective for certain MTKI target kinases. Most importantly, prediction accuracy is greatly improved compared to the peptide set derived from the 140-peptide set, illustrating the usefulness of the peptide selection strategy described above.

### Results are shown in Tables 5A and B

Ratio of initial velocity of peptide phosphorylation in the presence of solvent (DMSO) over the initial velocity of peptide phosphorylation in the presence of MTKI; 'NaN indicates that there is no detectable phosphorylation in the presence of DMSO

**Table 5A**

| Peptides | H3255 MTKI | A431 MTKI | H322 MTKI | DU145 MTKI | SUM149 MTKI | BT474 MTKI |
|---|---|---|---|---|---|---|
| ABLIM1_350_364_Y357 | 0.82 | **0.57** | 0.72 | 0.81 | **0.49** | 0.71 |
| ACHB_383_395_Y390 | **0.05** | **0.35** | **0.29** | **0.01** | **0.68** | **0.67** |
| C11orf35_217_231_Y224 | **0.14** | **0.36** | **0.05** | NaN | 0.75 | 0.80 |
| CBL_667_681_Y674 | 0.80 | **0.48** | **0.65** | 0.72 | **0.02** | **0.18** |
| CFL1_61_75_Y68 | **0.55** | **0.36** | **0.63** | **0.60** | **0.04** | **0.07** |
| DOK1_402_416_Y409 | 0.72 | **0.32** | 0.80 | 0.74 | **0.52** | 0.83 |
| BCAR1_380_394_Y387 | **0.11** | **0.13** | 0.74 | **0.51** | **0.05** | 0.82 |
| EPHB3_607_621_Y614 | **0.60** | 0.88 | **0.65** | 0.73 | **0.19** | **0.13** |
| FGFR1OP_330 344 Y33 7 | **0.65** | 0.96 | 1.04 | 0.97 | **0.51** | 0.94 |
| FKS | 0.71 | **0.55** | **0.68** | **0.69** | **0.11** | **0.12** |
| FYN_206_220_Y213 | **0.28** | **0.51** | **0.29** | **0.05** | 1.24 | **0.66** |
| KIRREL 550 564 Y557 | **0.22** | 0.78 | **0.69** | **0.63** | NaN | **0.17** |
| LAT_249_261_Y255 | **0.50** | 0.83 | 0.82 | 0.88 | **0.09** | 1.12 |
| LPHN2_1343_1357_Y13 50 | 0.83 | 0.87 | **0.70** | 0.75 | **0.10** | **0.47** |
| LYN_186_200_Y193 | **0.28** | **0.08** | **0.38** | **0.13** | **0.06** | **0.69** |
| MAPK8_178_192_Y185 | **0.29** | **0.41** | **0.27** | **0.02** | 0.74 | **0.62** |
| MCP_362_376_Y369 | 1.04 | **0.11** | **0.61** | 0.76 | **0.04** | **0.25** |
| MET_1227_1239_Y1230/ Y1234/Y1235 | 0.72 | 0.99 | **0.66** | **0.43** | **0.08** | **0.53** |
| NPT2_501_513_T508 | **0.55** | **0.66** | **0.22** | **0.05** | 0.92 | 0.87 |
| NAPG_298_312_Y307 | **0.59** | 0.75 | 0.79 | 0.74 | **0.26** | **0.69** |
| NCF1_313_325_S315/S3 20 | **0.47** | **0.57** | **0.22** | **0.10** | 0.80 | 0.93 |
| P2RY2_223_237_Y230 | **0.39** | **0.68** | **0.41** | **0.50** | 0.93 | 0.96 |
| P85A_600_612_Y607/S6 08 | **0.34** | **0.68** | 0.74 | 0.73 | **0.21** | 0.73 |
| RBM3_120_134_Y127 | **0.39** | 0.71 | **0.58** | **0.36** | 1.01 | 0.99 |
| SF3A3_472_486_Y479 | NaN | NaN | 0.85 | **0.33** | NaN | **0.53** |
| SHB_326_340_Y333 | **0.17** | **0.28** | 0.93 | 0.72 | NaN | **0.49** |
| SLC20A2_370_384_Y377 | **0.14** | **0.53** | **0.20** | **0.05** | 0.84 | 0.81 |
| VEGFR2_944_956_Y951 | **0.12** | **0.49** | **0.22** | **0.11** | 1.55 | 0.81 |
| response status | R | R | R | R | R | R |
| response prediction | R | R | R | R | R | R |

**Table 5B**

| Peptides | SKOV3 | U87MG | PC3 | H460 | H441 | HT29 |
|---|---|---|---|---|---|---|
| | MTKI | MTKI | MTKI | MTKI | MTKI | MTKI |
| ABLIM1_350_364_Y357 | 0.79 | 0.73 | 0.71 | 0.87 | 0.70 | 0.79 |
| ACHB_383_395_Y390 | **0.53** | **0.32** | **0.42** | NaN | **0.60** | **0.55** |
| C11orf35_217_231_ Y224 | 0.83 | **0.67** | **0.56** | NaN | **0.36** | 0.73 |
| CBL_667_681_Y674 | **0.48** | **0.50** | 0.82 | **0.68** | **0.55** | 0.71 |
| CFL1_61_75_Y68 | **0.52** | **0.52** | 0.71 | **0.67** | **0.46** | **0.63** |
| DOK1_402_416_Y409 | **0.68** | 0.81 | 0.85 | 0.77 | 0.72 | 0.97 |
| BCAR1_380_394_Y387 | **0.57** | **0.45** | **0.68** | 0.85 | **0.68** | 0.73 |
| EPHB3_607_621_Y614 | **0.67** | 0.72 | 0.83 | **0.66** | **0.63** | **0.63** |
| FGFR1OP_330_344_Y33 7 | 0.98 | 1.14 | 0.90 | 0.91 | 0.90 | 0.91 |
| FKS | **0.59** | **0.66** | 0.75 | 0.71 | **0.54** | **0.69** |
| FYN_206_220_Y213 | 0.83 | **0.61** | **0.40** | NaN | 0.73 | **0.52** |
| KIRREL 550 564 Y557 | 0.91 | **0.54** | 0.78 | 0.72 | **0.56** | **0.69** |
| LAT_249_261_Y255 | 0.78 | 1.09 | 0.96 | 0.71 | 0.93 | 0.87 |
| LPHN2_1343_1357_Y13 50 | 0.77 | **0.69** | 0.80 | 0.72 | **0.64** | 0.73 |
| LYN_186_200_Y193 | **0.49** | **0.48** | 0.76 | NaN | **0.57** | 0.78 |
| MAPK8_178_192_Y185 | **0.54** | **0.54** | **0.47** | NaN | **0.37** | **0.61** |
| MCP_362_376_Y369 | **0.65** | 0.71 | 0.83 | **0.67** | **0.55** | **0.64** |
| MET_1227_1239_Y1230/ Y1234/Y1235 | 0.78 | **0.64** | 0.81 | **0.67** | **0.64** | 0.90 |
| NPT2_501_513_T508 | 0.91 | 0.82 | **0.59** | 0.92 | 0.82 | 0.75 |
| NAPG_298_312_Y307 | 0.73 | 0.88 | 0.80 | 0.70 | 0.79 | 0.73 |
| NCF1_313_325_S315/S3 20 | 0.85 | 0.77 | **0.44** | 0.73 | 0.83 | **0.60** |
| P2RY2_223_237_Y230 | 0.92 | 0.87 | 0.79 | 0.85 | 0.80 | **0.64** |
| P85A_600_612_Y607/S6 08 | **0.63** | 1.01 | 0.85 | 0.73 | 0.76 | 0.72 |
| RBM3_120_134_Y127 | 0.86 | 0.76 | **0.60** | 0.89 | 0.72 | 0.71 |
| SF3A3_472_486_Y479 | **0.48** | 1.08 | 1.20 | **0.47** | 0.93 | 0.96 |
| SHB_326_340_Y333 | 0.96 | 0.83 | 0.92 | **0.68** | 0.87 | 1.16 |
| SLC20A2_370_384_Y377 | 0.88 | 0.95 | **0.49** | **0.63** | 0.76 | **0.59** |
| VEGFR2_944_956_Y951 | **0.63** | 0.76 | **0.69** | NaN | 0.77 | **0.36** |
| response status | NR | NR | NR | NR | NR | NR |
| response prediction | NR | NR | NR | NR | NR | NR |

R=Responder to MTKI

NR=Non-Responder to MTKI

A Responder is predicted when minimally 14 peptides have a ratio < 0.7 (indicated in bold font).

Overall accuracy: **100%**

### Example 6: MTKI-derived signature peptides can also be used to predict response to other EGFR inhibitors, such as Tarceva (erlotinib).

To test whether other EGFR inhibitors, related to MTKI, such as Tarceva (erlotinib; OSI pharmaceuticals), are also amenable to this response prediction methodology, 9 cell lines were profiled in the presence of MTKI, or Tarceva (5 µM in both cases). Although minor differences are detectable in the MTKI and Tarceva profiles (eg MTKI is slightly more potent on H3255, consistent with a lower IC50 on EGFR 1858R compared to Tarceva; MTKI is also more potent on N87, a cell line largely driven by Her2, for which MTKI is a better inhibitor than Tarceva; on the other hand Tarceva has additional activity in GTL16 lysates compared to MTKI, for unknown reasons), overall there is a striking resemblance in the inhibition pattern between MTKI and Tarceva (see figure 3). The value of the previously derived MTKI prediction peptides is illustrated in Tables 6A to C. Based on these peptides also response to Tarceva can be predicted with identical accuracy as for MTKI (and with the same false predictions, in casu H2009 and MOLT4). These results illustrate that the 16 peptides that correlate with response to MTKI likely also predict reponse to other EGFR inhibitors, such as Tarceva (erlotinib), or Iressa (gefitinib; AstraZeneca).

Ratio of initial velocity of peptide phosphorylation in the presence of solvent (DMSO) over the initial velocity of peptide phosphorylation in the presence of MTKI or Tarceva; 'NaN indicates that there is no detectable phosphorylation in the presence of DMSO.

**Table 6A**

| Peptides | H3255 | | A431 | | DU145 | |
|---|---|---|---|---|---|---|
| | MTKI | TARC | MTKI | TARC | MTKI | TARC |
| CDK7_157_169_S164 | **0.04** | **0.12** | **0.70** | 0.80 | **0.36** | **0.26** |
| CREB1_122_134_Y134/S133 | **0.35** | **0.46** | **0.71** | 0.81 | 0.80 | **0.78** |
| DDR2_733_745_Y740 | **0.13** | **0.18** | **0.73** | 0.83 | **0.44** | **0.50** |
| ENOG_37_49_Y43 | **0.74** | **0.80** | 0.91 | 0.84 | **0.72** | **0.67** |
| GSK3B_209_221_Y216 | **0.10** | **0.16** | **0.67** | **0.72** | **0.33** | **0.55** |
| MK10_216_228_T221/Y223 | **0.16** | **0.19** | **0.70** | **0.78** | **0.65** | **0.63** |
| NCF1_313_325_S315/S320 | **0.39** | **0.50** | **0.70** | **0.78** | 0.80 | **0.79** |
| NPT2_501_513_T508 | **0.35** | **0.50** | **0.58** | **0.70** | **0.76** | **0.57** |
| PECA1_706_718_Y713 | **0.78** | 0.80 | 0.96 | 0.82 | **0.74** | **0.68** |
| PGFRB_768_780_Y771/Y775/Y 778 | NaN | NaN | 1.15 | 1.00 | **0.64** | **0.49** |
| PLCG1_764_776_Y771 | 0.95 | 0.90 | 0.94 | 0.84 | **0.76** | **0.69** |
| PRRX2_202_214_Y214 | **0.62** | **0.66** | 0.90 | 0.80 | **0.64** | **0.75** |
| RASA1_453 465 Y460 | **0.42** | **0.47** | 0.84 | **0.72** | **0.62** | **0.56** |
| STAT2_683_695_Y690 | **0.28** | **0.39** | **0.72** | 0.82 | **0.79** | **0.71** |
| SYN1_2_14_S9 | **0.25** | **0.30** | **0.71** | 0.81 | **0.72** | **0.65** |
| TYRO3_679_691_Y686 | **0.12** | **0.17** | **0.68** | **0.78** | **0.42** | **0.48** |
| **response status** | R | R | R | R | R | R |
| **response prediction** | R | R | R | R | R | R |

**Table 6B**

| Peptides | N87 | | SKBR3 | | SUM149 | |
|---|---|---|---|---|---|---|
| | MTKI | TARC | MTKI | TARC | MTKI | TARC |
| CDK7_157_169_S164 | **0.05** | **0.04** | NaN | NaN | NaN | NaN |
| CREB1_122_134_Y134/S133 | **0.71** | 0.82 | 0.82 | **0.70** | 0.94 | 0.94 |
| DDR2_733_745_Y740 | **0.36** | **0.34** | **0.03** | **0.02** | NaN | NaN |
| ENOG_37_49_Y43 | **0.89** | 0.95 | 0.80 | 0.82 | 0.85 | 0.86 |
| GSK3B_209_221_Y216 | **0.26** | **0.46** | **0.30** | **0.21** | NaN | NaN |
| MK10_216_228_T221/Y223 | **0.58** | **0.78** | **0.21** | **0.47** | **0.03** | **0.07** |
| NCF1_313_325_S315/5320 | **0.79** | 0.91 | **0.79** | **0.78** | 1.05 | 1.18 |
| NPT2_501_513_T508 | 0.82 | 0.96 | **0.75** | **0.74** | 1.11 | 1.34 |
| PECA1_706_718_Y713 | **0.73** | **0.76** | 0.96 | **0.74** | **0.60** | **0.68** |
| PGFRB_768_780_Y771/Y775/Y 778 | NaN | NaN | 0.09 | 1.63 | **0.40** | **0.52** |
| PLCG1_764_776_Y771 | 0.89 | 0.86 | **0.74** | 0.81 | 0.84 | 0.80 |
| PRRX2_202_214_Y214 | **0.65** | **0.72** | **0.57** | **0.41** | **0.62** | 0.80 |
| RASA1_453_465_Y460 | **0.76** | 0.83 | **0.42** | **0.23** | **0.37** | **0.39** |
| STAT2_683_695_Y690 | 0.85 | 0.95 | **0.69** | **0.70** | 0.85 | 0.95 |
| SYN1_2_14_S9 | **0.79** | 0.93 | **0.69** | **0.67** | **0.43** | **0.78** |
| TYR03_679_691_Y686 | **0.44** | **0.40** | **0.05** | **0.04** | NaN | NaN |
| **response status** | R | R | R | R | R | R |
| **response prediction** | R | R | R | R | R | R |

**Table 6C**

| Peptides | H2009 | | GTL16 | | MOLT4 | |
|---|---|---|---|---|---|---|
| | MTKI | TARC | MTKI | TARC | MTKI | TARC |
| CDK7_157_169_S164 | 1.22 | 1.28 | 1.04 | 1.06 | 0.85 | 0.68 |
| CREB1_122_134_Y134/S133 | 1.16 | 1.13 | 1.09 | 0.99 | 0.90 | 0.83 |
| DDR2_733_745_Y740 | 1.15 | 1.08 | 1.08 | 1.01 | 0.91 | 0.81 |
| ENOG_37_49_Y43 | 0.90 | 0.96 | 0.99 | 0.98 | **0.73** | **0.72** |
| GSK3B_209_221_Y216 | 1.09 | 1.15 | 1.00 | 0.99 | 0.88 | **0.79** |
| MK 10_216_228_T221/Y223 | 1.14 | 1.16 | 1.03 | 0.96 | 0.82 | **0.78** |
| NCF1_313_325_S315/S320 | 1.10 | 1.07 | 1.03 | 0.96 | 0.91 | 0.82 |
| NPT2_501_513_T508 | 1.08 | 1.03 | 0.98 | 0.91 | 0.89 | **0.69** |
| PECA1_706_718_Y713 | 0.88 | 0.84 | 0.92 | 0.86 | **0.64** | **0.72** |
| PGFRB_768_780_Y771/Y775/Y 778 | **0.71** | **0.75** | 0.80 | 0.89 | **0.60** | **0.63** |
| PLCG1_764_776_Y771 | 0.87 | 0.86 | 0.95 | 0.92 | 0.85 | **0.74** |
| PRRX2_202_214_Y214 | 0.92 | 0.86 | 0.96 | **0.75** | **0.64** | **0.71** |
| RASA1_453_465_Y460 | 1.01 | 1.00 | 1.04 | 0.98 | **0.67** | **0.75** |
| STAT2_683_695_Y690 | 1.17 | 1.12 | 1.02 | 0.97 | 1.11 | 1.06 |
| SYN1_2_14_S9 | 1.17 | 1.17 | 1.07 | 1.02 | 0.97 | 0.86 |
| TYR03_679_691_Y686 | 1.10 | 1.03 | 0.99 | 0.91 | 0.88 | 0.76 |
| **response status** | R | R | NR | NR | NR | NR |
| **response prediction** | NR | NR | NR | NR | R | R |

A responder is predicted when minimally 4 peptides <0.8

MTKI: Accuracy: 78% (7/9); Sensitivity: 86% (6/7); Specificity: 50% (1/2)

Tarceva: Accuracy: 78% (7/9); Sensitivity: 86% (6/7); Specificity: 50% (1/2)

### Example 7: Inhibition of cMet in cell lysates can be correlated to a specific peptide set.

To examine whether the currently available 140-tyrosine peptide arrays from Pamgene will also detect other kinase activities then those described above, another compound was profiled using the same methodology: 6-{Difluoro-[6-(1-methyl-1H-pyrazol-4-yl)-[1,2,4]triazolo[4,3-b]pyridazin-3-yl]-methyl}-quinoline, referred to as compound 605 for the purpose of this description; this is an exquisitely selective inhibitor of the cMET receptor tyrosine kinase (see US2007 0203136 A1) and with formula :

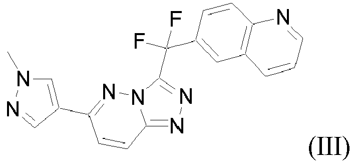

Similarly, a range of cell lines with varying responsiveness to the cMET inhibitor 605 in HGF-induced colony formation, were profiled (in this case 1µM compound was sufficient for significant inhibition of peptide phosphorylation in the responder cell lysates). As shown in Example 7, clear-cut differences in peptide phosphorylation inhibition by 605 could be identified in the responder lysates versus non-responder lysates. Note that the cMet signature peptide set features two peptides derived from Ron, the closest homologue to cMet. 605 does not inhibit purified Ron kinase at 1µM at all. However, these Ron sites have been demonstrated to be targets for phosphorylation by cMet (Follenzi et al., 2000, Oncogene 19, 4041-3049). The 605 signature peptides show no overlap at all with the MTKI signature peptides set, consistent with the absence of any overlap in kinase specificity between MTKI and 605. These data illustrate that for two types of tyrosine kinase inhibitors, the multi-targeted EGFR inhibitor, MTKI (and Tarceva), and the selective cMET inhibitor, 605, specific peptides can be identified of which the compound mediated inhibition of phosphorylation rates in sample lysates predict actual biological response of these samples to the compounds using the herein described methodology.

Results are shown in Tables 7A to C.

**Table 7A**

| Peptides | MKN45 605 | SNU5 605 | Kato2 605 | H441 605 | H1792 605 |
|---|---|---|---|---|---|
| ANXA1_13_25_Y20/T23 | **0.03** | **0.06** | **0.27** | **0.22** | 1.16 |
| CALM_95_107_Y99/S101 | **0.14** | **0.03** | **0.11** | **0.14** | **0.53** |
| EPHA4_589_601_Y596 | **0.27** | **0.26** | **0.29** | **0.32** | **0.77** |
| EGFR_1165_1177_Y1172 | **0.22** | **0.02** | **0.10** | **0.43** | **0.33** |
| FAK2_572_584_Y579/Y580 | **0.57** | **0.38** | **0.45** | **0.56** | 0.86 |
| FGFR1_759_771_Y766 | **0.10** | **0.04** | **0.01** | **0.01** | NaN |
| JAK1_1015_1027_Y1022/Y102 3 | **0.07** | **0.03** | **0.18** | **0.02** | 0.96 |
| LAT_194_206_Y200 | **0.11** | **0.04** | **0.07** | **0.16** | **0.04** |
| LAT_249_261_Y255 | **0.59** | **0.26** | **0.64** | **0.71** | 1.04 |
| LCK_387_399_Y394 | **0.01** | **0.01** | **0.01** | NaN | NaN |
| MK07_211_223_T218/Y220 | **0.41** | **0.29** | **0.31** | **0.37** | 0.85 |
| NTRK2_699_711_Y702/Y706/Y 707 | **0.64** | **0.43** | **0.42** | **0.54** | 0.86 |
| PDPK1_2_14_Y9 | **0.56** | **0.35** | **0.37** | **0.39** | 0.93 |
| PDPK1_369_381_Y373/Y376 | **0.36** | **0.10** | **0.27** | **0.23** | 0.84 |
| PGFRB_1002_1014_Y1009 | **0.19** | **0.13** | **0.05** | **0.02** | 1.05 |
| PGFRB_709_721_Y716 | **0.46** | **0.25** | **0.21** | **0.36** | 1.01 |
| PRRX2_202_214_Y214 | **0.44** | **0.28** | **0.27** | **0.33** | 0.81 |
| VEGFR2 1052 1064 Y1059 | **0.02** | **0.04** | **0.03** | **0.05** | 0.84 |
| RON_1346_1358_Y1353 | **0.12** | **0.06** | **0.08** | **0.05** | **0.71** |
| RON_1353_1365_Y1356/Y1360 | **0.04** | **0.16** | **0.17** | **0.07** | NaN |
| KSYK_518_530_Y525/Y526 | **0.26** | **0.20** | **0.14** | **0.22** | **0.79** |
| VINC_815_827_Y821 | **0.55** | **0.36** | **0.34** | **0.49** | **0.79** |
| response status | R | R | R | R | R |
| response prediction | R | R | R | R | R |

**Table 7B**

| Peptides | WIDR 605 | A549 605 | YKG 605 | N87 605 | Caco2 605 |
|---|---|---|---|---|---|
| ANXA1_13_25_Y20/T23 | **0.49** | 0.92 | NaN | **0.77** | NaN |
| CALM_95_107_Y99/S101 | **0.31** | NaN | NaN | **0.12** | NaN |
| EPHA4_589_601_Y596 | **0.59** | 1.08 | 1.36 | 0.88 | **0.75** |
| EGFR_1165_1177_Y1172 | 0.85 | 1.34 | NaN | 0.86 | NaN |
| FAK2_572_584_Y579/Y580 | 0.91 | 0.90 | 0.92 | 0.97 | 1.05 |
| FGFR1_759_771_Y766 | NaN | NaN | NaN | NaN | NaN |
| JAK1_1015_1027_Y1022/Y102 3 | **0.39** | 1.91 | NaN | NaN | NaN |
| LAT_194_206_Y200 | 0.86 | NaN | NaN | **0.63** | NaN |
| LAT_249_261_Y255 | 1.02 | 1.24 | **0.69** | 0.91 | NaN |
| LCK_387_399_Y394 | NaN | NaN | NaN | NaN | NaN |
| MK07_211_223_T218/Y220 | 0.87 | 0.97 | **0.79** | 1.06 | 0.80 |
| NTRK2_699_711_Y702/Y706/Y 707 | 0.88 | 0.95 | 1.09 | 0.96 | 0.92 |
| PDPK1_2_14_Y9 | 0.85 | 1.02 | 1.00 | **0.70** | **0.**77 |
| PDPK1_369_381_Y373/Y376 | **0.65** | 1.12 | NaN | **0.59** | NaN |
| PGFRB_1002_1014_Y1009 | NaN | NaN | NaN | NaN | NaN |
| PGFRB_709_721_Y716 | **0.71** | 1.10 | NaN | **0.78** | NaN |
| PRRX2_202_214_Y214 | **0.74** | 0.84 | 1.23 | **0.78** | 0.83 |
| VEGFR2 1052 1064 Y1059 | **0.54** | 0.81 | NaN | **0.68** | NaN |
| RON_1346_1358_Y1353 | **0.12** | **0.37** | NaN | NaN | NaN |
| RON_1353_1365_Y1356/Y1360 | **0.04** | NaN | NaN | NaN | NaN |
| KSYK_518_530_Y525/Y526 | 0.81 | 1.16 | **0.46** | 1.02 | 0.95 |
| VINC_815_827_Y821 | **0.71** | 0.81 | 1.13 | 0.96 | 0.89 |
| response status | R | NR | NR | NR | NR |
| response prediction | R | NR | NR | R | NR |

**Table 7C**

| Peptides | H322 605 | SNU484 605 | H2122 605 | H2106 605 |
|---|---|---|---|---|
| ANXA1_13_25_Y20/T23 | 1.08 | NaN | 1.04 | **0.69** |
| CALM_95_107_Y99/S101 | NaN | NaN | **0.49** | **0.14** |
| EPHA4_589_601_Y596 | 0.82 | 0.87 | 1.10 | 2.26 |
| EGFR_1165_1177_Y1172 | 1.43 | 0.86 | 1.06 | 1.01 |
| FAK2_572_584_Y579/Y580 | 1.06 | 0.91 | 1.07 | 1.10 |
| FGFR1_759_771_Y766 | NaN | NaN | 1.05 | NaN |
| JAK1_1015_1027_Y1022/Y1023 | 1.25 | NaN | 0.96 | **0.72** |
| LAT_194_206_Y200 | **0.65** | NaN | **0.71** | NaN |
| LAT_249_261_Y255 | 1.23 | 1.17 | 0.98 | 0.97 |
| LCK_387_399_Y394 | NaN | NaN | NaN | NaN |
| MK07_211_223_T218/Y220 | 0.90 | 1.02 | 0.98 | 0.90 |
| NTRK2_699_711_Y702/Y706/Y707 | 1.01 | 0.97 | 1.03 | 1.12 |
| PDPK1_2_14_Y9 | 1.02 | 0.96 | 0.94 | 0.99 |
| PDPK1_369_381_Y373/Y376 | 1.11 | **0.66** | 1.10 | 0.98 |
| PGFRB_1002_1014_Y1009 | NaN | NaN | 0.85 | **0.41** |
| PGFRB_709_721_Y716 | 0.92 | **0.69** | 1.02 | **0.67** |
| PRRX2_202_214_Y214 | 0.93 | 0.90 | 1.33 | 1.08 |
| VEGFR2_1052_1064_Y1059 | NaN | **0.59** | 1.02 | 0.96 |
| RON_1346_1358_Y1353 | NaN | NaN | **0.17** | NaN |
| RON_1353_1365_Y1356/Y1360 | NaN | NaN | **0.08** | NaN |
| KSYK_518_530_Y525/Y526 | 1.08 | 1.03 | 1.46 | 0.85 |
| VINC_815_827_Y821 | 0.89 | **0.76** | 1.44 | 1.00 |
| response status | NR | NR | NR | NR |
| response prediction | NR | NR | NR | NR |

R=Responder to cMET

NR=Non-Responder to cMET

A Responder is predicted when minimally 7 peptides have a ratio < 0.8 (indicated in bold font). overall prediction accuracy: 12/13 (92%)

sensitivity (number of predicted Responders over number of actual Responders):6/6 (100%)

specificity (number of predicted Non-Responders over number of actual Non-Responders): 7/8 (88%)

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A method for obtaining a pharmacological profile of a kinase inhibitor using a first and a second array of substrates immobilized on a porous matrix, said method comprising the subsequent steps of;
(i) preparing a cell lysate from a cell line, including cancer cell lines; primary and immortalized tissue cell lines; non-human animal model biopsies and patient biopsies;
(ii) filtering said cell lysate over a filter in the 10 to 0.1 micrometer range to obtain a filtered cell lysate;
(iii) contacting said first array of substrates in the presence of the kinase inhibitor with a first fraction of said filtered cell lysate and determining the response of said first array
(iv) contacting said second array of substrates in the absence of the kinase inhibitor with a second fraction of said filtered cell lysate and determining the response of said second array; and
obtaining the pharmacological profile as the ratio of the array substrate response in step (iii) *over* the array substrate response in step (iv).

2. The method as in claim 1, wherein said substrates are selected from the group consisting of hormone receptors, peptides, enzymes, oligonucleotides, monoclonal antibodies, haptens and aptamers.

3. The method as in claim 1, wherein said substrates are kinase substrates.

4. The method as in claim 1, wherein said substrates are peptide kinase substrates.

5. The method as in claim 1, wherein said substrates are at least two peptide kinase substrates selected from the group consisting of the peptide kinase substrates with sequence numbers 1 to 337.

6. The method of claim 5, wherein said substrates consist of the peptide kinase substrates with sequence numbers 15, 16, 22, 34, 62, 83, 86, 87, 100, 105, 108, 110, 113, 125, 129, and 133.

7. The method of claim 5, wherein said substrates consist of the peptide kinase substrates with sequence numbers 15, 16, 21, 23, 38, 42, 53, 62, 69, 77, 83, 86, 91, 94, 103, 112, 114, 129, 133, 136 and 138.

8. The method of claim 5, wherein said substrates consist of the peptide kinase substrates with sequence numbers 142, 2, 163, 173, 177, 190, 161, 197, 207, 208, 213, 241, 73, 252, 255, 258, 262, 79, 87, 266, 86, 269, 95, 296, 303, 305, 308, and 138.

9. The method of claim 5, wherein said substrates consist of the peptide kinase substrates with sequence numbers 5, 10, 38, 30, 54, 57, 68, 72, 73, 74, 82, 91, 98, 99, 102, 104, 110, 135, 118, 119, 71, 138.

10. The method as in any of the preceding claims 1 to 9, wherein the cell lysate is prepared from a cancer cell line; xenograft tumor or cancer patient biopsy, including tumor and normal tissue.

11. A method as in any of the preceding claims 1 to 10, wherein the response of the array of substrates is determined using a detectable signal, said signal resulting from the interaction of the sample with the array of substrates.

12. A method as claimed in any one of claims 1 to 11 wherein the response of the array of substrates to the sample is determined using detectably labeled antibodies.

13. A method as claimed in claim 12 wherein the response of the array of substrates is determined using fluorescently labeled anti-phosphotyrosine antibodies.

14. A method for predicting kinase inhibitor response in a cancer patient comprising the step of obtaining a pharmacological profile according to the methods of Claim 1 to 13 using a biopsy from said cancer patient for the preparation of the cell lysate, wherein said pharmacological profile predict the response of said patient to said kinase inhibitor.
